# EUROPEAN PATENT SPECIFICATION

(11) **EP 4 329 610 B1**
(45) Date of publication and mention of the grant of the patent: **25.11.2026**
(21) Application number: 22795124.1
(22) Date of filing: 29.04.2022
(51) Int. Cl.: A61M 16/14, A61M 16/16, A61M 16/08, A61M 16/20, A61M 16/00, A61M 16/06

(54) **PATIENT INTERFACE**
PATIENTENSCHNITTSTELLE
INTERFACE PATIENT

(30) Priority: 30.04.2021 US 202163182251 P; 24.08.2021 AU 2021221460
(43) Date of publication of application: 06.03.2024
(73) Proprietor: Fisher & Paykel Healthcare Limited, Auckland, 2013 (NZ)
(72) Inventor: PINKHAM, Maximilian Ichabod, Auckland, 2013 (NZ); GARCIA, Enrico Alvarez, Auckland, 2013 (NZ); VAN SCHALKWYK, Andre, Auckland, 2013 (NZ); O'DONNELL, Kevin Peter, Auckland, 2013 (NZ); TATKOV, Stanislav, Auckland, 2013 (NZ)
(74) Representative: Forresters IP LLP
(86) International application number: PCT/IB2022/053976
(87) International publication number: WO 2022/229909

(56) References cited:
- EP-A1- 3 782 684
- WO-A1-2015/020540
- WO-A1-2015/020540
- WO-A1-2016/043607
- WO-A1-2018/068085
- US-A1- 2018 064 898
- US-A1- 2018 064 898

## Description

### TECHNICAL FIELD

The present disclosure generally relates to a patient interface for delivering breathing gases to airways of a patient.

### BACKGROUND

Humidifiers are used to provide humidified respiratory gases to a patient. Gases are delivered to the patient via a patient interface. Examples of a patient interface include an oral mask, a nasal mask, a nasal cannula, a combination of oral and nasal mask, and the like.

Patient interfaces comprising nasal interfaces can be used to deliver a high flow of gases to a patient. Nasal delivery elements are inserted into the nose of a patient to deliver the required therapy. The nasal delivery elements may be required to seal or semi-seal at the nose, or may not be required to seal at the nose, to deliver the therapy. Nasal high flow typically is a non-sealing therapy that delivers relatively high-volume flow to the patient through a nasal interface, which flow may be sufficient to meet or exceed the patient's inspiratory flow rate.

WO 2015/020540 A1 discloses a nasal interface using asymmetrical nasal delivery elements to deliver an asymmetrical flow through the interface to both nares or to either nare, and a mouthpiece may be inserted to maintain a leak, to improve dead space clearance in the upper airways, decrease peak expiratory pressure, reduce noise, increase safety of the therapy for smaller patients and reduce resistance in the interface allowing desired flow rates to be achieved at reduced motor speeds of associated flow generating devices.

### SUMMARY

The invention is defined by the appended claims. In accordance with the invention, there is provided a nasal interface comprising
a gases inlet,
a first prong and a second prong that are asymmetrical to each other,
and a gases flow path from the gases inlet to the first prong and the second prong,
wherein the first prong has a larger inner cross-sectional area in a direction transverse to gases flow through the first prong than a corresponding inner cross-sectional area of the second prong,
and wherein the first prong is downstream in the gases flow path from the second prong.

Although prongs for nasal interfaces exist in the art, an aspect of at least one of the configurations disclosed herein includes the realization that there are problems with the insertion of some prior art prongs into the nose of a patient. Prongs in the art require high motor speeds of the flow generating device to deliver the desired flow rate to the patient. A flow generating device is a device that delivers a flow of gases to a patient.

If the interface is suddenly occluded, the static pressure may increase to equal the backpressure in the system, which may potentially reach undesirable levels. The undesirably high static pressure is intensified for child and infant prongs because the reduced prong diameter required to fit the nares of a child or infant can increase resistance to flow through the interface to the patient.

Currently there are few different sized nasal delivery elements available to better fit a patient, and it can be difficult to optimise dead space clearance and delivered pressure to the patient. Some options may use supplemental oxygen, require more heating, more water and may not provide a high level of patient comfort. Undesirably high flows or excessively high flows are being provided to patients to achieve the desired pressure effects with the existing interfaces. A nasal delivery element of a nasal interface with a smaller diameter may have a high leak and as a result will deliver lower pressure to a patient. A large diameter may not be as efficient at clearing anatomical dead space from the patient airways.

A nasal interface and respiratory therapy system are disclosed that may use nasal high flow in combination with asymmetrical nasal delivery elements for a nasal interface to deliver respiratory gases to a patient via an asymmetrical flow. Asymmetrical nasal delivery elements can provide the patient with increased dead space clearance in the upper airways. Due to a decrease in peak expiratory pressure, noise can be reduced, and asymmetrical nasal delivery elements may provide a more desirable therapy for infant use due to mitigation of the risk of completely sealing the airways of the patient. The asymmetry of the nasal delivery elements can reduce the resistance to flow through the nasal interface, which can achieve desired flow rates using lower backpressure and/or lower motor speeds of the flow generating device. A nasal interface with asymmetrical nasal delivery elements can reduce the risk of both of a patient's nares being fully occluded due to an improperly sized nasal interface.

In an aspect of the disclosure not necessarily according to the invention, but useful for understanding the invention, in accordance with certain features, aspects and advantages of at least one of the embodiments disclosed herein, a nasal interface is disclosed, the nasal interface comprising
a first prong and a second prong that are asymmetrical to each other,
and a gases manifold comprising a gases inlet,
wherein the first prong and the second prong are in fluid communication with the gases inlet,
and wherein the nasal interface is configured such that at least about 60% of a total volumetric flow rate of gases flow into the gases inlet is delivered out of the nasal interface through the first prong.

The first prong and the second prong are asymmetrical to each other and/or are not symmetrical to each other and/or differ in shape and configuration to each other and/or are asymmetrical when compared to each other.

In some configurations, the nasal interface comprises a cannula body comprising the first prong and the second prong.

In some configurations, the gases manifold is integral with the cannula body or is separate from and couplable with the cannula body.

In some configurations, the first and second prongs are configured to engage with the nasal passages in an unsealed manner.

In some configurations, the first and second prongs allow exhaled gases to escape around the first and second prongs.

In some configurations, the first and second prongs are configured to provide gases to a patient without interfering with the patient's spontaneous respiration.

In some configurations, the first prong has a larger inner diameter and/or inner cross-sectional area in a direction transverse to gases flow through the first prong than a corresponding inner diameter and/or inner cross-sectional area of the second prong in a direction transverse to gases flow through the second prong.

In some configurations, the direction transverse to gases flow is substantially perpendicular or normal to gases flow through the respective prong.

In some configurations, the inner diameters and/or inner cross-sectional areas are at outlets of the first and second prongs.

In some configurations, the nasal interface is configured such that between about 60% and about 90% of the total volumetric flow rate of gases flow into the gases inlet is delivered out of the nasal interface through the first prong.

In some configurations, the nasal interface is configured such that between about 60% and about 80% of the total volumetric flow rate of gases flow into the gases inlet is delivered out of the nasal interface through the first prong.

In some configurations, the nasal interface is configured such that between about 65% and about 80% of the total volumetric flow rate of gases flow into the gases inlet is delivered out of the nasal interface through the first prong.

In some configurations, the nasal interface is configured such that between about 70% and about 80% of the total volumetric flow rate of gases flow into the gases inlet is delivered out of the nasal interface through the first prong.

In some configurations, the nasal interface is configured such that between about 70% and about 75% of the total volumetric flow rate of gases flow into the gases inlet is delivered out of the nasal interface through the first prong.

In some configurations, the nasal interface is configured such that about 70% of the total volumetric flow rate of gases flow into the gases inlet is delivered out of the nasal interface through the first prong.

In some configurations, the nasal interface is configured such that between about 75% and about 80% of the total volumetric flow rate of gases flow into the gases inlet is delivered out of the nasal interface through the first prong.

In some configurations, the nasal interface is configured such that about 75% of the total volumetric flow rate of gases flow into the gases inlet is delivered out of the nasal interface through the first prong.

In some configurations, the nasal interface is configured such that about 80% of the total volumetric flow rate of gases flow into the gases inlet is delivered out of the nasal interface through the first prong.

In some configurations, the first prong has an inner diameter of between about 4 mm and about 10 mm, optionally between about 5 mm and about 9 mm, optionally between about 6 mm and about 8 mm, optionally about 4 mm, about 5 mm, about 6 mm, about 7 mm, about 8 mm, about 9 mm, about 10 mm, or any diameter between any two of those diameters.

In some configurations, the second prong has an inner diameter of between about 2 mm and about 8 mm, optionally between about 3 mm and about 7 mm, optionally between about 4 mm and about 6 mm, optionally about 2 mm, about 3 mm, about 4 mm, about 5 mm, about 6 mm, about 7 mm, about 8 mm, or any diameter between any two of those diameters.

In some configurations, the first prong and/or the second prong has a wall thickness of between about 0.1 mm and about 0.5 mm.

In some configurations, the first prong has an inner cross-sectional area of between about 15 mm² and about 80 mm², optionally between about 20 mm² and about 75 mm², optionally between about 25 mm² and about 70 mm², optionally between about 30 mm² and about 65 mm², optionally between about 35 mm² and about 60 mm², optionally between about 40 mm² and about 55 mm², optionally between about 45 mm² and about 50 mm², optionally about 15 mm², about 16 mm², about 17 mm², about 18 mm², about 19 mm², about 20 mm², about 21 mm², about 22 mm², about 23 mm², about 24 mm², about 25 mm², about 26 mm², about 27 mm², about 28 mm², about 29 mm², about 30 mm², about 31 mm², about 32 mm², about 33 mm², about 34 mm², about 35 mm², about 36 mm², about 37 mm², about 38 mm², about 39 mm², about 40 mm², about 41 mm², about 42 mm², about 43 mm², about 44 mm², about 45 mm², about 46 mm², about 47 mm², about 48 mm², about 49 mm², about 50 mm², about 51 mm², about 52 mm², about 53 mm², about 54 mm², about 55 mm², about 56 mm², about 57 mm², about 58 mm², about 59 mm², about 60 mm², about 61 mm², about 62 mm², about 63 mm², about 64 mm², about 65 mm², about 66 mm², about 67 mm², about 68 mm², about 69 mm², about 70 mm², about 71 mm², about 72 mm², about 73 mm², about 74 mm², about 75 mm², about 76 mm², about 77 mm², about 78 mm², about 79 mm², about 80 mm², or any cross-sectional area between any two of those cross-sectional areas.

In some configurations, the second prong has an inner cross-sectional area of between about 5 mm² and about 50 mm², optionally between about 10 mm² and about 45 mm², optionally between about 15 mm² and about 40 mm², optionally between about 20 mm² and about 35 mm², optionally between about 25 mm² and about 30 mm², optionally about 5 mm², about 6 mm², about 7 mm², about 8 mm², about 9 mm², about 10 mm², about 11 mm², about 12 mm², about 13 mm², about 14 mm², about 15 mm², about 16 mm², about 17 mm², about 18 mm², about 19 mm², about 20 mm², about 21 mm², about 22 mm², about 23 mm², about 24 mm², about 25 mm², about 26 mm², about 27 mm², about 28 mm², about 29 mm², about 30 mm², about 31 mm², about 32 mm², about 33 mm², about 34 mm², about 35 mm², about 36 mm², about 37 mm², about 38 mm², about 39 mm², about 40 mm², about 41 mm², about 42 mm², about 43 mm², about 44 mm², about 45 mm², about 46 mm², about 47 mm², about 48 mm², about 49 mm², about 50 mm², or any cross-sectional area between any two of those cross-sectional areas.

In some configurations, a combined inner cross-sectional area of the first prong and the second prong is between about 20 mm² and about 130 mm², optionally between about 30 mm² and about 120 mm², optionally between about 40 mm² and about 110 mm², optionally between about 50 mm² and about 100 mm², optionally between about 60 mm² and about 90 mm², optionally between about 70 mm² and about 80 mm², optionally about 20 mm², about 25 mm², about 30 mm², about 35 mm², about 40 mm², about 45 mm², about 50 mm², about 55 mm², about 60 mm², about 65 mm², about 70 mm², about 75 mm², about 80 mm², about 85 mm², about 90 mm², about 95 mm², about 100 mm², about 105 mm², about 110 mm², about 115 mm², about 120 mm², about 125 mm², about 130 mm², or any cross-sectional area between any two of those cross-sectional areas.

In some configurations, a ratio of the inner cross-sectional area of the first prong to the inner cross-sectional area of the second prong is between about 60:40 and about 80:20; optionally between about 65:35 and about 80:20; optionally between about 70:30 and about 80:20; optionally between about 70:30 and about 75:25; optionally about 70:30, about 71:29, about 72:28, about 73:27, about 74:26, or about 75:25; optionally between about 75:25 and 80:20; optionally about 75:25, about 76:24, about 77:23, about 78:22, about 79:21, or about 80:20.

In some configurations, a gap between adjacent outer surfaces of the first prong and the second prong adjacent a base of the first prong and the second prong is between about 5 mm and about 15 mm, optionally between about 6 mm and about 14 mm, optionally between about 7 mm and about 13 mm, optionally between about 8 mm and about 12 mm, optionally between about 9 mm and about 11 mm, optionally about 5 mm, about 6 mm, about 7 mm, about 8 mm, about 9 mm, about 10 mm, about 11 mm, about 12 mm, about 13 mm, about 14 mm, about 15 mm, or any value between any two of those values.

In some configurations, the gases inlet is in fluid communication with a breathable tube.

In some configurations, water vapour can pass through a wall of the tube, but liquid water and a bulk flow of gases cannot flow through the wall of the tube.

In some configurations, the nasal interface comprises a cannula body comprising the first prong and the second prong, wherein the gases manifold is reconfigurable relative to the cannula body between a first configuration and a second configuration, wherein the first configuration corresponds to the gases manifold being inserted into the cannula body from a first side of the cannula body such that the second prong is more proximal the gases inlet and the first prong is more distal the gases inlet, and the second configuration corresponds to the gases manifold being inserted into the cannula body from a second side of the cannula body such that the first prong is more proximal the gases inlet and the second prong is more distal the gases inlet.

In a further aspect of the disclosure not necessarily according to the invention, but useful for understanding the invention, in accordance with certain features, aspects and advantages of at least one of the embodiments disclosed herein, a nasal interface is disclosed, the nasal interface comprising
a first prong and a second prong that are asymmetrical to each other,
and a gases manifold comprising a gases inlet,
wherein the first prong and the second prong are in fluid communication with the gases inlet,
wherein the nasal interface is configured to cause an asymmetrical flow of gases at a patient's nares,
and wherein the nasal interface is configured such that between about 60% and about 80% of a total volumetric flow rate of gases flow into the gases inlet is delivered out of the nasal interface through the first prong when the total volumetric flow rate of gases flow into the gases inlet is between about 5 liters per minute (lpm) and about 70 lpm.

The first prong and the second prong are asymmetrical to each other and/or are not symmetrical to each other and/or differ in shape and configuration to each other and/or are asymmetrical when compared to each other.

In some configurations, the nasal interface comprises a cannula body comprising the first prong and the second prong.

In some configurations, the gases manifold is integral with the cannula body or is separate from and couplable with the cannula body.

In some configurations, the first and second prongs are configured to engage with the nasal passages in an unsealed manner.

In some configurations, the first and second prongs allow exhaled gases to escape around the first and second prongs.

In some configurations, the first and second prongs are configured to provide gases to the patient without interfering with the patient's spontaneous respiration.

In some configurations, the nasal interface is configured such that between about 70% and about 80% of the total volumetric flow rate of gases flow into the gases inlet is delivered out of the nasal interface through the first prong when the total flow rate of gases flow into the gases inlet is between about 5 lpm and about 70 lpm.

In some configurations, the nasal interface is configured such that between about 70% and about 75% of the total volumetric flow rate of gases flow into the gases inlet is delivered out of the nasal interface through the first prong when the total flow rate of gases flow into the gases inlet is between about 5 lpm and about 70 lpm.

In some configurations, the nasal interface is configured such that between about 75% and about 80% of the total volumetric flow rate of gases flow into the gases inlet is delivered out of the nasal interface through the first prong when the total flow rate of gases flow into the gases inlet is between about 5 lpm and about 70 lpm.

In some configurations, the nasal interface is configured such that about 75% of the total volumetric flow rate of gases flow into the gases inlet is delivered out of the nasal interface through the first prong when the total flow rate of gases flow into the gases inlet is between about 5 lpm and about 70 lpm.

In some configurations, the nasal interface is configured such that an amount of asymmetry of flow from the first prong and second prong is a function of the total flow rate of gases flow into the gases inlet.

In some configurations, the nasal interface is configured such that a higher total flow rate of gases flow into the gases inlet results in a larger portion of the total volumetric flow rate of gases flow being delivered out of the nasal interface through the first prong, and wherein a lower total flow rate of gases flow into the gases inlet results in a smaller portion of the total volumetric flow rate of gases flow being delivered out of the nasal interface through the first prong.

In some configurations, the gases inlet is in fluid communication with a breathable tube.

In some configurations, water vapour can pass through a wall of the tube, but liquid water and a bulk flow of gases cannot flow through the wall of the tube.

In some configurations, the nasal interface comprises a cannula body comprising the first prong and the second prong, wherein the gases manifold is reconfigurable relative to the cannula body between a first configuration and a second configuration, wherein the first configuration corresponds to the gases manifold being inserted into the cannula body from a first side of the cannula body such that the second prong is more proximal the gases inlet and the first prong is more distal the gases inlet, and the second configuration corresponds to the gases manifold being inserted into the cannula body from a second side of the cannula body such that the first prong is more proximal the gases inlet and the second prong is more distal the gases inlet.

In a further aspect of the disclosure not necessarily according to the invention, but useful for understanding the invention, in accordance with certain features, aspects and advantages of at least one of the embodiments disclosed herein, a nasal interface is disclosed, the nasal interface comprising
a gases inlet,
a first prong and a second prong that are asymmetrical to each other,
and a gases manifold comprising a gases inlet,
wherein the first prong and the second prong are in fluid communication with the gases inlet,
wherein the first prong has a larger inner diameter and/or inner cross-sectional area in a direction transverse to gases flow through the first prong than a corresponding inner diameter and/or inner cross-sectional area of the second prong in a direction transverse to gases flow through the second prong.

The first prong and the second prong are asymmetrical to each other and/or are not symmetrical to each other and/or differ in shape and configuration to each other and/or are asymmetrical when compared to each other.

In some configurations, the direction transverse to gases flow is substantially perpendicular or normal to gases flow through the respective prong.

In some configurations, the inner diameters and/or inner cross-sectional areas are at outlets of the first and second prongs.

In some configurations, the nasal interface comprises a cannula body comprising the first prong and the second prong.

In some configurations, the gases manifold is integral with the cannula body or is separate from and couplable with the cannula body.

In some configurations, the first and second prongs are configured to engage with the nasal passages in an unsealed manner.

In some configurations, the first and second prongs allow exhaled gases to escape around the first and second prongs.

In some configurations, the first prong has an inner diameter of between about 4 mm and about 10 mm, optionally between about 5 mm and about 9 mm, optionally between about 6 mm and about 8 mm, optionally about 4 mm, about 5 mm, about 6 mm, about 7 mm, about 8 mm, about 9 mm, about 10 mm, or any diameter between any two of those values.

In some configurations, the second prong has an inner diameter of between about 2 mm and about 8 mm, optionally between about 3 mm and about 7 mm, optionally between about 4 mm and about 6 mm, optionally about 2 mm, about 3 mm, about 4 mm, about 5 mm, about 6 mm, about 7 mm, about 8 mm, or any diameter between any two of those values.

In some configurations, the first prong has an inner cross-sectional area of between about 15 mm² and about 80 mm², optionally between about 20 mm² and about 75 mm², optionally between about 25 mm² and about 70 mm², optionally between about 30 mm² and about 65 mm², optionally between about 35 mm² and about 60 mm², optionally between about 40 mm² and about 55 mm², optionally between about 45 mm² and about 50 mm², optionally about 15 mm², about 16 mm², about 17 mm², about 18 mm², about 19 mm², about 20 mm², about 21 mm², about 22 mm², about 23 mm², about 24 mm², about 25 mm², about 26 mm², about 27 mm², about 28 mm², about 29 mm², about 30 mm², about 31 mm², about 32 mm², about 33 mm², about 34 mm², about 35 mm², about 36 mm², about 37 mm², about 38 mm², about 39 mm², about 40 mm², about 41 mm², about 42 mm², about 43 mm², about 44 mm², about 45 mm², about 46 mm², about 47 mm², about 48 mm², about 49 mm², about 50 mm², about 51 mm², about 52 mm², about 53 mm², about 54 mm², about 55 mm², about 56 mm², about 57 mm², about 58 mm², about 59 mm², about 60 mm², about 61 mm², about 62 mm², about 63 mm², about 64 mm², about 65 mm², about 66 mm², about 67 mm², about 68 mm², about 69 mm², about 70 mm², about 71 mm², about 72 mm², about 73 mm², about 74 mm², about 75 mm², about 76 mm², about 77 mm², about 78 mm², about 79 mm², about 80 mm², or any cross-sectional area between any two of those cross-sectional areas.

In some configurations, the second prong has an inner cross-sectional area of between about 5 mm² and about 50 mm², optionally between about 10 mm² and about 45 mm², optionally between about 15 mm² and about 40 mm², optionally between about 20 mm² and about 35 mm², optionally between about 25 mm² and about 30 mm², optionally about 5 mm², about 6 mm², about 7 mm², about 8 mm², about 9 mm², about 10 mm², about 11 mm², about 12 mm², about 13 mm², about 14 mm², about 15 mm², about 16 mm², about 17 mm², about 18 mm², about 19 mm², about 20 mm², about 21 mm², about 22 mm², about 23 mm², about 24 mm², about 25 mm², about 26 mm², about 27 mm², about 28 mm², about 29 mm², about 30 mm², about 31 mm², about 32 mm², about 33 mm², about 34 mm², about 35 mm², about 36 mm², about 37 mm², about 38 mm², about 39 mm², about 40 mm², about 41 mm², about 42 mm², about 43 mm², about 44 mm², about 45 mm², about 46 mm², about 47 mm², about 48 mm², about 49 mm², about 50 mm², or any cross-sectional area between any two of those cross-sectional areas.

In some configurations, a combined inner cross-sectional area of the first prong and the second prong is between about 20 mm² and about 130 mm², optionally between about 30 mm² and about 120 mm², optionally between about 40 mm² and about 110 mm², optionally between about 50 mm² and about 100 mm², optionally between about 60 mm² and about 90 mm², optionally between about 70 mm² and about 80 mm², optionally about 20 mm², about 25 mm², about 30 mm², about 35 mm², about 40 mm², about 45 mm², about 50 mm², about 55 mm², about 60 mm², about 65 mm², about 70 mm², about 75 mm², about 80 mm², about 85 mm², about 90 mm², about 95 mm², about 100 mm², about 105 mm², about 110 mm², about 115 mm², about 120 mm², about 125 mm², about 130 mm², or any cross-sectional area between any two of those cross-sectional areas.

In some configurations, a ratio of the inner cross-sectional area of the first prong to the inner cross-sectional area of the second prong is between about 60:40 and about 80:20; optionally between about 65:35 and about 80:20; optionally between about 70:30 and about 80:20; optionally between about 70:30 and about 75:25; optionally about 70:30, about 71:29, about 72:28, about 73:27, about 74:26, or about 75:25; optionally between about 75:25 and 80:20; optionally about 75:25, about 76:24, about 77:23, about 78:22, about 79:21, or about 80:20.

In some configurations, a gap between adjacent outer surfaces of the first prong and the second prong adjacent a base of the first prong and the second prong is between about 5 mm and about 15 mm, optionally between about 6 mm and about 14 mm, optionally between about 7 mm and about 13 mm, optionally between about 8 mm and about 12 mm, optionally between about 9 mm and about 11 mm, optionally about 5 mm, about 6 mm, about 7 mm, about 8 mm, about 9 mm, about 10 mm, about 11 mm, about 12 mm, about 13 mm, about 14 mm, about 15 mm, or any value between any two of those values.

In some configurations, the nasal interface is configured such that at least about 60% of a total volumetric flow rate of gases flow into the gases inlet is delivered out of the nasal interface through the first prong, optionally such that between about 60% and about 90% of the total volumetric flow rate of gases flow into the gases inlet is delivered out of the nasal interface through the first prong, optionally such that between about 60% and about 80% of the total volumetric flow rate of gases flow into the gases inlet is delivered out of the nasal interface through the first prong, optionally such that between about 65% and about 80% of the total volumetric flow rate of gases flow into the gases inlet is delivered out of the nasal interface through the first prong, optionally such that between about 70% and about 80% of the total volumetric flow rate of gases flow into the gases inlet is delivered out of the nasal interface through the first prong, optionally such that between about 70% and about 75% of the total volumetric flow rate of gases flow into the gases inlet is delivered out of the nasal interface through the first prong, optionally such that about 70% of the total volumetric flow rate of gases flow into the gases inlet is delivered out of the nasal interface through the first prong, optionally such that between about 75% and about 80% of the total volumetric flow rate of gases flow into the gases inlet is delivered out of the nasal interface through the first prong, optionally such that about 75% of the total volumetric flow rate of gases flow into the gases inlet is delivered out of the nasal interface through the first prong, optionally such that about 80% of the total gases flow into the gases inlet is delivered out of the nasal interface through the first prong.

In some configurations, the gases inlet is in fluid communication with a breathable tube.

In some configurations, water vapour can pass through a wall of the tube, but liquid water and a bulk flow of gases cannot flow through the wall of the tube.

In some configurations, the nasal interface comprises a cannula body comprising the first prong and the second prong, wherein the gases manifold is reconfigurable relative to the cannula body between a first configuration and a second configuration, wherein the first configuration corresponds to the gases manifold being inserted into the cannula body from a first side of the cannula body such that the second prong is more proximal the gases inlet and the first prong is more distal the gases inlet, and the second configuration corresponds to the gases manifold being inserted into the cannula body from a second side of the cannula body such that the first prong is more proximal the gases inlet and the second prong is more distal the gases inlet.

In a further aspect of the disclosure not necessarily according to the invention, but useful for understanding the invention, in accordance with certain features, aspects and advantages of at least one of the embodiments disclosed herein, a nasal interface is disclosed, the nasal interface comprising
a first prong and a second prong that are asymmetrical to each other,
and a gases manifold comprising a gases inlet,
wherein the first prong and the second prong are in fluid communication with the gases inlet,
wherein the first prong has a larger inner cross-sectional area in a direction transverse to gases flow through the first prong than a corresponding inner cross-sectional area of the second prong in a direction transverse to gases flow through the second prong,
wherein the second prong has a substantially ovate or substantially elliptical cross-sectional shape in the direction transverse to gases flow through the second prong, the substantially ovate or substantially elliptical cross-sectional shape having a first ratio of a widest dimension to a narrowest dimension,
and wherein the first prong has a less ovate or less elliptical cross-sectional shape in the direction transverse to gases flow through the first prong, the less ovate or less elliptical cross-sectional shape having either a second ratio of a widest dimension to a narrowest dimension that is smaller than the first ratio or a substantially circular cross-sectional shape.

The first prong and the second prong are asymmetrical to each other and/or are not symmetrical to each other and/or differ in shape and configuration to each other and/or are asymmetrical when compared to each other.

In some configurations, the nasal interface comprises a cannula body comprising the first prong and the second prong.

In some configurations, the gases manifold is integral with the cannula body or is separate from and couplable with the cannula body.

In some configurations, the first and second prongs are configured to engage with the nasal passages in an unsealed manner.

In some configurations, the first and second prongs allow exhaled gases to escape around the first and second prongs.

In some configurations, the first and second prongs are configured to provide gases to the patient without interfering with the patient's spontaneous respiration.

In some configurations, the direction transverse to gases flow is substantially perpendicular or normal to gases flow through the respective prong.

In some configurations, the inner cross-sectional areas and inner cross-sectional shapes of the first and second prongs are at the outlets of the first and second prongs.

In some configurations, the first prong is more flexible than the second prong.

In some configurations, the first prong has a substantially circular shape.

In some configurations, the first prong has a first terminal end and wherein the second prong has a second terminal end, wherein the first terminal end comprises a substantially scalloped surface

In some configurations, the second terminal end has a substantially planar face.

In some configurations, the gases inlet is in fluid communication with a breathable tube.

In some configurations, water vapour can pass through a wall of the tube, but liquid water and a bulk flow of gases cannot flow through the wall of the tube.

In some configurations, the nasal interface comprises a cannula body comprising the first prong and the second prong, wherein the gases manifold is reconfigurable relative to the cannula body between a first configuration and a second configuration, wherein the first configuration corresponds to the gases manifold being inserted into the cannula body from a first side of the cannula body such that the second prong is more proximal the gases inlet and the first prong is more distal the gases inlet, and the second configuration corresponds to the gases manifold being inserted into the cannula body from a second side of the cannula body such that the first prong is more proximal the gases inlet and the second prong is more distal the gases inlet.

The first prong and the second prong are asymmetrical to each other and/or are not symmetrical to each other and/or differ in shape and configuration to each other and/or are asymmetrical when compared to each other.

In some configurations, the direction transverse to gases flow is substantially perpendicular or normal to gases flow through the respective prong.

In some configurations, the inner cross-sectional areas are at outlets of the first and second prongs.

In some configurations, the nasal interface comprises a cannula body comprising the first prong and the second prong.

In some configurations, the gases manifold is integral with the cannula body or is separate from and couplable with the cannula body.

In some configurations, the first and second prongs are configured to engage with the nasal passages in an unsealed manner.

In some configurations, the first and second prongs allow exhaled gases to escape around the first and second prongs.

In some configurations, the first and second prongs are configured to provide gases to the patient without interfering with the patient's spontaneous respiration.

In some configurations, the gases flow path is defined by a flow channel that has a gases flow direction that is substantially perpendicular to gases flow paths through the first prong and the second prong, and wherein the first prong is more distal the gases inlet and the second prong is more proximal the gases inlet.

In some configurations, the nasal interface is configured such that at least about 60% of a total volumetric flow rate of gases flow into the gases inlet is delivered out of the nasal interface through the first prong, optionally such that between about 60% and about 90% of the total volumetric flow rate of gases flow into the gases inlet is delivered out of the nasal interface through the first prong, optionally such that between about 60% and about 80% of the total volumetric flow rate of gases flow into the gases inlet is delivered out of the nasal interface through the first prong, optionally such that between about 65% and about 80% of the total volumetric flow rate of gases flow into the gases inlet is delivered out of the nasal interface through the first prong, optionally such that between about 70% and about 80% of the total volumetric flow rate of gases flow into the gases inlet is delivered out of the nasal interface through the first prong, optionally such that between about 70% and about 75% of the total volumetric flow rate of gases flow into the gases inlet is delivered out of the nasal interface through the first prong, optionally such that about 70% of the total volumetric flow rate of gases flow into the gases inlet is delivered out of the nasal interface through the first prong, optionally such that between about 75% and about 80% of the total volumetric flow rate of gases flow into the gases inlet is delivered out of the nasal interface through the first prong, optionally such that about 75% of the total volumetric flow rate of gases flow into the gases inlet is delivered out of the nasal interface through the first prong, optionally such that about 80% of the total volumetric flow rate of gases flow into the gases inlet is delivered out of the nasal interface through the first prong.

In some configurations, the gases inlet is in fluid communication with a breathable tube.

In some configurations, water vapour can pass through a wall of the tube, but liquid water and a bulk flow of gases cannot flow through the wall of the tube.

In some configurations, the nasal interface comprises a cannula body comprising the first prong and the second prong, wherein the gases manifold is reconfigurable relative to the cannula body between a first configuration and a second configuration, wherein the first configuration corresponds to the gases manifold being inserted into the cannula body from a first side of the cannula body such that the second prong is more proximal the gases inlet and the first prong is more distal the gases inlet, and the second configuration corresponds to the gases manifold being inserted into the cannula body from a second side of the cannula body such that the first prong is more proximal the gases inlet and the second prong is more distal the gases inlet.

In a further aspect of the disclosure not necessarily according to the invention, but useful for understanding the invention, in accordance with certain features, aspects and advantages of at least one of the embodiments disclosed herein, a nasal interface is disclosed, the nasal interface comprising
a first prong and a second prong,
and a gases manifold comprising a gases inlet,
wherein the first prong and the second prong are in fluid communication with the gases inlet,
wherein the nasal interface is configured to cause an asymmetrical flow of gases at a patient's nares,
and wherein the gases inlet is in fluid communication with a breathable tube.

In some configurations, the first prong and the second prong are asymmetrical to each other or are not symmetrical to each other or differ in shape and configuration to each other or are asymmetrical when compared to each other.

In some configurations, the nasal interface comprises a cannula body comprising the first prong and the second prong.

In some configurations, the gases manifold is integral with the cannula body or is separate from and couplable with the cannula body.

In some configurations, the first and second prongs are configured to engage with the nasal passages in an unsealed manner.

In some configurations, the first and second prongs allow exhaled gases to escape around the first and second prongs.

In some configurations, the first and second prongs are configured to provide gases to the patient without interfering with the patient's spontaneous respiration.

In some configurations, the gases manifold is integrally formed with the breathable tube or is coupled to the breathable tube.

In some configurations, water vapour can pass through a wall of the tube, but liquid water and a bulk flow of gases cannot flow through the wall of the tube.

In some configurations, the nasal interface comprises a cannula body comprising the first prong and the second prong, wherein the gases manifold is reconfigurable relative to the cannula body between a first configuration and a second configuration, wherein the first configuration corresponds to the gases manifold being inserted into the cannula body from a first side of the cannula body and the second configuration corresponds to the gases manifold being inserted into the cannula body from a second side of the cannula body such that the first prong is more proximal the gases inlet and the second prong is more distal the gases inlet.

In some configurations, the nasal interface comprises a cannula body comprising the first prong and the second prong, and wherein an external surface of the cannula body between the first prong and the second prong comprises a dip to accommodate a portion of a patient's nose and reduce pressure on an underside of the accommodated portion.

In a further aspect of the disclosure not necessarily according to the invention, but useful for understanding the invention, in accordance with certain features, aspects and advantages of at least one of the embodiments disclosed herein, a nasal interface is disclosed, the nasal interface comprising
a cannula body comprising a first prong and a second prong, wherein the first prong and the second prong are asymmetrical to each other,
and a gases manifold comprising a gases inlet,
wherein the first prong and the second prong are in fluid communication with the gases inlet,
wherein the nasal interface is configured to cause an asymmetrical flow of gases at a patient's nares,
and wherein the gases manifold is reconfigurable relative to the cannula body between a first configuration and a second configuration, wherein the first configuration corresponds to the gases manifold being inserted into the cannula body from a first side of the cannula body and such that the second prong is more proximal the gases inlet and the first prong is more distal the gases inlet, and the second configuration corresponds to the gases manifold being inserted into the cannula body from a second side of the cannula body such that the first prong is more proximal the gases inlet and the second prong is more distal the gases inlet.

In some configurations, the first prong and the second prong are asymmetrical to each other and/or are not symmetrical to each other and/or differ in shape and configuration to each other and/or are asymmetrical when compared to each other.

In some configurations, the first and second prongs are configured to engage with the nasal passages in an unsealed manner.

In some configurations, the first and second prongs allow exhaled gases to escape around the first and second prongs.

In some configurations, the first and second prongs are configured to provide gases to the patient without interfering with the patient's spontaneous respiration.

In some configurations, the gases manifold comprise a flow channel that has a gases flow direction that is substantially perpendicular to gases flow paths through the first prong and the second prong.

In some configurations, the gases inlet is in fluid communication with a breathable tube.

In some configurations, the gases manifold is integrally formed with the breathable tube or is coupled to the breathable tube.

In a further aspect of the disclosure not necessarily according to the invention, but useful for understanding the invention, in accordance with certain features, aspects and advantages of at least one of the embodiments disclosed herein, a nasal interface is disclosed, the nasal interface comprising
a cannula body comprising a first prong and a second prong that are asymmetrical to each other,
and a gases manifold comprising a gases inlet,
wherein the first prong and the second prong are in fluid communication with the gases inlet,
and wherein an external surface of the cannula body between the first prong and the second prong comprises a dip.

The first prong and the second prong are asymmetrical to each other and/or are not symmetrical to each other and/or differ in shape and configuration to each other and/or are asymmetrical when compared to each other.

In some configurations, the dip is arranged to accommodate a portion of a patient's nose and reduce pressure on an underside of the accommodated portion.

In some configurations, the gases manifold is integral with the cannula body or is separate from and couplable with the cannula body.

In some configurations, the first and second prongs are configured to engage with the nasal passages in an unsealed manner.

In some configurations, the first and second prongs allow exhaled gases to escape around the first and second prongs.

In some configurations, the first and second prongs are configured to provide gases to a patient without interfering with the patient's spontaneous respiration.

In some configurations, a portion of the gases manifold is complementary to the dip.

In some configurations, the portion of the gases manifold that is complementary to the dip is an outlet of the gases manifold, and optionally a periphery of the outlet of the gases manifold.

In some configurations, the cannula body and/or the gases manifold comprises retaining feature(s) to removably retain the gases manifold in engagement in the cannula body.

In some configurations, the retaining features comprise a resilient annular portion of the cannula body that is received in a complementary recess of the gases manifold.

In a further aspect of the disclosure not necessarily according to the invention, but useful for understanding the invention, in accordance with certain features, aspects and advantages of at least one of the embodiments disclosed herein, a nasal interface is disclosed, the nasal interface comprising
a cannula body comprising a first prong and a second prong that are asymmetrical to each other,
and a gases manifold comprising a gases inlet,
wherein the first prong and the second prong are in fluid communication with the gases inlet,
the nasal interface further comprising two side arms comprising wing portions extending laterally from either side of the cannula body,
the nasal interface comprising or provided in combination a tube retention clip.

The first prong and the second prong are asymmetrical to each other and/or are not symmetrical to each other and/or differ in shape and configuration to each other and/or are asymmetrical when compared to each other.

In some configurations, the gases manifold is integral with the cannula body or is separate from and couplable with the cannula body.

In some configurations, the first and second prongs are configured to engage with the nasal passages in an unsealed manner.

In some configurations, the first and second prongs allow exhaled gases to escape around the first and second prongs.

In some configurations, the first and second prongs are configured to provide gases to a patient without interfering with the patient's spontaneous respiration.

In some configurations, the tube retention clip is configured to support a patient conduit or other gases supply tube.

In a further aspect of the disclosure not necessarily according to the invention, but useful for understanding the invention, in accordance with certain features, aspects and advantages of at least one of the embodiments disclosed herein, a nasal interface is disclosed, the nasal interface comprising
a first prong having a shape and a second prong having a shape,
and a gases manifold comprising a gases inlet,
wherein the first prong and the second prong are in fluid communication with the gases inlet,
wherein the first prong has a larger inner cross-sectional area in a direction transverse to gases flow through the first prong than a corresponding inner cross-sectional area of the second prong in a direction transverse to gases flow through the second prong, and
wherein at least the first prong is made of an elastomeric material that enables the first prong to deform and set its shape in use in response to temperature and contact with the patient's naris.

In some configurations, the temperature may be between about 20°C and about 41°C, optionally more than 20°C and up to about 41°C, optionally between about 31°C and about 41°C, optionally between about 36°C and about 39°C, optionally about 37°C.

In some configurations, the first prong is configured to deform and set its shape in use to substantially match the internal shape of the patient's naris.

In some configurations, the elastomeric material enables the first prong to deform and set its shape to substantially match the internal shape of the patient's naris at therapy temperatures of between about 31°C and about 41°C, optionally between about 36°C and about 39°C, optionally about 37°C.

In some configurations, the first prong is not made of silicone.

In some configurations, at least the first prong is made of a thermoplastic elastomer.

In some configurations, the material exhibits between about 10% and about 50% compression set at temperatures between about 20°C and about 40°C after 72 hours when tested according to Method A of ISO 815-1:2014.

In some configurations, the elastomeric material exhibits between about 10% and about 45%, optionally between about 10% and about 40%, optionally between about 10% and about 35%, optionally between about 10% and about 30%, optionally between about 10% and about 25%, optionally between about 10% and about 20%, optionally between about 11% and about 19%, optionally between about 12% and about 18%, optionally between about 13% and about 17%, optionally between about 14% and about 16%, optionally about 15% compression set at temperatures between about 20°C and about 40°C after 72 hours when tested according to Method A of ISO 815-1:2014.

In some configurations, the elastomeric material exhibits between about 10% and about 45%, optionally between about 10% and about 40%, optionally between about 10% and about 35%, optionally between about 10% and about 30%, optionally between about 10% and about 25%, optionally between about 10% and about 20%, optionally between about 11% and about 19%, optionally between about 12% and about 18%, optionally between about 13% and about 17%, optionally between about 14% and about 16%, optionally about 15% compression set at temperatures above about 20°C and up to about 35°C, optionally at temperatures above about 20°C and up to about 30°C, optionally at temperatures above about 20°C and up to about 25°C, optionally at a temperature of about 21°C or about 22°C or about 23°C or about 24°C or about 25°C or higher after 72 hours when tested according to Method A of ISO 815-1:2014.

In some configurations, both the first prong and the second prong are made of the elastomeric material.

In some configurations, the second prong has a substantially ovate or substantially elliptical cross-sectional shape in the direction transverse to gases flow through the second prong, the substantially ovate or substantially elliptical cross-sectional shape having a first ratio of a widest dimension to a narrowest dimension, and wherein the first prong has a less ovate or less elliptical cross-sectional shape in the direction transverse to gases flow through the first prong, the less ovate or less elliptical cross-sectional shape having either a second ratio of a widest dimension to a narrowest dimension that is smaller than the first ratio or a substantially circular cross-sectional shape.

In some configurations, the first prong has a substantially circular shape.

In some configurations, the first prong has a first terminal end and wherein the second prong has a second terminal end, wherein the first terminal end comprises a substantially scalloped surface.

In some configurations, the second terminal end has a substantially planar face.

In some configurations, the first prong has an inner diameter of between about 4 mm and about 10 mm, optionally between about 5 mm and about 9 mm, optionally between about 6 mm and about 8 mm, optionally about 4 mm, about 5 mm, about 6 mm, about 7 mm, about 8 mm, about 9 mm, about 10 mm, or any diameter between any two of those values.

In some configurations, the second prong has an inner diameter of between about 2 mm and about 8 mm, optionally between about 3 mm and about 7 mm, optionally between about 4 mm and about 6 mm, optionally about 2 mm, about 3 mm, about 4 mm, about 5 mm, about 6 mm, about 7 mm, about 8 mm, or any diameter between any two of those values.

In some configurations, the first prong and/or the second prong has a wall thickness of between about 0.1 mm and about 0.5 mm.

In some configurations, the first prong has an inner cross-sectional area of between about 15 mm² and about 80 mm², optionally between about 20 mm² and about 75 mm², optionally between about 25 mm² and about 70 mm², optionally between about 30 mm² and about 65 mm², optionally between about 35 mm² and about 60 mm², optionally between about 40 mm² and about 55 mm², optionally between about 45 mm² and about 50 mm², optionally about 15 mm², about 16 mm², about 17 mm², about 18 mm², about 19 mm², about 20 mm², about 21 mm², about 22 mm², about 23 mm², about 24 mm², about 25 mm², about 26 mm², about 27 mm², about 28 mm², about 29 mm², about 30 mm², about 31 mm², about 32 mm², about 33 mm², about 34 mm², about 35 mm², about 36 mm², about 37 mm², about 38 mm², about 39 mm², about 40 mm², about 41 mm², about 42 mm², about 43 mm², about 44 mm², about 45 mm², about 46 mm², about 47 mm², about 48 mm², about 49 mm², about 50 mm², about 51 mm², about 52 mm², about 53 mm², about 54 mm², about 55 mm², about 56 mm², about 57 mm², about 58 mm², about 59 mm², about 60 mm², about 61 mm², about 62 mm², about 63 mm², about 64 mm², about 65 mm², about 66 mm², about 67 mm², about 68 mm², about 69 mm², about 70 mm², about 71 mm², about 72 mm², about 73 mm², about 74 mm², about 75 mm², about 76 mm², about 77 mm², about 78 mm², about 79 mm², about 80 mm², or any cross-sectional area between any two of those cross-sectional areas.

In some configurations, the second prong has an inner cross-sectional area of between about 5 mm² and about 50 mm², optionally between about 10 mm² and about 45 mm², optionally between about 15 mm² and about 40 mm², optionally between about 20 mm² and about 35 mm², optionally between about 25 mm² and about 30 mm², optionally about 5 mm², about 6 mm², about 7 mm², about 8 mm², about 9 mm², about 10 mm², about 11 mm², about 12 mm², about 13 mm², about 14 mm², about 15 mm², about 16 mm², about 17 mm², about 18 mm², about 19 mm², about 20 mm², about 21 mm², about 22 mm², about 23 mm², about 24 mm², about 25 mm², about 26 mm², about 27 mm², about 28 mm², about 29 mm², about 30 mm², about 31 mm², about 32 mm², about 33 mm², about 34 mm², about 35 mm², about 36 mm², about 37 mm², about 38 mm², about 39 mm², about 40 mm², about 41 mm², about 42 mm², about 43 mm², about 44 mm², about 45 mm², about 46 mm², about 47 mm², about 48 mm², about 49 mm², about 50 mm², or any cross-sectional area between any two of those cross-sectional areas.

In some configurations, a combined inner cross-sectional area of the first prong and the second prong is between about 20 mm² and about 130 mm², optionally between about 30 mm² and about 120 mm², optionally between about 40 mm² and about 110 mm², optionally between about 50 mm² and about 100 mm², optionally between about 60 mm² and about 90 mm², optionally between about 70 mm² and about 80 mm², optionally about 20 mm², about 25 mm², about 30 mm², about 35 mm², about 40 mm², about 45 mm², about 50 mm², about 55 mm², about 60 mm², about 65 mm², about 70 mm², about 75 mm², about 80 mm², about 85 mm², about 90 mm², about 95 mm², about 100 mm², about 105 mm², about 110 mm², about 115 mm², about 120 mm², about 125 mm², about 130 mm², or any cross-sectional area between any two of those cross-sectional areas.

In some configurations, a ratio of the inner cross-sectional area of the first prong to the inner cross-sectional area of the second prong is between about 60:40 and about 80:20; optionally between about 65:35 and about 80:20; optionally between about 70:30 and about 80:20; optionally between about 70:30 and about 75:25; optionally about 70:30, about 71:29, about 72:28, about 73:27, about 74:26, or about 75:25; optionally between about 75:25 and 80:20; optionally about 75:25, about 76:24, about 77:23, about 78:22, about 79:21, or about 80:20.

In some configurations, the gases inlet is in fluid communication with a breathable tube.

In some configurations, the nasal interface comprises a cannula body comprising the first prong and the second prong, wherein the gases manifold is reconfigurable relative to the cannula body between a first configuration and a second configuration, wherein the first configuration corresponds to the gases manifold being inserted into the cannula body from a first side of the cannula body such that the second prong is more proximal the gases inlet and the first prong is more distal the gases inlet, and the second configuration corresponds to the gases manifold being inserted into the cannula body from a second side of the cannula body such that the first prong is more proximal the gases inlet and the second prong is more distal the gases inlet.

In a further aspect of the disclosure not necessarily according to the invention, but useful for understanding the invention, in accordance with certain features, aspects and advantages of at least one of the embodiments disclosed herein, a nasal interface is disclosed, the nasal interface comprising
a first prong and a second prong that are asymmetrical to each other,
and a gases manifold comprising a gases inlet,
wherein the first prong and the second prong are in fluid communication with the gases inlet,
wherein the nasal interface is configured to cause an asymmetrical flow of gases at a patient's nares,
wherein the first prong has an inner cross-sectional area of between about 15 mm² and about 80 mm², wherein the second prong has an inner cross-sectional area of between about 5 mm² and about 50 mm², wherein a combined inner cross-sectional area of the first prong and the second prong is between about 20 mm² and about 130 mm², and wherein a ratio of the inner cross-sectional area of the first prong to the inner cross-sectional area of the second prong is between about 60:40 and about 80:20.

In some configurations, the first prong has an inner cross-sectional area of between about 20 mm² and about 75 mm², optionally between about 25 mm² and about 70 mm², optionally between about 30 mm² and about 65 mm², optionally between about 35 mm² and about 60 mm², optionally between about 40 mm² and about 55 mm², optionally between about 45 mm² and about 50 mm², optionally about 15 mm², about 16 mm², about 17 mm², about 18 mm², about 19 mm², about 20 mm², about 21 mm², about 22 mm², about 23 mm², about 24 mm², about 25 mm², about 26 mm², about 27 mm², about 28 mm², about 29 mm², about 30 mm², about 31 mm², about 32 mm², about 33 mm², about 34 mm², about 35 mm², about 36 mm², about 37 mm², about 38 mm², about 39 mm², about 40 mm², about 41 mm², about 42 mm², about 43 mm², about 44 mm², about 45 mm², about 46 mm², about 47 mm², about 48 mm², about 49 mm², about 50 mm², about 51 mm², about 52 mm², about 53 mm², about 54 mm², about 55 mm², about 56 mm², about 57 mm², about 58 mm², about 59 mm², about 60 mm², about 61 mm², about 62 mm², about 63 mm², about 64 mm², about 65 mm², about 66 mm², about 67 mm², about 68 mm², about 69 mm², about 70 mm², about 71 mm², about 72 mm², about 73 mm², about 74 mm², about 75 mm², about 76 mm², about 77 mm², about 78 mm², about 79 mm², about 80 mm², or any cross-sectional area between any two of those cross-sectional areas.

In some configurations, the second prong has an inner cross-sectional area of between about 10 mm² and about 45 mm², optionally between about 15 mm² and about 40 mm², optionally between about 20 mm² and about 35 mm², optionally between about 25 mm² and about 30 mm², optionally about 5 mm², about 6 mm², about 7 mm², about 8 mm², about 9 mm², about 10 mm², about 11 mm², about 12 mm², about 13 mm², about 14 mm², about 15 mm², about 16 mm², about 17 mm², about 18 mm², about 19 mm², about 20 mm², about 21 mm², about 22 mm², about 23 mm², about 24 mm², about 25 mm², about 26 mm², about 27 mm², about 28 mm², about 29 mm², about 30 mm², about 31 mm², about 32 mm², about 33 mm², about 34 mm², about 35 mm², about 36 mm², about 37 mm², about 38 mm², about 39 mm², about 40 mm², about 41 mm², about 42 mm², about 43 mm², about 44 mm², about 45 mm², about 46 mm², about 47 mm², about 48 mm², about 49 mm², about 50 mm², or any cross-sectional area between any two of those cross-sectional areas.

In some configurations, the combined inner cross-sectional area of the first prong and the second prong is between about 30 mm² and about 120 mm², optionally between about 40 mm² and about 110 mm², optionally between about 50 mm² and about 100 mm², optionally between about 60 mm² and about 90 mm², optionally between about 70 mm² and about 80 mm², optionally about 20 mm², about 25 mm², about 30 mm², about 35 mm², about 40 mm², about 45 mm², about 50 mm², about 55 mm², about 60 mm², about 65 mm², about 70 mm², about 75 mm², about 80 mm², about 85 mm², about 90 mm², about 95 mm², about 100 mm², about 105 mm², about 110 mm², about 115 mm², about 120 mm², about 125 mm², about 130 mm², or any cross-sectional area between any two of those cross-sectional areas.

In some configurations, the ratio of the inner cross-sectional area of the first prong to the inner cross-sectional area of the second prong is between about 65:35 and about 80:20; optionally between about 70:30 and about 80:20; optionally between about 70:30 and about 75:25; optionally about 70:30, about 71:29, about 72:28, about 73:27, about 74:26, or about 75:25; optionally between about 75:25 and 80:20; optionally about 75:25, about 76:24, about 77:23, about 78:22, about 79:21, or about 80:20.

In some configurations, the first prong has an inner cross-sectional area of between about 24 mm² and 25 mm² and the second prong has an inner cross-sectional area of between about 6 mm² and about 17 mm².

In some configurations, the first prong has an inner cross-sectional area of between about 44 mm² and about 45 mm², and the second prong has an inner cross-sectional area of between about 11 mm² and about 30 mm².

In some configurations, the first prong has an inner cross-sectional area of between about 69 mm² and about 70 mm², and the second prong has an inner cross-sectional area of between about 17 mm² and about 47 mm².

In a further aspect of the disclosure not necessarily according to the invention, but useful for understanding the invention, in accordance with certain features, aspects and advantages of at least one of the embodiments disclosed herein, a nasal interface is disclosed, the nasal interface comprising
a gases inlet,
a first prong and a second prong that are asymmetrical to each other, and wherein the first prong has a first prong outlet and the second prong has a second prong outlet,
and a gases flow path from the gases inlet to the first prong and the second prong,
wherein the first prong has a larger inner cross-sectional area in a direction transverse to gases flow through the first prong than a corresponding inner cross-sectional area of the second prong,
wherein, for a given flow rate of gases at the gases inlet in use, different flow rates of gases are provided through the first prong and the second prong and a velocity of gases exiting the first prong outlet and the second prong outlet gases outlets is substantially similar.

The first prong and the second prong are asymmetrical to each other and/or are not symmetrical to each other and/or differ in shape and configuration to each other and/or are asymmetrical when compared to each other.

In some configurations, the velocity of gases exiting the first prong outlet is within about 20% of the velocity of gases exiting the second prong outlet.

In some configurations, the velocity of gases exiting the first prong outlet is within about 16% of the velocity of gases exiting the second prong outlet.

In some configurations, the velocity of gases exiting the first prong outlet is within about 10% of the velocity of gases exiting the second prong outlet at flow rates above about 40 lpm.

In some configurations, the velocity of gases exiting the first prong outlet is within about 10% of the velocity of gases exiting the second prong outlet at flow rates above about 42 Ipm.

In some configurations, the velocity of gases exiting each of the first prong outlet and the second prong outlet is more than 0 m/s and less than about 32 m/s for a total volumetric flow rate of gases flow into the gases inlet of more than 0 lpm and up to about 70 lpm.

In some configurations, the velocity of gases exiting each of the first prong outlet and the second prong outlet is more than 0 m/s and less than 32 m/s for a total volumetric flow rate of gases flow into the gases inlet of more than 0 lpm and up to about 70 lpm.

In some configurations, the velocity of gases exiting each of the first prong outlet and the second prong outlet is more than about 2 m/s and less than about 32 m/s, optionally more than about 2 m/s and less than 32 m/s, optionally more than about 2 m/s and up to about 25 m/s, and optionally more than about 2.5 m/s and up to about 20 m/s for a total volumetric flow rate of gases flow into the gases inlet of more than 9 lpm and up to about 70 lpm.

In some configurations, the nasal interface is configured such that a total volumetric flow rate of gases flow into the gases inlet is at least about 5 liters per minute (lpm).

In some configurations, the nasal interface is configured such that the total volumetric flow rate of gases flow into the gases inlet is between about 5 lpm and about 120 Ipm.

In some configurations, the nasal interface is configured such that the total volumetric flow rate of gases flow into the gases inlet is between about 5 lpm and about 70 lpm.

In some configurations, the nasal interface is configured such that at least about 60% of a total volumetric flow rate of gases flow into the gases inlet is delivered out of the nasal interface through the first prong.

In some configurations, the nasal interface is configured such that between about 60% and about 90% of the total volumetric flow rate of gases flow into the gases inlet is delivered out of the nasal interface through the first prong.

In some configurations, the nasal interface is configured such that between about 60% and about 80% of the total volumetric flow rate of gases flow into the gases inlet is delivered out of the nasal interface through the first prong.

In some configurations, the nasal interface is configured such that between about 65% and about 80% of the total volumetric flow rate of gases flow into the gases inlet is delivered out of the nasal interface through the first prong.

In some configurations, the nasal interface is configured such that between about 70% and about 80% of the total volumetric flow rate of gases flow into the gases inlet is delivered out of the nasal interface through the first prong.

In some configurations, the nasal interface is configured such that between about 70% and about 75% of the total volumetric flow rate of gases flow into the gases inlet is delivered out of the nasal interface through the first prong.

In some configurations, the nasal interface is configured such that about 70% of the total volumetric flow rate of gases flow into the gases inlet is delivered out of the nasal interface through the first prong.

In some configurations, the nasal interface is configured such that between about 75% and about 80% of the total volumetric flow rate of gases flow into the gases inlet is delivered out of the nasal interface through the first prong.

In some configurations, the nasal interface is configured such that about 75% of the total volumetric flow rate of gases flow into the gases inlet is delivered out of the nasal interface through the first prong.

In some configurations, the nasal interface is configured such that about 80% of the total volumetric flow rate of gases flow into the gases inlet is delivered out of the nasal interface through the first prong.

In some configurations, the first prong has an inner diameter of between about 4 mm and about 10 mm, optionally between about 5 mm and about 9 mm, optionally between about 6 mm and about 8 mm, optionally about 4 mm, about 5 mm, about 6 mm, about 7 mm, about 8 mm, about 9 mm, about 10 mm, or any diameter between any two of those diameters.

In some configurations, the second prong has an inner diameter of between about 2 mm and about 8 mm, optionally between about 3 mm and about 7 mm, optionally between about 4 mm and about 6 mm, optionally about 2 mm, about 3 mm, about 4 mm, about 5 mm, about 6 mm, about 7 mm, about 8 mm, or any diameter between any two of those diameters.

In some configurations, the inner cross-sectional area of the first prong is between about 15 mm² and about 80 mm², optionally between about 20 mm² and about 75 mm², optionally between about 25 mm² and about 70 mm², optionally between about 30 mm² and about 65 mm², optionally between about 35 mm² and about 60 mm², optionally between about 40 mm² and about 55 mm², optionally between about 45 mm² and about 50 mm², optionally about 15 mm², about 16 mm², about 17 mm², about 18 mm², about 19 mm², about 20 mm², about 21 mm², about 22 mm², about 23 mm², about 24 mm², about 25 mm², about 26 mm², about 27 mm², about 28 mm², about 29 mm², about 30 mm², about 31 mm², about 32 mm², about 33 mm², about 34 mm², about 35 mm², about 36 mm², about 37 mm², about 38 mm², about 39 mm², about 40 mm², about 41 mm², about 42 mm², about 43 mm², about 44 mm², about 45 mm², about 46 mm², about 47 mm², about 48 mm², about 49 mm², about 50 mm², about 51 mm², about 52 mm², about 53 mm², about 54 mm², about 55 mm², about 56 mm², about 57 mm², about 58 mm², about 59 mm², about 60 mm², about 61 mm², about 62 mm², about 63 mm², about 64 mm², about 65 mm², about 66 mm², about 67 mm², about 68 mm², about 69 mm², about 70 mm², about 71 mm², about 72 mm², about 73 mm², about 74 mm², about 75 mm², about 76 mm², about 77 mm², about 78 mm², about 79 mm², about 80 mm², or any cross-sectional area between any two of those cross-sectional areas.

In some configurations, the inner cross-sectional area of the second prong is between about 5 mm² and about 50 mm², optionally between about 10 mm² and about 45 mm², optionally between about 15 mm² and about 40 mm², optionally between about 20 mm² and about 35 mm², optionally between about 25 mm² and about 30 mm², optionally about 5 mm², about 6 mm², about 7 mm², about 8 mm², about 9 mm², about 10 mm², about 11 mm², about 12 mm², about 13 mm², about 14 mm², about 15 mm², about 16 mm², about 17 mm², about 18 mm², about 19 mm², about 20 mm², about 21 mm², about 22 mm², about 23 mm², about 24 mm², about 25 mm², about 26 mm², about 27 mm², about 28 mm², about 29 mm², about 30 mm², about 31 mm², about 32 mm², about 33 mm², about 34 mm², about 35 mm², about 36 mm², about 37 mm², about 38 mm², about 39 mm², about 40 mm², about 41 mm², about 42 mm², about 43 mm², about 44 mm², about 45 mm², about 46 mm², about 47 mm², about 48 mm², about 49 mm², about 50 mm², or any cross-sectional area between any two of those cross-sectional areas.

In some configurations, a combined inner cross-sectional area of the first prong and the second prong is between about 20 mm² and about 130 mm², optionally between about 30 mm² and about 120 mm², optionally between about 40 mm² and about 110 mm², optionally between about 50 mm² and about 100 mm², optionally between about 60 mm² and about 90 mm², optionally between about 70 mm² and about 80 mm², optionally about 20 mm², about 25 mm², about 30 mm², about 35 mm², about 40 mm², about 45 mm², about 50 mm², about 55 mm², about 60 mm², about 65 mm², about 70 mm², about 75 mm², about 80 mm², about 85 mm², about 90 mm², about 95 mm², about 100 mm², about 105 mm², about 110 mm², about 115 mm², about 120 mm², about 125 mm², about 130 mm², or any cross-sectional area between any two of those cross-sectional areas.

In some configurations, a ratio of the inner cross-sectional area of the first prong to the inner cross-sectional area of the second prong is between about 60:40 and about 80:20; optionally between about 65:35 and about 80:20; optionally between about 70:30 and about 80:20; optionally between about 70:30 and about 75:25; optionally about 70:30, about 71:29, about 72:28, about 73:27, about 74:26, or about 75:25; optionally between about 75:25 and 80:20; optionally about 75:25, about 76:24, about 77:23, about 78:22, about 79:21, or about 80:20.

In some configurations, the inner diameters and/or inner cross-sectional areas of the first prong and the second prong are measured along the same plane (i.e. a common plane).

In some configurations, the direction transverse to gases flow is substantially perpendicular or normal to gases flow through the respective prong.

In some configurations, the inner diameters and/or inner cross-sectional areas are at the first prong outlet and the second prong outlet.

In some configurations, the nasal interface is configured such that at least about 60% of a total volumetric flow rate of gases flow into the gases inlet is delivered out of the nasal interface through the first prong, optionally such that between about 60% and about 90% of the total volumetric flow rate of gases flow into the gases inlet is delivered out of the nasal interface through the first prong, optionally such that between about 60% and about 80% of the total volumetric flow rate of gases flow into the gases inlet is delivered out of the nasal interface through the first prong, optionally such that between about 65% and about 80% of the total volumetric flow rate of gases flow into the gases inlet is delivered out of the nasal interface through the first prong, optionally such that between about 70% and about 80% of the total volumetric flow rate of gases flow into the gases inlet is delivered out of the nasal interface through the first prong, optionally such that between about 70% and about 75% of the total volumetric flow rate of gases flow into the gases inlet is delivered out of the nasal interface through the first prong, optionally such that about 70% of the total volumetric flow rate of gases flow into the gases inlet is delivered out of the nasal interface through the first prong, optionally such that between about 75% and about 80% of the total volumetric flow rate of gases flow into the gases inlet is delivered out of the nasal interface through the first prong, optionally such that about 75% of the total volumetric flow rate of gases flow into the gases inlet is delivered out of the nasal interface through the first prong, optionally such that about 80% of the total gases flow into the gases inlet is delivered out of the nasal interface through the first prong.

In some configurations, the nasal interface is configured such that about 7 lpm is delivered out of the nasal interface through the first prong at a volumetric flow rate of about 9.5 lpm at the gases inlet and/or such that about 13.5 lpm is delivered out of the nasal interface through the first prong at a volumetric flow rate of about 19 lpm at the gases inlet and/or such that about 21 Ipm is delivered out of the nasal interface through the first prong at a volumetric flow rate of about 29 Ipm at the gases inlet and/or such that about 28 Ipm is delivered out of the nasal interface through the first prong at a volumetric flow rate of about 38.5 lpm at the gases inlet and/or about 35 lpm is delivered out of the nasal interface through the first prong at a volumetric flow rate of about 47.5 lpm at the gases inlet and/or about 44 lpm is delivered out of the nasal interface through the first prong at a volumetric flow rate of about 58 lpm at the gases inlet and/or about 48.5 lpm is delivered out of the nasal interface through the first prong at a volumetric flow rate of about 64 lpm at the gases inlet.

In some configurations, the nasal interface comprises a gases manifold comprising the gases inlet.

In some configurations, the nasal interface comprises a cannula body comprising the first prong and the second prong.

In some configurations, the gases manifold is integral with the cannula body or is separate from and couplable with the cannula body.

In some configurations, the gases inlet is at a side of the gases manifold 120.

In some configurations, the gases manifold comprises one or more internal angled walls to direct the gases flow into the first prong and/or the second prong.

In some configurations, the nasal interface is a non-sealing nasal interface.

In accordance with certain features, aspects and advantages of at least one of the embodiments disclosed herein, a patient interface is disclosed, the patient interface comprising the nasal interface as outlined above or herein.

In some configurations, the patient interface further comprises a headgear to retain the nasal interface against a patient's face.

In some configurations, the patient interface further comprises a tube that is in fluid communication with the gases inlet.

In some configurations, the tube is a breathable tube.

In some configurations, water vapour can pass through a wall of the tube, but liquid water and a bulk flow of gases cannot flow through the wall of the tube.

In some configurations, the gases manifold is integrally formed with the breathable tube or is coupled to the breathable tube.

In some configurations, the patient interface further comprises a tube retention clip.

In a further aspect of the disclosure not necessarily according to the invention, but useful for understanding the invention, in accordance with certain features, aspects and advantages of at least one of the embodiments disclosed herein, a respiratory therapy system is disclosed, the respiratory therapy system comprising:
a respiratory therapy apparatus comprising:
   a controller;
   a blood oxygen saturation sensor;
   an ambient air inlet;
   an oxygen inlet;
   a valve in fluid communication with the oxygen inlet to control a flow of oxygen through the oxygen inlet; and
   a gases outlet;
   wherein the controller is configured to control the valve based on at least one measurement of oxygen saturation from the blood oxygen saturation sensor; and
a patient interface comprising a nasal interface, wherein the nasal interface comprises:
   a first prong and a second prong that are asymmetrical to each other;
   and a gases manifold comprising a gases inlet, wherein the first prong and the second prong are in fluid communication with the gases inlet;
   wherein the nasal interface is configured to cause an asymmetrical flow of gases at a patient's nares.

The first prong and the second prong are asymmetrical to each other and/or are not symmetrical to each other and/or differ in shape and configuration to each other and/or are asymmetrical when compared to each other.

In some configurations, the nasal interface comprises a cannula body comprising the first prong and the second prong.

In some configurations, the gases manifold is integral with the cannula body or is separate from and couplable with the cannula body.

In some configurations, the first and second prongs are configured to engage with the nasal passages in an unsealed manner.

In some configurations, the first and second prongs allow exhaled gases to escape around the first and second prongs.

In some configurations, the first and second prongs are configured to provide gases to the patient without interfering with the patient's spontaneous respiration.

In some configurations, the nasal interface is as outlined above or herein.

In some configurations, the respiratory therapy apparatus comprises a flow generator and a humidifier.

In some configurations, the respiratory therapy system comprises a patient conduit with a heater.

In some configurations, the patient interface comprises a breathable tube that is in fluid communication with the gases inlet, and wherein the patient interface further comprises a headgear to retain the nasal interface against a patient's face.

In some configurations, water vapour can pass through a wall of the tube, but liquid water and a bulk flow of gases cannot flow through the wall of the tube.

In some configurations, the gases manifold is integrally formed with the breathable tube or is coupled to the breathable tube.

In some configurations, the patient interface further comprises a tube retention clip.

In some configurations, the patient interface is as outlined above or herein.

In a further aspect of the disclosure not necessarily according to the invention, but useful for understanding the invention, in accordance with certain features, aspects and advantages of at least one of the embodiments disclosed herein, a respiratory therapy system is disclosed, the respiratory therapy system comprising:
a respiratory therapy apparatus comprising:
   a gases inlet;
   a gases outlet;
   a nebulizer to deliver one or more substances into a gases flow; and
a patient interface comprising a nasal interface, wherein the nasal interface comprises:
   a first prong and a second prong that are asymmetrical to each other;
   a gases manifold comprising a gases inlet, wherein the first prong and the second prong are in fluid communication with the gases inlet, wherein the gases inlet is in fluid communication with the gases outlet to receive gases and the one or more substances from the respiratory therapy apparatus;
   wherein the nasal interface is configured to cause an asymmetrical flow of gases at a patient's nares.

The first prong and the second prong are asymmetrical to each other and/or are not symmetrical to each other and/or differ in shape and configuration to each other and/or are asymmetrical when compared to each other.

In some configurations, the nasal interface comprises a cannula body comprising the first prong and the second prong.

In some configurations, the gases manifold is integral with the cannula body or is separate from and couplable with the cannula body.

In some configurations, the first and second prongs are configured to engage with the nasal passages in an unsealed manner.

In some configurations, the first and second prongs allow exhaled gases to escape around the first and second prongs.

In some configurations, the first and second prongs are configured to provide gases to the patient without interfering with the patient's spontaneous respiration.

In some configurations, the respiratory therapy system comprises a conduit to receive the gases and the one or more substances from the respiratory therapy apparatus and deliver the gases and the one or more substances to the gases inlet of the nasal interface.

In some configurations, the conduit comprises a smooth bore heating tube.

In some configurations, the nasal interface is as outlined above or herein.

In some configurations, the patient interface is as outlined above or herein.

In a further aspect of the disclosure not necessarily according to the invention, but useful for understanding the invention, in accordance with certain features, aspects and advantages of at least one of the embodiments disclosed herein, a respiratory therapy system is disclosed, the respiratory therapy system comprising:
a respiratory therapy apparatus comprising:
   at least one gases inlet;
   a humidifier to humidify gases; and
   a gases outlet;
and a patient interface comprising a nasal interface, wherein the nasal interface comprises:
   a first prong and a second prong that are asymmetrical to each other, and wherein the first prong has a first prong outlet and the second prong has a second prong outlet;
   and a gases manifold comprising a gases inlet, wherein the first prong and
   the second prong are in fluid communication with the gases inlet;
   wherein the nasal interface is configured to cause an asymmetrical flow of
   gases at a patient's nares;
wherein the respiratory therapy system is configured to deliver gases through the first prong outlet and the second prong outlet at a temperature range of between about 27°C - 37°C, at a relative humidity of greater than about 33 mg/l, and/or at a velocity of more than 0 m/s and less than about 32 m/s for a total volumetric flow rate of gases flow into the gases inlet of more than 0 lpm and up to about 70 lpm.

The first prong and the second prong are asymmetrical to each other and/or are not symmetrical to each other and/or differ in shape and configuration to each other and/or are asymmetrical when compared to each other.

In some configurations, the respiratory therapy system is configured to deliver gases through the first prong outlet and the second prong outlet at a temperature range of between about 31°C - 37°C.

In some configurations, the respiratory therapy system is configured to deliver gases through the first prong outlet and the second prong outlet with a relative humidity of up to about 44 mg/l.

In some configurations, the respiratory therapy system is configured to provide a total volumetric flow rate of gases flow into the gases inlet of at least about 5 liters per minute (lpm), optionally of between about 5 lpm and about 120 Ipm, and optionally of between about 5 lpm and about 70 lpm.

In some configurations, the respiratory therapy system is configured to deliver at least about 60% of a total volumetric flow rate of gases flow into the gases inlet out of the nasal interface through the first prong, optionally between about 60% and about 90% of the total volumetric flow rate of gases flow into the gases inlet out of the nasal interface through the first prong, optionally between about 60% and about 80% of the total volumetric flow rate of gases flow into the gases inlet out of the nasal interface through the first prong, optionally between about 65% and about 80% of the total volumetric flow rate of gases flow into the gases inlet out of the nasal interface through the first prong, optionally between about 70% and about 80% of the total volumetric flow rate of gases flow into the gases inlet out of the nasal interface through the first prong, optionally between about 70% and about 75% of the total volumetric flow rate of gases flow into the gases inlet out of the nasal interface through the first prong, optionally about 70% of the total volumetric flow rate of gases flow into the gases inlet out of the nasal interface through the first prong, optionally between about 75% and about 80% of the total volumetric flow rate of gases flow into the gases inlet out of the nasal interface through the first prong, optionally about 75% of the total volumetric flow rate of gases flow into the gases inlet out of the nasal interface through the first prong, optionally about 80% of the total volumetric flow rate of gases flow into the gases inlet out of the nasal interface through the first prong.

In some configurations, the respiratory therapy system is configured to provide different flow rates of gases through the first prong and the second prong and to deliver a substantially similar velocity of gases through the first prong outlet and the second prong outlet.

In some configurations, the velocity of gases exiting the first prong outlet is within about 20% of the velocity of gases exiting the second prong outlet, optionally within about 16% of the velocity of gases exiting the second prong outlet, and optionally within about 10% of the velocity of gases exiting the second prong outlet at flow rates above about 40 Ipm, and optionally within about 10% of the velocity of gases existing the second prong outlet of flow rates above about 42 lpm.

In some configurations, the velocity of gases exiting each of the first prong outlet and the second prong outlet is more than 0 m/s and less than 32 m/s for a total volumetric flow rate of gases flow into the gases inlet of more than 0 lpm and up to about 70 lpm.

In some configurations, the velocity of gases exiting each of the first prong outlet and the second prong outlet is more than about 2 m/s and less than about 32 m/s, optionally more than about 2 m/s and less than 32 m/s, optionally more than about 2 m/s and up to about 25 m/s, and optionally more than about 2.5 m/s and up to about 20 m/s for a total volumetric flow rate of gases flow into the gases inlet of more than 9 Ipm and up to about 70 lpm.

In some configurations, the nasal interface comprises a cannula body comprising the first prong and the second prong.

In some configurations, the gases manifold is integral with the cannula body or is separate from and couplable with the cannula body.

In some configurations, the first and second prongs are configured to engage with the nasal passages in an unsealed (non-sealing) manner.

In some configurations, the first and second prongs allow exhaled gases to escape around the first and second prongs.

In some configurations, the first and second prongs are configured to provide gases to the patient without interfering with the patient's spontaneous respiration.

In some configurations, the first and second prongs are configured to provide gases to the patient independent of the patient's respiration.

In some configurations, the respiratory therapy system comprises a conduit to receive the gases from the respiratory therapy apparatus and deliver the gases to the gases inlet of the nasal interface.

In some configurations, the conduit comprises a smooth bore heating tube.

In some configurations, the nasal interface is as outlined above or herein.

In a further aspect of the disclosure not necessarily according to the invention, but useful for understanding the invention, in accordance with certain features, aspects and advantages of at least one of the embodiments disclosed herein, a method of providing respiratory support to a patient is provided, the method comprising:
providing a respiratory therapy system comprising:
   a respiratory therapy apparatus comprising:
      at least one gases inlet;
      a flow generator; and
      a gases outlet;
   and a patient interface comprising a nasal interface, wherein the nasal interface comprises:
      a first prong and a second prong that are asymmetrical to each other, through the first prong outlet and the second prong outlet;
      and a gases manifold comprising a gases inlet, wherein the first prong and the second prong are in fluid communication with the gases inlet;
operating the respiratory therapy apparatus to provide a flow of gases to the nasal interface; and
delivering an asymmetrical flow of gases from the respiratory therapy apparatus through the first prong outlet and the second prong outlet at a patient's nares.

The first prong and the second prong are asymmetrical to each other and/or are not symmetrical to each other and/or differ in shape and configuration to each other and/or are asymmetrical when compared to each other.

In some configurations, the method comprises delivering the asymmetrical flow of gases at a temperature range of between about 27°C - 37°C, at a relative humidity of greater than about 33 mg/l, and/or at a velocity of more than 0 m/s and less than about 32 m/s for a total volumetric flow rate of gases flow into the gases inlet of more than 0 lpm and up to about 70 lpm.

In some configurations, the method comprises delivering the asymmetrical flow of gases at a temperature range of between about 31°C - 37°C.

In some configurations, the method comprises providing a total volumetric flow rate of gases flow into the gases inlet of at least about 5 liters per minute (lpm), optionally providing a total volumetric flow rate of gases flow into the gases inlet of between about 5 lpm and about 120 Ipm, and optionally providing a total volumetric flow rate of gases flow into the gases inlet of between about 5 lpm and about 70 lpm.

In some configurations, the method comprises delivering at least about 60% of a total volumetric flow rate of gases flow into the gases inlet out of the nasal interface through the first prong, optionally delivering between about 60% and about 90% of the total volumetric flow rate of gases flow into the gases inlet out of the nasal interface through the first prong, optionally delivering between about 60% and about 80% of the total volumetric flow rate of gases flow into the gases inlet out of the nasal interface through the first prong, optionally delivering between about 65% and about 80% of the total volumetric flow rate of gases flow into the gases inlet out of the nasal interface through the first prong, optionally delivering between about 70% and about 80% of the total volumetric flow rate of gases flow into the gases inlet out of the nasal interface through the first prong, optionally delivering between about 70% and about 75% of the total volumetric flow rate of gases flow into the gases inlet out of the nasal interface through the first prong, optionally delivering about 70% of the total volumetric flow rate of gases flow into the gases inlet out of the nasal interface through the first prong, optionally delivering between about 75% and about 80% of the total volumetric flow rate of gases flow into the gases inlet out of the nasal interface through the first prong, optionally delivering about 75% of the total volumetric flow rate of gases flow into the gases inlet out of the nasal interface through the first prong, optionally delivering about 80% of the total volumetric flow rate of gases flow into the gases inlet out of the nasal interface through the first prong.

In some configurations, the method comprises delivering gases through the first prong outlet and the second prong outlet with a relative humidity of up to about 44 mg/l.

In some configurations, the method comprises providing different flow rates of gases through the first prong and the second prong and delivering a substantially similar velocity of gases through the first prong outlet and the second prong outlet.

In some configurations, the velocity of gases exiting the first prong outlet is within about 20% of the velocity of gases exiting the second prong outlet, optionally within about 16% of the velocity of gases exiting the second prong outlet, and optionally within about 10% of the velocity of gases exiting the second prong outlet at flow rates above about 42 lpm.

In some configurations, the velocity of gases exiting each of the first prong outlet and the second prong outlet is more than 0 m/s and less than 32 m/s for a total volumetric flow rate of gases flow into the gases inlet of more than 0 lpm and up to about 70 lpm.

In some configurations, the velocity of gases exiting each of the first prong outlet and the second prong outlet is more than about 2 m/s and less than about 32 m/s, optionally more than about 2 m/s and less than 32 m/s, optionally more than about 2 m/s and up to about 25 m/s, and optionally more than about 2.5 m/s and up to about 20 m/s for a total volumetric flow rate of gases flow into the gases inlet of more than 9 lpm and up to about 70 lpm.

In some configurations, the nasal interface comprises a cannula body comprising the first prong and the second prong.

In some configurations, the gases manifold is integral with the cannula body or is separate from and couplable with the cannula body.

In some configurations, the method comprises engaging the first and second prongs with the nasal passages in an unsealed (non-sealing) manner.

In some configurations, the method comprises allowing exhaled gases to escape around the first and second prongs.

In some configurations, the method comprises providing gases to the patient without interfering with the patient's spontaneous respiration.

In some configurations, the method comprises providing gases to the patient independent of the patient's respiration.

In some configurations, the nasal interface is as outlined above or herein.

In some configurations, the respiratory therapy apparatus comprises a humidifier, and the method comprises humidifying the flow of gases using the humidifier.

In some configurations, the respiratory therapy system comprises a patient conduit with a heater and the method comprises operating the heater.

In some configurations, the patient interface comprises a breathable tube that is in fluid communication with the gases inlet, and the method comprises allowing water vapour to pass through a wall of the tube, but preventing liquid water and a bulk flow of gases from flowing through the wall of the tube.

Features from one or more embodiments or configurations may be combined with features of one or more other embodiments or configurations. Additionally, more than one embodiment or configuration may be used together in a respiratory support system during a process of respiratory support of a patient.

As used herein the term "(s)" following a noun means the plural and/or singular form of that noun.

As used herein the term "and/or" means "and" or "or", or where the context allows both.

The term "comprising" as used in this specification means "consisting at least in part of". When interpreting each statement in this specification that includes the term "comprising", features other than that or those prefaced by the term may also be present. Related terms such as "comprise" and "comprises" are to be interpreted in the same manner.

It is intended that reference to a range of numbers disclosed herein (for example, 1 to 10) also incorporates reference to all rational numbers within that range (for example, 1, 1.1, 2, 3, 3.9, 4, 5, 6, 6.5, 7, 8, 9 and 10) and also any range of rational numbers within that range (for example, 2 to 8, 1.5 to 5.5 and 3.1 to 4.7) and, therefore, all sub-ranges of all ranges expressly disclosed herein are hereby expressly disclosed. These are only examples of what is specifically intended and all possible combinations of numerical values between the lowest value and the highest value enumerated are to be considered to be expressly stated in this application in a similar manner.

This disclosure may also be said broadly to consist in the parts, elements and features referred to or indicated in the specification of the application, individually or collectively, and any or all combinations of any two or more said parts, elements or features.

Where specific integers are mentioned herein which have known equivalents in the art to which this disclosure relates, such known equivalents are deemed to be incorporated herein as if individually set forth.

The disclosure consists in the foregoing and also envisages constructions of which the following gives examples only.

### BRIEF DESCRIPTION OF THE DRAWINGS

Specific embodiments and modifications thereof will become apparent to those skilled in the art from the detailed description herein having reference to the figures that follow, of which:
Figure 1A is a front left perspective view of an exemplary configuration patient interface of the present disclosure comprising a nasal interface with asymmetrical nasal delivery elements.
Figure 1B is a front right perspective view of the patient interface.
Figure 1C is a front left exploded perspective view of the patient interface.
Figure 2 shows the nasal interface, where Figure 2(a) is a top view, Figure 2(b) is a front view, and Figure 2(c) is a bottom view.
Figure 3 is a front sectional view of a nasal interface of the present disclosure inserted into the nares of a user.
Figure 4A is a rear view of a small size nasal interface of the present disclosure.
Figure 4B is a rear view of a medium size nasal interface of the present disclosure.
Figure 4C is a rear view of a large size nasal interface of the present disclosure.

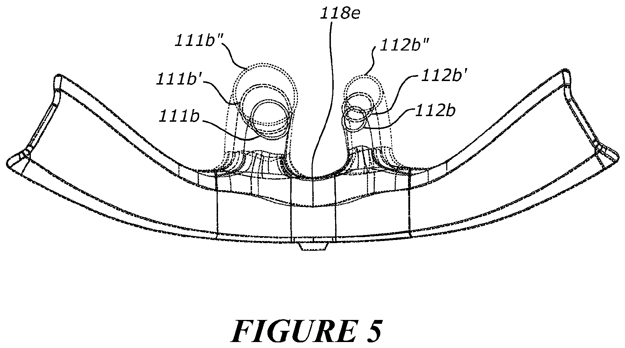

Figure 5 is a rear view of the small, medium, and large size nasal interfaces overlaid on one another.
Figure 6 shows the results of desktop testing of the nasal interfaces, where Figures 6(a), 6(b), and 6(c) show dead space clearance for larger upper airways at 25, 35, and 45 breaths per minute respectively, and Figures 6(d) and 6(e) show dead space clearance for smaller upper airways at 15 and 25 breaths per minute respectively, where I:E is the ratio of inspiratory time to expiratory time.
Figure 7 shows the results of testing of the nasal interfaces, where Figure 7(a) shows results for the Optiflow^{™}+ OPT944+ nasal interface from Fisher & Paykel Healthcare Limited, Figure 7(b) shows results for the nasal interfaces of the present disclosure, and Figure 7(c) shows comparative results.
Figure 8 shows exemplary septum spacings and nasal prong heights for the (a) small nasal interface, (b) medium nasal interface, and (c) large nasal interface of the present disclosure.
Figure 9 shows an exemplary gases manifold for use in the small nasal interface, where Figure 9(a) shows a top view and Figure 9(b) shows a front sectional view along line b-b of Figure 9(a).
Figure 10 shows an exemplary gases manifold for use in the medium or large nasal interface, where Figure 10(a) shows a top view and Figure 10(b) shows a front sectional view along line b-b of Figure 10(a).
Figure 11 shows the effects of prong orientation relative to gases inlet for the nasal interface of the present disclosure.
Figure 12 shows possible configurations of the gases manifold relative to the cannula body, where Figure 12(a) shows a first insertion direction of the gases manifold into the cannula body and Figure 12(b) shows the gases manifold coupled to the cannula body in a first configuration, and wherein Figure 12(c) shows a second insertion direction of the gases manifold into the cannula body and Figure 12(d) shows the gases manifold coupled to the cannula body in a second configuration.
Figure 13 shows details of prong geometry of the outlets of the nasal prongs of the nasal interface of the present disclosure.
Figure 14 shows details of the terminal ends of the prongs of the nasal interface of the present disclosure, where Figure 14(a) shows a left side sectional view of the nasal interface showing an exemplary geometry of the outlet of the large nasal prong, Figure 14(b) shows a right side sectional view of the nasal interface showing an exemplary geometry of the outlet of the small nasal prong, and Figure 14(c) shows a comparison of the outlet geometries.
Figure 15 shows a respiratory therapy system incorporating the patient interface and nasal interface of the present disclosure.
Figure 16 shows a control loop of the respiratory therapy system for closed loop blood oxygen saturation (SpO2) control.
Figure 17 shows an alternative respiratory therapy system incorporating the patient interface and nasal interface of the present disclosure.
Figure 18 shows a sectional view of a patient conduit that can be used in the respiratory therapy systems and/or with the nasal interfaces of the present disclosure.
Figure 19 shows a sectional view of an alternative patient conduit that can be used in the respiratory therapy systems and/or with the nasal interfaces of the present disclosure.

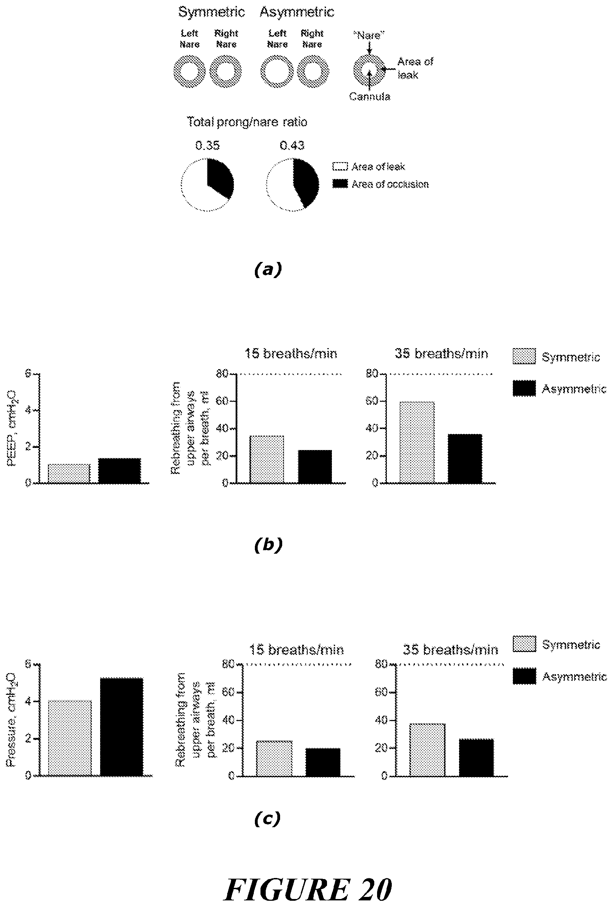

Figure 20 shows the results of testing of the nasal interfaces, where Figure 20(a) shows how a nasal interface of the present disclosure can be used to achieve an increased area of occlusion while still maintaining a safe clearance in one naris, Figure 20(b) shows test data showing increased positive-end expiratory pressure (PEEP) and reduced rebreathing when using a nasal interface of the present disclosure with asymmetric prongs vs a nasal interface with symmetric prongs when nasal high flow of 30 liters per minute is applied, and Figure 20(c) shows similar test data to Figure 20(b) but for nasal high flow of 60 liters per minute.
Figure 21 shows the maximum airway pressure that can be achieved for each size of nasal interface of the present disclosure when the larger prong fully occludes one of the patient's nares.
Figures 22A and 22B are schematic cross-sectional views of exemplary configurations for single walled breathable patient conduits.

### DETAILED DESCRIPTION

Patient interfaces can be used for delivering breaking gases to airways of a patient. The patient interfaces may comprise nasal interfaces that can be used to deliver a high flow of gases to a patient. Nasal delivery elements, such as nasal prongs which may optionally comprise nasal pillows, are inserted into the nose of a patient to deliver the required therapy. The nasal delivery elements may be desired to seal or partially occlude at the nose, or may not be required to seal at the nose, to deliver the therapy. Nasal prongs typically refer to nasal delivery elements designed to not seal or to only partially occlude at the nose. When one or more of the nasal prongs comprises a nasal pillow, the nasal delivery elements are designed to seal at the nose. Nasal high flow (NHF) typically is a non-sealing therapy that delivers relatively high-volume flow to the patient through a patient interface, such as a nasal interface. A nasal interface as herein described may refer to, but is not limited to, a nasal cannula.

Disclosed is a system to deliver gases to a patient through an asymmetrical nasal cannula or nasal interface. An asymmetrical interface or asymmetrical nasal delivery elements, as described herein, refers to an interface where the nasal delivery elements differ in size such as internal and/or external transverse dimensions or diameters, and/or internal and/or external cross-sectional areas. The external cross-sectional area is the cross-sectional area bounded by the outer wall of the nasal delivery element. For non-circular cross-sections, the references herein to a diameter may be interpreted as a transverse dimension. In some configurations, references herein to a diameter include but are not limited to a hydraulic diameter.

The system allows an asymmetrical flow to be delivered through the interface to both nares or to either nare. Asymmetrical flow as described herein refers to a flow that differs within the interface or within the nose or within the interface and the nose. In this way, a different flow may be delivered by each nasal delivery element, or the flow may differ between inspiration and expiration, or the delivered flow may be a combination of the above. An asymmetrical flow may also include partial unidirectional flow.

Delivery of asymmetrical flow may improve clearance of dead space in the upper airways, decrease peak expiratory pressure, increase safety of the therapy particularly for children and infants, and reduce resistance to flow in the interface. An asymmetrical nasal interface and/or nasal delivery elements as described herein includes interfaces or systems configured to produce such asymmetrical flow through asymmetrical nasal delivery elements.

Pressure generated by NHF depends on flow through the nasal interface, the size of the nasal delivery elements and/or nares of the patient, and the breathing cycle. If flow, leak, or a combination of flow and leak, is asymmetrical through the nasal interface, the flow through the nose may become asymmetrical during breathing. Partial and total unidirectional flow may be types of asymmetrical flow. Partial or total unidirectional flow may provide improved clearance of anatomical dead space as the air is continuously flushed from the upper airways. Partial unidirectional flow may be more comfortable than total unidirectional flow. Total unidirectional flow as described herein includes flow entering one nare by a nasal delivery element and exiting via the other nare via a nasal delivery element, venting to the atmosphere, due to the absence of a nasal delivery element, or the like. Partial unidirectional flow as described herein includes flow that may enter the nose via both nares and leave the nose from one nare, flow that may enter the nose through one nare and leave the nose via both nares, or different proportions of flow that may enter the nose through both nares and different proportions of flow that may leave the nose through both nares, and may be flow that may enter the nose via both nares and leave the nose from one or both nares and optionally via the mouth.

NHF delivered through an asymmetrical nasal interface can involve making an interface in which the nasal delivery elements are of different size, e.g. different length and/or internal diameter or cross-sectional area and/or external diameter or cross-sectional area. Particularly for children or infants, nasal delivery elements will have a small internal diameter and thus higher resistance to gas flow. By using nasal delivery elements that are different lengths, each nasal delivery element may have a different internal diameter (e.g., minimum internal diameter or area). A longer nasal delivery element may have a smaller internal diameter and higher resistance to gas flow; a shorter nasal delivery element may have a larger internal diameter (e.g., larger minimum internal diameter), hence lower resistance to gas flow at the interface. A decreased resistance to flow allows the desired flow to be achieved using lower backpressure, or a lower motor speed of the gas generating device, or a combination of the two.

Asymmetrical nasal delivery elements may cause the peak expiratory pressure to decrease due to the different cross-sectional areas of the nasal delivery elements at the nose which may provide different internal diameters for each nasal delivery element.

The pressure when exhaling against the asymmetric nasal interface may be higher than with a symmetric one, which is beneficial as higher positive-end expiratory pressure (PEEP) is part of the treatment for COPD (pressure here referring to the intrathoracic pressure). Expiratory pressure is dependent on the combined cross-sectional area of the two prongs. Increasing the cross-section of symmetric prongs carries the risk of fully occluding the patient's nares. Using asymmetric prongs allows for an increase in total cross-sectional area without the accompanying occlusion risk. The partially unidirectional flow may reduce turbulence in the patient's nasal cavity, which could improve comfort.

In an example, an asymmetrical nasal interface used with (e.g., coupled via a conduit or breathing tube) a gas generating device, such as an AIRVO^{™} flow generator from Fisher & Paykel Healthcare Limited, decreases the resistance to flow. This may cause the motor speed of the AIRVO^{™} to drop from a range of 18,000 - 22,000 RPM to a range of 14,000 - 18,000 RPM while continuing to achieve a suitable flow for the desired therapy (e.g., NHF), such as about 8 liters per minute (lpm). The asymmetrical nasal delivery elements may cause a reduction of the backpressure generated in the system if, for example, an incorrectly sized prong forms a seal with a patient's nare.

For a smaller patient, as in an infant or a child, use of asymmetrical nasal delivery elements may reduce over-insertion of both prongs into the nares, when the nares are too small with respect to the prongs, which could result in an undesired semi-seal or seal. Asymmetrical flow may be delivered to the patient even if only one prong is positioned tightly in the nose. The asymmetrical interface improves the performance of the therapy for infants as compressed gas may be used in a system without pressure control.

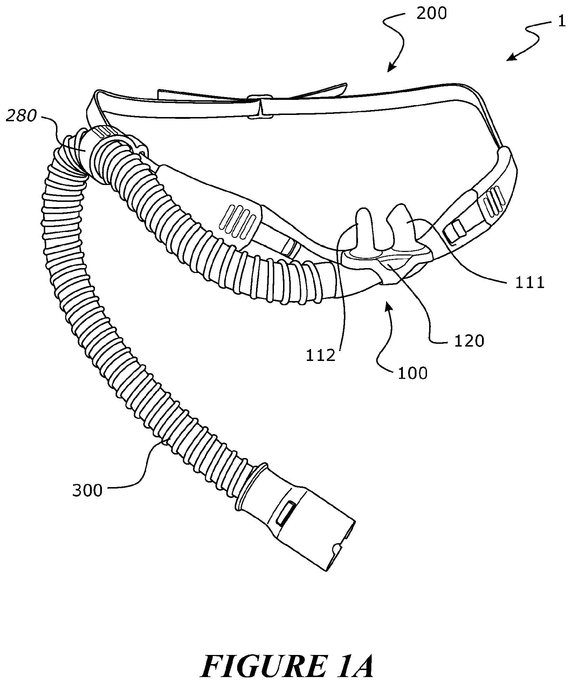

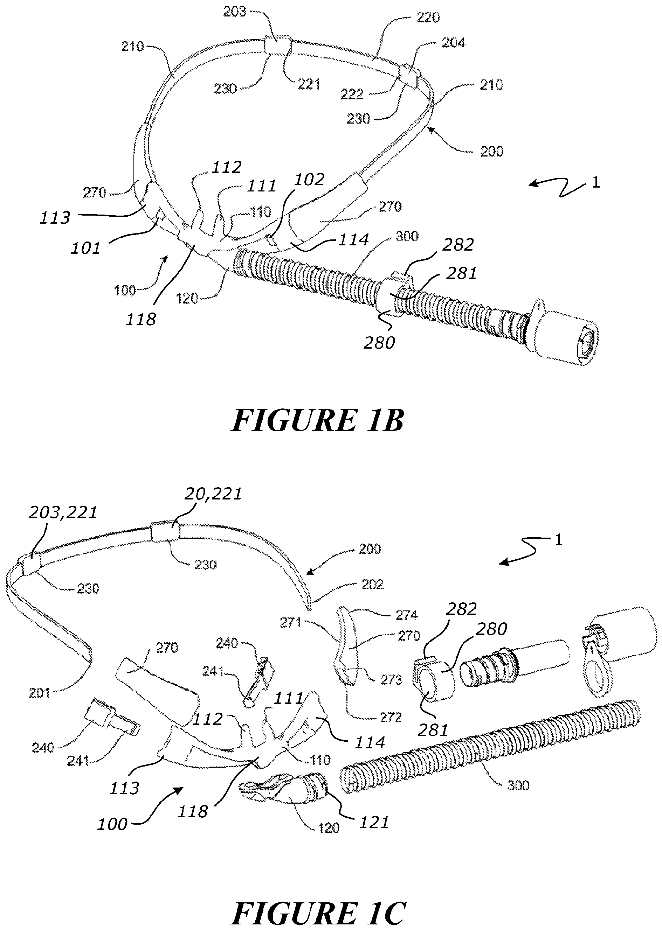

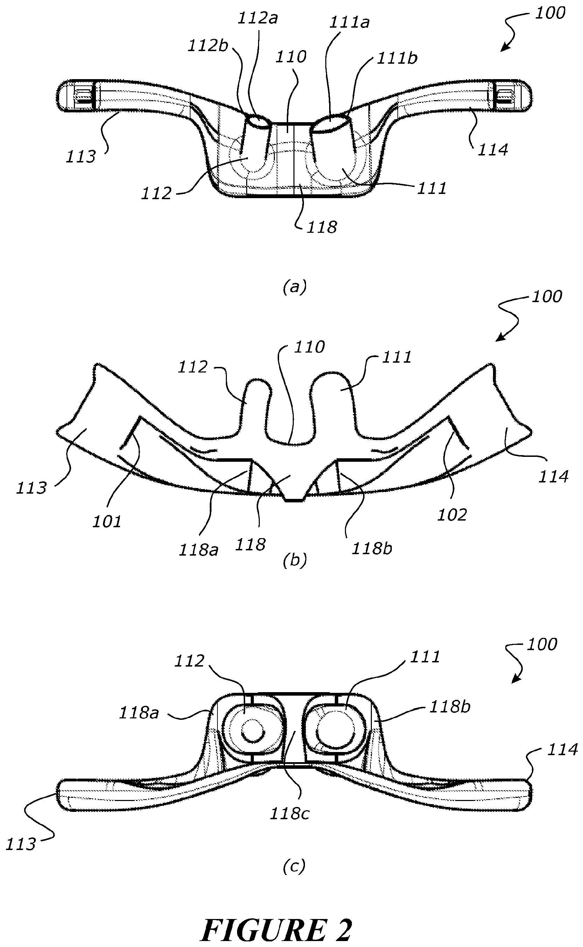

Figures 1A to 1C and Figure 2 show an exemplary patient interface 1 that comprises a nasal cannula or nasal interface 100 with asymmetrical nasal delivery elements 111, 112.

The nasal interface 100 provides a patient with a patient interface suitable for the delivery of high airflow, high humidity gas flow to the patient's nasal cavity/nares. In some configurations, the nasal interface 100 is adapted to deliver a high flow of gases over a wide flow range (e.g. about 8 Ipm, or higher depending on other therapy applications, perhaps such as 10 - 50 lpm or higher). In some configurations, the nasal interface 100 is adapted to deliver relatively low pressure gases.

The nasal interface 100 comprises a face mount part 110 including a pair of asymmetrical tubular nasal prongs 111 and 112, integrally moulded with or removably attached to the face mount part 110, and a gases manifold 120 part that is removably attached or integrally moulded to the conduit 300.

The gases manifold 120 is insertable into the face mount part 110. The face mount part 110 may comprise at least one substantially horizontal side entry passage 118a, 118b to the interior of a base portion or cannula body 118 of the face mount part 110 for releasably receiving the outlet of the gases manifold 120 therethrough.

The gases manifold 120 is optionally insertable into the face mount part 110 from either of two opposing horizontal directions, i.e. from either left side or the right side. In this manner, the position or location of the gases manifold 120 is reconfigurable with respect to the face mount part 110. In other words, a user may choose to have the manifold part 120 (and the conduit 300 extending therefrom) extend from either the left side or the right side of the face mount part 110 of the nasal interface 100 depending on what is most convenient, for example depending on which side of the user the gas source or ventilator is located. In an alternative configuration, the gases manifold 120 is not reconfigurable with respect to the face mount part 110.

The face mount part 110 may comprise a pair of opposed side entry passages 118a, 118b to the interior of the base portion or cannula body 118, each adapted to releasably receive the outlet of the gases manifold 120 therethrough.

The face mount part 100 is formed from a soft, flexible material such as silicone or other cannula material known in the art. The nasal prongs 111 and 112 are preferably supple and may be formed from a sufficiently thin layer of silicone to achieve this property.

The gases manifold 120 is formed from a relatively harder material such as Polycarbonate, a High-Density Polyethylene (HDPE) or any other suitable plastics material known in the art. The face mount part 110 provides a soft interfacing component to the patient for comfortably delivering the flow of gases through the nasal prongs 111 and 112, while the gases manifold 120 fluidly couples the conduit 300 to the nasal prongs 111 and 112 of the face mount part 110.

The nasal prongs 111 and 112 are curved to extend into the patient's nares in use and to provide a smooth flow path for gases to flow through. The inner surfaces of the prongs 111 and 112 may be contoured to reduce noise. The bases of the prongs 111 and 112 may include curved surfaces to provide for smoother gases flow. This may reduce the noise level during operation.

The nasal prongs 111 and 112 are substantially hollow and substantially tubular in shape.

The nasal prongs 111 and 112 may be consistent in diameter along their lengths or alternatively may be shaped to fit the contours of the nares.

The face mount part 110 is shaped to generally follow the contours of a patient's face around the upper lip area. The face mount part 110 is moulded or preformed to be able to conform to and/or is pliable to adapt, accommodate and/or correspond with the contours of the user's face, in the region of the face where the cannula is to be located.

The asymmetry of the nasal prongs 111 and 112 may reduce the chance of accidental occlusion of both nares. At least one of the nasal prongs 111 and 112 is therefore sized to maintain a sufficient gap between the outer surface of the prongs 111 and 112 and the patient's skin to avoid sealing the gas path between the nasal interface 100 and patient. It should be understood that in the context of the present disclosure, the nasal prongs 111 and 112 are asymmetric, as described below.

The face mount part 110 comprises the base part or cannula body 118 from which the nasal prongs 111 and 112 extend, and two side arms comprising wing portions 113 and 114 extending laterally from either side of the cannula body 118. The wing portions 113 and 114 are integrally formed with the cannula body 118 but may alternatively be separate parts.

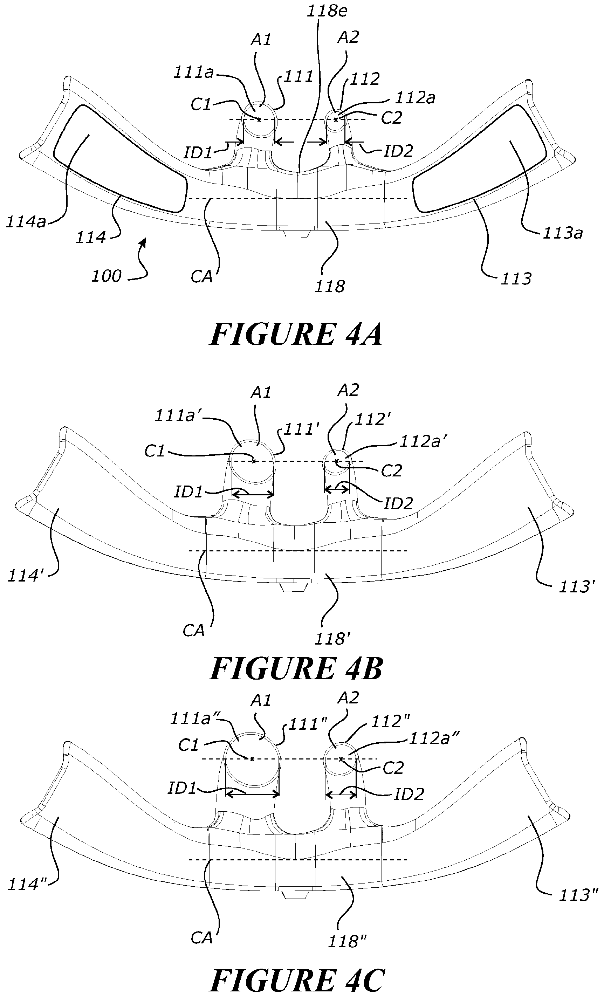

Adhesive pads 113a, 114a (Figure 4A) may be provided on each wing portion 113, 114 to facilitate coupling of the cannula 100 to the patient - especially for younger children (e.g. under 5 years old).

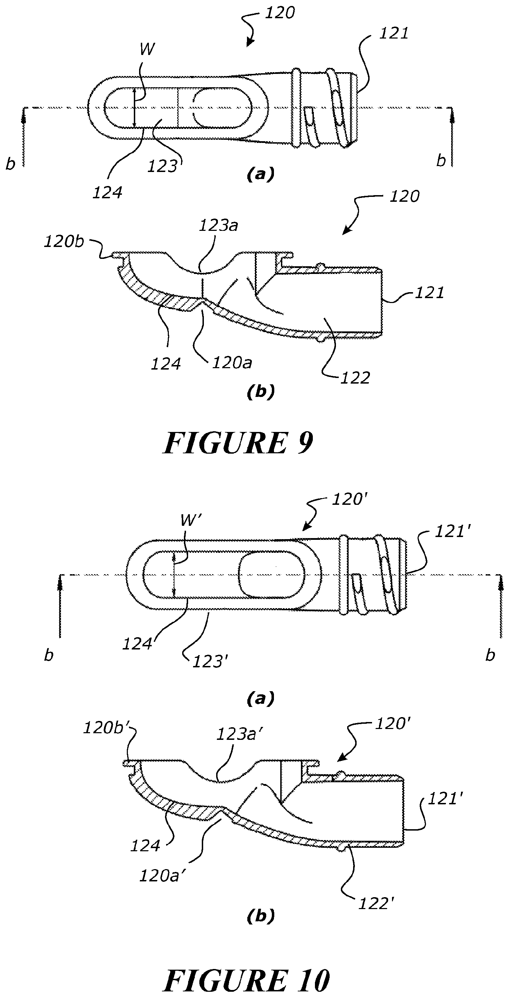

The gases manifold 120 is generally tubular in shape having a substantially annular gases inlet 121 at one end, and that curves around into an elongate oval outlet 123 at the opposing end (Figures 9 and 10). The inlet 121 may be removably attachable to a conduit 300, such as via a threaded engagement but alternatively via a snap-fit or any other type of coupling known in the art. Alternatively, the inlet is fixedly coupled or integrally formed with a conduit 300.

The shape of the outlet 123 corresponds with and fits into the cannula body 118 e.g. with a friction fit or snap fit engagement, such that substantial force, or at least a deliberate force applied by a user or a carer, is required to separate the manifold 120 from the face mount part 110.

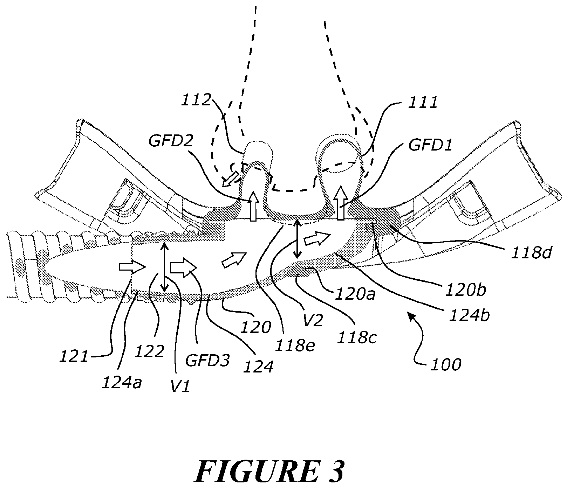

An effective seal is formed between the outlet 123 and the cannula body 118 upon engagement of the two parts 118 and 120. As discussed below, as shown in Figure 3, the gases manifold 120 may comprise a retaining flange 120b around a face thereof which is removably received in a complementary resilient rim 118d of the cannula body 118. The engagement of the retaining flange 120b with the complementary resilient rim 118d of the cannula body 118 assists with forming a seal between the gases manifold 120 and the cannula body 118.

The nasal prongs 111, 112 are aligned with corresponding apertures extending through an upper surface of the cannula body 118 to fluidly connect the manifold outlet 123 with the nasal prongs 111 and 112 when coupled.

A headgear may be used to retain the nasal interface 100 against the patient's face. The headgear comprises a head strap 200. The head strap 200 may be a single continuous length and adapted to extend in use along the patient's cheeks, above the ears and about the back of the head, may be adjustable, and/or may extend around other portions of the patient's head.

In the exemplary configuration shown, primary end portions 201 and 202 of the head strap 200 are adapted to releasably connect to respective formations 101 and 102 on either side of the nasal interface 100 to hold the nasal interface 100 in position during use.

In one configuration, a clip component is provided at each end portion 201, 202 capable of being received and retained within the corresponding formation 101, 102. The clip component may be coupled to the strap at the respective primary end portion. Furthermore, the head strap 200 is adjustable in length to help customise the strap to the wearer's head. The strap 200 may be formed from a soft and stretchable/elastic material such as an elastic, textile material/fabric that is comfortable to the wearer. Alternatively, the strap 200 may be formed from a substantially more rigid, or less flexible, material such as a hard plastics material.

The headgear may further comprise an additional strap or other headgear component that couples the strap 200 to extend over the patient's crown in use. A crown strap or crown component can have the benefit of pulling the strap 200 up and above the patient's ears in use to improve fit and comfort.

Generally, but also with reference to Figures 1A to 1C, in one exemplary configuration of an adjustable strap 200, the adjustment mechanism is provided in the form of one or more insertable/removable strap segments or strap extensions 220.

Strap segments 220 of a fixed length can be releasably connected to the main strap 210 to extend its length. The main strap 210 in this configuration comprises a pair of intermediate or secondary end portions 203, 204 that are releasably connectable with one another, and that are also releasably connectable with respective ends 221 and 222 of the strap segments 220. When the secondary end portions 203 and 204 are connected to one another, the main strap 210 is of a continuous starting length/size for the wearer. To extend the length of the strap 200 beyond this starting length, the main strap 210 can be disconnected at the secondary end portions 203/204 and one or more additional strap segments 220 are connected therebetween.

A number of strap segments 220 of varying predetermined lengths may be provided to provide alternative adjustment lengths. For example, one or more strap segments 220 may be provided having a length within the range of about 1cm to about 10cm, or within the range of about 2cm to about 6cm. The strap segments 220 have lengths of, for example, about 2cm, about 4cm or about 6cm. It will be appreciated that these examples are not intended to be limiting and the length of each strap segments can be of any size as it is dependent on the user and/or application.

Furthermore, each end 221, 222 of each strap segment 220 may be connectable to a respective end 221, 222 of another strap segment 220 and/or to a respective secondary end portion 203, 204 of the main strap 210 to thereby enable a user to combine one or more strap segments 220 of the same or varying lengths to customise the overall length of the extension as desired.

The additional strap segments may be formed from a soft and stretchable/elastic material such as an elastic, textile material/fabric that are comfortable to the wearer. For example, a tubular knitted type head strap or sections of the head straps 210 may be utilised, particular for comfort over a user's ears.

It will be appreciated that particular comfort may be achieved from a head strap which is able to provide suitable locating of the nasal interface 100 in a relatively stable position on a user's face, yet simultaneously provide for a relatively loose fit or low tension fit about the user's head.

Alternatively, the additional strap segments may be formed from a substantially rigid material such as a hard plastics material.

A strap connector 230 is provided at each of the secondary end portions 203, 204 of the main strap 210 and the respective end portions 203, 204 of the strap segments 220.

Each connector 230 is provided with a strap connection mechanism at one end to couple to the strap material, and a coupling mechanism at an opposing end to releasably couple the respective end of a similar connector 230.

In an alternative, the connector 230 may be various different forms of adjustable buckles suitable for adjusting the length or tension of the head strap sections 210 which hold the patient interface in position about a user's head.

It will also be appreciated that the connector 230 may be located so as to be offset from a mid-point from the rear of a user's head, or may be offset to one side of a user's head. This may be advantageous so as to avoid impinging upon a part of a user's head which may otherwise be, in some positions such as sleeping, uncomfortable for the user.

In yet a further configuration, the strap segments may be of different lengths, so as to be asymmetrically provided or to help be operational with an offset connector 230 position. Further, it may be that of the two strap segments 210, one of those straps may be adjustable in length while the other is not. For example, one strap segment 210 may be of a permanent length or permanently connected to the connector 230.

In an exemplary configuration, the strap connection mechanism may comprise a series of internal teeth located within the body of the connector for establishing a friction fit engagement with the respective end of the strap. A hinged jaw of the body is provided and closes upon the teeth to securely retain the end of the strap upon the teeth. The releasable coupling mechanism at the other end comprises a pair of male and female members, such as a protrusion and aperture respectively, both adapted to connect to corresponding male and female members of a similar connector 230. A lug on the protrusion may couple a recess in the female member to provide a snap-fit engagement between the members. It will be appreciated that in alternative configurations, any other suitable connector configuration may be used to releasably connect the secondary end portions of the strap to one another, and to the end portions of the additional strap segments.

Cannula connectors 240 are provided at the primary end portions 201 and 202 of the main strap 210. These connectors 240 have a similar strap connection mechanism to the strap connectors 230 of the secondary end portions 203 and 204, but include a clip member, such as a push fit clip 241, at an end of the connector 240 opposing the strap ends. The clip 241 is configured to releasably couple to the respective formation 101, 102 at the side of the nasal interface 100. The clip member 241 may be a bendable part, such as a plastic part, that forms a hinged portion relative to the strap. The clip 241 may be preformed to have a curved shape along its length, such as one with an angle between 0 (flat) and 20 degrees for example. In some configurations, the clip 241 may be pre-formed to have a bend. The clip 241 comprises at least two portions that are angled relative to each other. The at least two portions may be positioned at an angle between more than 0 degrees to 20 degrees. That is, the two portions may be about 180 degrees relative to each other, or may differ from 180 degrees by up to 20 degrees. This curve or angle allows the clip 241 to fit the contour of the patient's face in the region of the clip 241.

The nasal interface may comprise sleeves 270. Each sleeve 270 may be pre-formed to have a curved shape along its length, such as one with an angle between 0 degrees (flat) and 20 degrees for example. The curve allows the sleeve to fit the contour of the patient's face or cheek in the region of the sleeve in use. Alternatively, the sleeve 270 may take on the shape of a curved sleeve upon engagement with the primary end portion 201, 202 or connector 240 of the head strap 200.

The sleeve 270 provides a surface region of relatively higher frictional surface material for frictionally engaging with the user's face or facial skin. This surface region is to be positioned for frictional engagement with the facial cheek skin of a user. The surface region is at least localised to the strap or the section of strap which is to be positioned upon the cheeks of a user. The surface region provided with the relatively higher frictional surface material may be of a material that is smooth and comfortable on the skin of the patient. The sleeve 270 or at least the surface region 271 is therefore formed from a relatively softer material than the connector 240.

In one configuration, the surface region 271 or the sleeve 270 is formed from a soft Thermoplastic Elastomer (TPE), but may alternatively be formed from another plastics material such as silicone, or any other biocompatible materials.

The surface region 271 may be a surface of wider surface area more adjacent to the patient interface than the surface area more distant from the patient interface. In one configuration, the sleeve 270 tapers from a relatively wider surface area 273 to a relatively lesser surface area 274 in a direction extending away from a connection point between the connector 240 and the nasal interface 100. The width of the sleeve at the end 273 may be the same or similar to the width of the tapered distal end of the corresponding wing portion 113, 114 of the face mount part 110. This provides a smooth transition between the nasal interface 100 and the headgear for improving aesthetics and achieving a visually appealing effect.

The sleeves 270 may be coloured to provide an identification of the nasal interface 100. As described herein, the nasal interfaces may be provided in different sizes such as small, medium, and large, for example. The sleeves 270 of each of those sizes may comprise different colours to represent the different sizes. Alternatively, or additionally, the sleeves may be coloured in a specific way to represent that the nasal interfaces have asymmetrical nasal delivery elements rather than symmetrical.

Headgear for other forms of interface in addition to nasal cannula may comprise cheek supports 270 as described or similar, at or adjacent either side end of straps of headgear of the interface, which connect to the nasal interface, for frictionally engaging with the user's face to stabilise the mask on the face at the cheeks. Such headgear may again comprise a single head strap adapted to extend in use along the patient's cheeks, above the ears and about the back of the head, with ends comprising clips in any suitable form which couple to the nasal interface on either side (or are permanently attached to the nasal interface).

Referring to Figures 1A-1C, in the configuration shown, the patient interface 1 comprises a tube retention clip 280. The tube retention clip 280 can support the patient conduit 300 or other gases supply tube from part of the patient interface 1. By supporting the patient conduit 300 or other gases supply tube from or near the nasal interface 100, bending moment applied to the patient conduit 300 or other gases supply tube 300 as a result of asymmetrical flow through the first and second prongs 111, 112 and/or movement of the patient's head will be resisted by the tube retention clip 280, thereby enhancing patient comfort.

In the configuration shown, the tube retention clip 280 comprises a tubular body 281 for receiving and accommodating a portion of the patient conduit 300 or other gases supply tube therein.

In the configuration shown, the tube retention clip 280 supports the patient conduit 300 or other gases supply tube from the head gear of the patient interface. In an alternative configuration, the tube retention clip 280 could support the patient conduit 300 or other gases supply tube from part of the nasal interface 100 of the patient interface. For example, the tube retention clip 280 could support the patient conduit 300 or other gases supply tube from the cannula body 118 or another part of the face mount part 110. In some configurations, the tube retention clip 280 could support the patient interface from one or either of the wing portions 114, 115 of the nasal interface 100.

A hook 282 projects from the body 281 to couple the strap or other component of the headgear. In this manner the conduit 300 can be coupled or tethered to the head strap 210 or headgear in use. If the conduit 300 is pulled, the force will be exerted onto the head strap 210 and not directly on the cannula 100. This relocation of force will reduce the likelihood of the prongs 111 and 112 of the nasal interface 100 flicking out of the patient's nostrils.

A protrusion or bump is provided at or near the free end of the hook 282. The protrusion extends inwardly toward the body 281. The protrusion or bump narrows the gap at the entrance of the hook which helps to retain the clip on a strap when the hook is engaged; i.e. the strap does not slide out of the hook channel. This also provides the advantage of the hook being retained on the strap when it is hooked in a bottom-up direction - the protrusion or bump retains the hook on the strap against gravity.

One or more tethering points for connecting the tube retention clip 280 may be available on the headgear, with preferably at least two symmetric tethering points on either side of the headgear to increase usability.

It will also be appreciated the tube retention clip 280 may be removable from or may be a permanent fitting on the patient conduit 300 or other gases supply tube.

The tube retention clip 280 could have any suitable form. In an alternative configuration, the tube retention clip 280 may comprise or consist of a band or loop. The loop may comprise a fabric, elastomeric, or textile band or loop.

The retention clip 280 may be connected or retained to a part of the patient interface 1, such as for example a part of an interface which provides for a relatively more rigid region (such as to facilitate support of the patient conduit 300). The retention clip may also be positioned or affixed at a particular location on the patient conduit 300, for example a predetermined location may be provided which holds the retention clip in place.

The patient interface 1 may have any one or more of the features and functionality described in PCT publication no. WO 2014/182179 or US patent no. 10,406,311.

As an alternative to a headgear, the patient interface may comprise a securement system of the type described in PCT publication number WO 2012/053910 or US patent no. 10,238,828.

Referring to Figures 1C and 2 to 3, in some configurations a nasal interface 100 of the present disclosure comprises a first prong 111 and a second prong 112 that are asymmetrical to each other, and a gases manifold 120 comprising a gases inlet 121. The first prong 111 and the second prong 112 are in fluid communication with the gases inlet 121. The nasal interface is configured such that at least about 60% of a total volumetric flow rate of gases flow into the gases inlet 121 is delivered out of the nasal interface through the first prong 111.

The gases inlet 121 may be at a side of the gases manifold 120. In alternative configuration, the gases inlet 121 may be at a different location on the gases manifold 120. For example, the gases inlet 121 may enter the front of the gases manifold 120, at or near a centre of the gases manifold 120 or at or near one side of the gases manifold 120.

This may change based on a patient's breathing cycle and internal nasal geometry. The figures and proportions herein are when the nasal interface isn't being worn and before any influence from the patient's respiration and/or nasal geometry.

By way of example, if a blower of a respiratory therapy apparatus is generating flow of 100 liters per minute (Ipm) and that is delivered into the gases inlet 121, at least about 60 lpm would pass through the first prong 111 and be delivered out of the nasal interface 100 through the first prong 111.

The remainder of the total gases flow is delivered through the second prong 112. In the example above, about 40 lpm or less would pass through the second prong 112 and be delivered out of the nasal interface 100 through the second prong 112. Alternatively, some of the remainder of the total gases flow may be vented to atmosphere rather than being delivered through the first prong 111 or the second prong 112.

The first prong 111 and the second prong can be considered asymmetrical nasal delivery elements.

The first prong 111 and the second prong 112 are asymmetrical to each other and/or are not symmetrical to each other and/or differ in shape and configuration to each other and/or are asymmetrical when compared to each other.

The nasal interface 100 is configured to cause an asymmetrical flow of gases at, into and/or out of a patient's nares.

In some configurations, the nasal interface 100 comprises a cannula body 118 comprising the first prong 111 and the second prong 112.

In some configurations, the gases manifold 120 is integral with the cannula body 118 or is separate from and couplable with the cannula body 118.

In some configurations, the first and second prongs 111, 112 are configured to engage with the nasal passages in an unsealed (non-sealing) manner. In some configurations, at least the second prong 112 is configured to engage with a nasal passage in a non-sealing manner.

In some configurations, the first and second prongs 111, 112 allow exhaled gases to escape around the first and second prongs.

In some configurations, the first and second prongs 111, 112 are configured to provide gases to the patient without interfering with the patient's spontaneous respiration.

The first prong 111 has a first prong outlet 111a defined by an opening at its tip or terminal end 111b for delivery of gases from the first prong 111. Gases delivered through the first prong 111 exit the first prong via the first prong outlet 111a.

The second prong 112 has a second prong outlet 112a defined by an opening at its tip or terminal end 112b for delivery of gases from the second prong 112. Gases delivered through the second prong 112 exit the second prong via the second prong outlet 112a.

Referring to Figures 3 and 4A, in some configurations of a nasal interrace 100, the first prong 111 has a larger inner diameter ID1 and/or a larger inner cross-sectional area A1 in a direction transverse to gases flow GFD1 through the first prong 111 than a corresponding inner diameter ID2 and/or inner cross-sectional area A2 of the second prong 112 in a direction transverse to gases flow GFD2 through the second prong 112.

ID1, ID2, A1 and A2 may be measured at substantially the same location along first prong 111 and second prong 112 (for example, the same distance along the prong length from the base of each prong or from the outlet of each prong). This may be a useful reference for curved and/or angled prongs. In some embodiments, ID1, ID2, A1 and A2 may be measured along the same plane. This may be a useful reference for straight prongs.

In some configurations, the direction transverse to gases flow is substantially perpendicular or normal to gases flow through the respective prong 111, 112. Alternatively, the direction transverse to gases flow could be at an acute or obtuse angle relative to gases flow through the respective prong 111, 112.

The nasal interface 100 is configured to cause an asymmetrical flow of gases at a patient's nares.

The inner diameter ID1, ID2 and/or inner cross-sectional area A1, A2 could be substantially constant along the length of the prongs 111, 112. Alternatively, the inner diameter ID1, ID2 and/or inner cross-sectional area A1, A2 could vary along at least part of the length of the prongs 111, 112. For example, the prongs 111, 112 may taper from a wider dimension at their bases near the cannula body 118 than at their tips or terminal ends 111b, 112b. The inner diameter ID1, ID2 and cross-sectional area A1, A2 of relevance could be at the outlets 111a, 112a of the prongs and/or at the distal portions of the prongs 111, 112 adjacent the outlets 111a, 112a.

The inner surface at the base of each prong 111, 112 may be radiused or chamfered to reduce pressure and velocity drop of gases as the gases change flow direction within the manifold. This can help reduce noise and improve delivery of therapy.

The nasal interface 100 may be configured such that between about 60% and about 90% of the total volumetric flow rate of gases flow into the gases inlet 121 is delivered out of the nasal interface 100 through the first prong 111. The nasal interface may be configured such that between about 60% and about 80% of the total volumetric flow rate of gases flow into the gases inlet 121 is delivered out of the nasal interface 100 through the first prong 111. The nasal interface may be configured such that between about 65% and about 80% of the total volumetric flow rate of gases flow into the gases inlet 121 is delivered out of the nasal interface 100 through the first prong 111. The nasal interface may be configured such that between about 70% and about 80% of the total volumetric flow rate of gases flow into the gases inlet 121 is delivered out of the nasal interface 100 through the first prong 111. The nasal interface may be configured such that between about 70% and about 75% of the total volumetric flow rate of gases flow into the gases inlet 121 is delivered out of the nasal interface 100 through the first prong 111. The nasal interface may be configured such that about 70% of the total volumetric flow rate of gases flow into the gases inlet 121 is delivered out of the nasal interface 100 through the first prong 111.

Having a ratio of flow rates between the prongs 111, 112 of at least about 60:40 has been found sufficient to start seeing the benefits of asymmetrical flow describe below. A ratio of between about 70:30 and about 75:25 is believed to be optimal.

The proportion of the total volumetric flow rate being delivered through each prong 111, 112 can be determined by delivering gases with a known volumetric flow rate to the gases inlet 121 of the nasal interface 100 while the nasal interface is not applied to a patient's nares. The volumetric flow rate exiting each outlet 111a, 112a can be measured by a suitable flow meter or sensor to determine the proportion of the total volumetric flow rate of gases flow into the gases inlet 121 that is exiting the outlet 111a, 112a of each prong 111, 112.

The first prong 111 may have an inner diameter ID1 of between about 4 mm and about 10 mm, optionally between about 5 mm and about 9 mm, optionally between about 6 mm and about 8 mm, optionally about 4 mm, about 5 mm, about 6 mm, about 7 mm, about 8 mm, about 9 mm, about 10 mm, or any diameter between any two of those diameters.

The second prong 112 may have an inner diameter ID2 of between about 2 mm and about 8 mm, optionally between about 3 mm and about 7 mm, optionally between about 4 mm and about 6 mm, optionally about 2 mm, about 3 mm, about 4 mm, about 5 mm, about 6 mm, about 7 mm, about 8 mm, or any diameter between any two of those diameters.

In some configurations, the first prong 111 and/or the second prong 112 has a wall thickness of between about 0.1 mm and about 0.5 mm. Therefore, 2x the wall thickness can be added to the inner diameter values to get the associated outer diameter values.

The nasal interface 100 may be configured such that between about 75% and about 80% of the total gases flow is delivered through the first prong 111.

The nasal interface 100 may be configured such that about 75% of the total gases flow is delivered through the first prong 111.

The nasal interface 100 may be configured such that about 80% of the total gases flow is delivered through the first prong 111.

The first prong 111 may have has an inner cross-sectional area A1 of between about 15 mm² and about 80 mm², optionally between about 20 mm² and about 75 mm², optionally between about 25 mm² and about 70 mm², optionally between about 30 mm² and about 65 mm², optionally between about 35 mm² and about 60 mm², optionally between about 40 mm² and about 55 mm², optionally between about 45 mm² and about 50 mm², optionally about 15 mm², about 16 mm², about 17 mm², about 18 mm², about 19 mm², about 20 mm², about 21 mm², about 22 mm², about 23 mm², about 24 mm², about 25 mm², about 26 mm², about 27 mm², about 28 mm², about 29 mm², about 30 mm², about 31 mm², about 32 mm², about 33 mm², about 34 mm², about 35 mm², about 36 mm², about 37 mm², about 38 mm², about 39 mm², about 40 mm², about 41 mm², about 42 mm², about 43 mm², about 44 mm², about 45 mm², about 46 mm², about 47 mm², about 48 mm², about 49 mm², about 50 mm², about 51 mm², about 52 mm², about 53 mm², about 54 mm², about 55 mm², about 56 mm², about 57 mm², about 58 mm², about 59 mm², about 60 mm², about 61 mm², about 62 mm², about 63 mm², about 64 mm², about 65 mm², about 66 mm², about 67 mm², about 68 mm², about 69 mm², about 70 mm², about 71 mm², about 72 mm², about 73 mm², about 74 mm², about 75 mm², about 76 mm², about 77 mm², about 78 mm², about 79 mm², about 80 mm², or any cross-sectional area between any two of those cross-sectional areas.

The second prong 112 may have an inner cross-sectional area A2 of between about 5 mm² and about 50 mm², optionally between about 10 mm² and about 45 mm², optionally between about 15 mm² and about 40 mm², optionally between about 20 mm² and about 35 mm², optionally between about 25 mm² and about 30 mm², optionally about 5 mm², about 6 mm², about 7 mm², about 8 mm², about 9 mm², about 10 mm², about 11 mm², about 12 mm², about 13 mm², about 14 mm², about 15 mm², about 16 mm², about 17 mm², about 18 mm², about 19 mm², about 20 mm², about 21 mm², about 22 mm², about 23 mm², about 24 mm², about 25 mm², about 26 mm², about 27 mm², about 28 mm², about 29 mm², about 30 mm², about 31 mm², about 32 mm², about 33 mm², about 34 mm², about 35 mm², about 36 mm², about 37 mm², about 38 mm², about 39 mm², about 40 mm², about 41 mm², about 42 mm², about 43 mm², about 44 mm², about 45 mm², about 46 mm², about 47 mm², about 48 mm², about 49 mm², about 50 mm², or any cross-sectional area between any two of those cross-sectional areas.

Having specific differences between the inner diameters ID1, ID2 and/or the inner cross-sectional areas A1, A2 can contribute to desired levels of asymmetry.

A combined inner cross-sectional area (A1 + A2) of the first prong 111 and the second prong 112 may be between about 20 mm² and about 130 mm², optionally between about 30 mm² and about 120 mm², optionally between about 40 mm² and about 110 mm², optionally between about 50 mm² and about 100 mm², optionally between about 60 mm² and about 90 mm², optionally between about 70 mm² and about 80 mm², optionally about 20 mm², about 25 mm², about 30 mm², about 35 mm², about 40 mm², about 45 mm², about 50 mm², about 55 mm², about 60 mm², about 65 mm², about 70 mm², about 75 mm², about 80 mm², about 85 mm², about 90 mm², about 95 mm², about 100 mm², about 105 mm², about 110 mm², about 115 mm², about 120 mm², about 125 mm², about 130 mm², or any cross-sectional area between any two of those cross-sectional areas.

A ratio of the inner cross-sectional area A1 of the first prong 111 to the inner cross-sectional area A2 of the second prong 112 may be between about 60:40 and about 80:20; optionally between about 65:35 and about 80:20; optionally between about 70:30 and about 80:20; optionally between about 70:30 and about 75:25; optionally about 70:30, about 71:29, about 72:28, about 73:27, about 74:26, or about 75:25; optionally between about 75:25 and 80:20; optionally about 75:25, about 76:24, about 77:23, about 78:22, about 79:21, or about 80:20.

Referring to Figures 1C, 2, and 3, in some configurations a nasal interface 100 of the present disclosure comprises a first prong 111 and a second prong 112 that are asymmetrical to each other, and a gases manifold 120 comprising a gases inlet 121. The first prong 111 and the second prong 112 are in fluid communication with the gases inlet 121. The nasal interface 100 is configured to cause an asymmetrical flow of gases at a patient's nares. The nasal interface 100 is configured such that between about 60% and about 80% of a total volumetric flow rate of gases flow into the gases inlet 121 is delivered out of the nasal interface 100 through the first prong 111 when the total volumetric flow rate of gases flow into the gases inlet 121 is between about 5 liters per minute (lpm) and about 70 lpm. In some configurations, the total volumetric flow rate of gases flow into the gases inlet 121 is at least about 5 lpm. In some configurations, the total volumetric flow rate of gases flow into the gases inlet 121 is more than about 5 lpm. In some configurations, the total volumetric flow rate of gases flow into the gases inlet 121 is between about 5 lpm and about 120 lpm. In some configurations, the total volumetric flow rate of gases flow into the gases inlet 121 is between about 5 lpm and about 70 lpm.

The nasal interface 100 may be configured such that between about 70% and about 80% of the total volumetric flow rate of gases flow into the gases inlet 121 is delivered out of the nasal interface 100 through the first prong 111 when the total flow rate of gases flow into the gases inlet is between about 5 lpm and about 70 lpm.

The nasal interface 100 may be configured such that between about 70% and about 75% of the total volumetric flow rate of gases flow into the gases inlet 121 is delivered out of the nasal interface 100 through the first prong 111 when the total flow rate of gases flow into the gases inlet 121 is between about 5 lpm and about 70 lpm.

The nasal interface 100 may be configured such that between about 75% and about 80% of the total volumetric flow rate of gases flow into the gases inlet 121 is delivered out of the nasal interface 100 through the first prong 111 when the total flow rate of gases flow into the gases inlet 121 is between about 5 lpm and about 70 lpm.

The nasal interface 100 may be configured such that about 75% of the total volumetric flow rate of gases flow into the gases inlet 121 is delivered out of the nasal interface 100 through the first prong 111 when the total flow rate of gases flow into the gases inlet 121 is between about 5 lpm and about 70 lpm.

The nasal interface may be configured such that an amount of asymmetry of flow from the first prong 111 and second prong 112 is a function of the total flow rate of gases flow through into the gases inlet 121. A higher total flow rate of gases flow into the gases inlet 121 may generally result in a larger portion of the total volumetric flow rate of gases flow being delivered out of the nasal interface 100 through the first prong 111, and a lower total flow rate of gases flow into the gases inlet 121 results in a smaller portion of the total volumetric flow rate of gases flow being delivered out of the nasal interface 100 through the first prong 111.

Table 1 shows volumetric flow rates for a benchtop test of an exemplary nasal cannula.

**Table 1**

| **Total volumetric flow rate into gases inlet 121 (lpm)** | **Flow rate out of first prong 111 (lpm)** | **Flow rate out of second prong 112 (lpm)** | **Flow ratio second prong:first prong (lpm)** |
|---|---|---|---|
| 9.6 | 7.1 | 2.5 | 2.84 |
| 19 | 13.6 | 5.4 | 2.52 |
| 29.1 | 21 | 8.1 | 2.59 |
| 38.6 | 28.1 | 10.5 | 2.68 |
| 47.7 | 35 | 12.7 | 2.76 |
| 57.8 | 44.2 | 13.6 | 3.25 |
| 64.2 | 48.6 | 15.6 | 3.12 |

Testing and modelling indicates that by using asymmetrical prongs 111, 112 in the nasal interfaces 100 of the present disclosure, a reduction of dead space (i.e. the volume of air that would need to be rebreathed at the start of inspiration) can be achieved. This is most notable at higher flows, higher breath rates, and at higher degrees of asymmetry. It is understood that within the upper airway of the patient, some proportion of the gas moves in a unidirectional manner, flowing in one nostril and out the other, reducing the upper airway dead space. Increasing pressure on expiration has the effect of slowing breath rate. Slowing the breath rate also leads to a longer expiratory phase compared to inspiratory phase. Reduced breath rate increases the time at the end of expiration for flushing the upper airway to occur.

Dead space clearance has been found to improve with the degree of asymmetry. For example, with a total volumetric flow rate of 30 lpm and a breathing rate of 45 breaths per minute, a nasal interface with symmetric prongs results in an anatomical dead space of about 87 ml, a nasal interface 100 of the present disclosure with a 60:40 ratio of inner cross-sectional area of the first prong 111 : the second prong 112 results in an anatomical dead space of about 80 ml, and a nasal interface 100 of the present disclosure with a 70:30 ratio of cross sectional area of the first prong 111 : the second prong 112 results in an anatomical dead space of about 78 ml. The respective values change to about 66 ml, about 62 ml, and about 36 ml at 50 Ipm, and to about 49 ml, 41 ml, and 21 ml at 70 lpm.

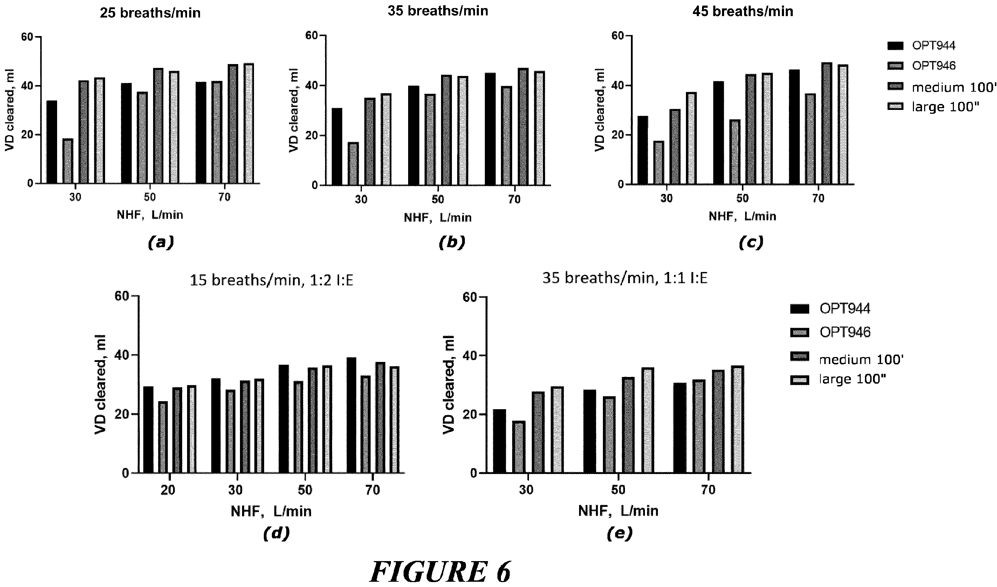

Figures 6(a)-6(e) show the dependency that dead space clearance has on the volume of upper airways, breath rate, and flow rate. Figures 6(a)-6(c) show results for larger upper airways and Figures 6(d) and 6(e) show results for smaller upper airways. The results are for the Opitflow^{™}+ OPT944 (medium) cannula from Fisher & Paykel Healthcare Limited, the Optiflow^{™}+ 946 (large) cannula from Fisher & Paykel Healthcare Limited, a medium nasal interface 100' in accordance with the present disclosure, and a large nasal interface 100" in accordance with the present disclosure.

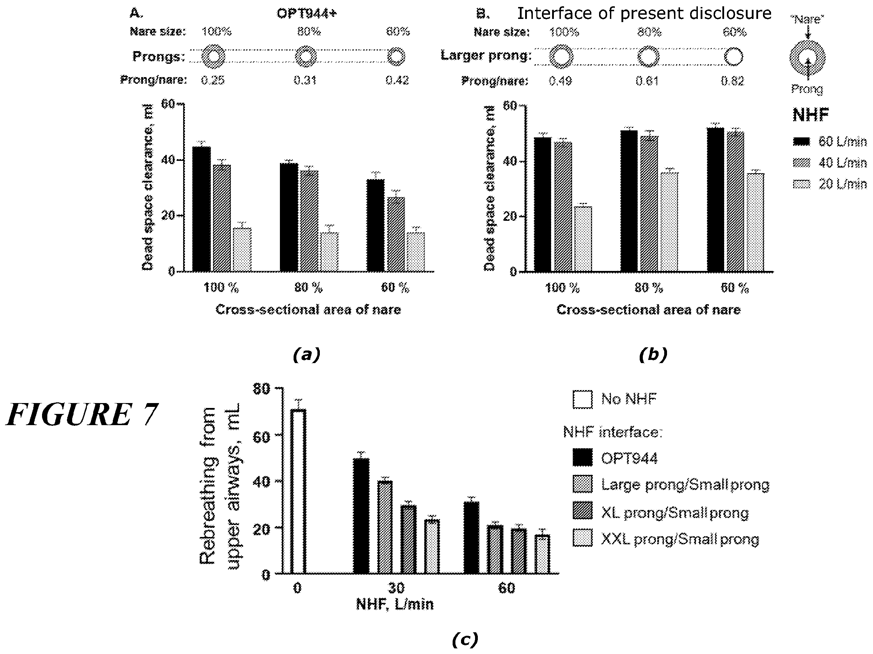

Figure 7 shows the effect of the degree of occlusion of the nostrils. Increasing the occlusion of the nostrils increases the pressure delivered to the patient for a given flow rate of gases. For the asymmetrical nasal interface of the present disclosure, the decreased cross-sectional area of the small second prong 112 helps to prevent simultaneous occlusion of both nares. A substantially smaller nasal interface may be uncomfortable and noisy due to jetting and high velocity gas in the nose of the patient. The pressure drop, or resistance to flow, of a substantially smaller nasal interface may limit the flow range able to be provided by a flow generator. A substantially larger nasal interface may be less likely to fit patients comfortably because the prongs may either touch the septum or ala. With a larger prong as per the present disclosure, the low gas velocity may result in quieter gases delivery.

Referring to Figures 4A to 4C, the nasal interfaces 100 may be provided in multiple sizes, such as a small size nasal interface 100 (Figure 4A), medium size nasal interface 100' (Figure 4B), and large size nasal interface 100" (Figure 4C) for example. The wings 113, 113', 113", 114, 114', 114" will generally have the same spacing and dimensions in each size of the nasal interface, to enable all of the nasal interfaces to be used with the same headgear. The size and spacing of the nasal prongs may be different in each of the small size nasal interface 100, medium size nasal interface 100', and large size nasal interface 100".

The nasal interfaces 100', 100" may have any one or more of the features and/or functionality described and shown herein for nasal interface 100. Like reference numbers indicate like parts with the addition of prime (') for the medium size nasal interface 100' and double prime (") for the large size nasal interface 100". It will be appreciated that any reference herein to nasal interface 100 could instead be a reference to nasal interface 100' or nasal interface 100".

The nasal interfaces 100', 100" may be used in any of the combinations, systems, or applications described herein for nasal interface 100.

Exemplary dimensions are outlined below in Table 2. As outlined in Table 2, each size nasal interface 100, 100', 100" may have several different sizes of first prong 111, 111', 111" and/or second prong 112, 112', 112" available.

Table 2 shows exemplary dimensions and ratios for small, medium, and large size nasal interfaces in accordance with the present disclosure. It will be appreciated that these are exemplary dimensions only and could vary.

**Table 2**

| **Cannula size** | **First prong 111 inner diameter ID1 (mm)** | **First prong 111 inner perimeter (mm)** | **First prong 111 inner cross-sectional area A1 (mm²)** | **Second prong 112 inner diameter (mm)** | **Second prong 112 inner perimeter (mm)** | **Second prong 112 inner cross-sectional area A2 (mm²)** | **Ratio of inner cross-sectional area A1/A2** | **Combined inner cross-sectional area A1+A2 (mm²)** |
|---|---|---|---|---|---|---|---|---|
| **Small** | 5.58 | 17.54 | 24.48 | 4.56 | 14.32 | 16.32 | 60/40 | 40.80 |
| | 5.58 | 17.54 | 24.48 | 3.65 | 11.48 | 10.49 | 70/30 | 34.97 |
| | 5.58 | 17.54 | 24.48 | 3.22 | 10.13 | 8.16 | 75/25 | 32.64 |
| | 5.58 | 17.54 | 24.48 | 2.79 | 8.77 | 6.12 | 80/20 | 30.60 |
| **Medium** | 7.50 | 23.56 | 44.16 | 6.12 | 19.23 | 29.44 | 60/40 | 73.60 |
| | 7.50 | 23.56 | 44.16 | 4.91 | 15.42 | 18.93 | 70/30 | 63.09 |
| | 7.50 | 23.56 | 44.16 | 4.33 | 13.59 | 14.70 | 75/25 | 58.86 |
| | 7.50 | 23.56 | 44.16 | 3.75 | 11.78 | 11.04 | 80/20 | 55.20 |
| **Large** | 9.43 | 29.64 | 69.90 | 7.70 | 24.20 | 46.60 | 60/40 | 116.50 |
| | 9.43 | 29.64 | 69.90 | 6.18 | 19.40 | 29.96 | 70/30 | 99.86 |
| | 9.43 | 29.64 | 69.90 | 5.42 | 17.03 | 23.08 | 75/25 | 92.98 |
| | 9.43 | 29.64 | 69.90 | 4.72 | 14.82 | 17.48 | 80/20 | 87.38 |

Referring to Figures 4A to 4C, with respect to a vertical dimension, a centre of flow C1 for the first prong and a centre of flow C2 for the second prong are at the same height above a central axis CA of the gases manifold 120 and the cannula body 118, 118', 118" for each size nasal interface 100, 100', 100". This is indicated by the constant distance between the upper and lower broken lines in Figures 4A, 4B, 4C. This is believed to provide benefits in terms of easily clearing expiratory gases around the second prong 112, having centre of inspiratory flow from the two outlets 111a, 112a at the same height, and enhancing comfort and usability.

In alternative configurations, a lower edge of the outlet 111a, 111a', 111a" of the first prong 111, 111', 111" may be aligned with the lower edge of the outlet 112a, 112a', 112a" of the second prong 112, 112', 112" or an upper edge of the outlet 111a, 111a', 111a" of the first prong 111, 111', 111" may be aligned with the upper edge of the outlet 112a, 112a', 112a" of the second prong 112, 112', 112".

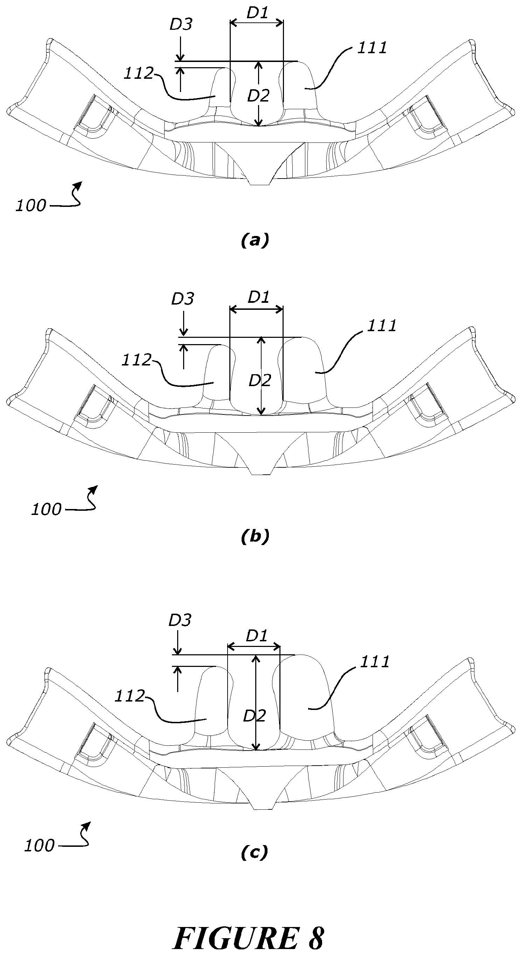

Figure 8 shows exemplary septum spacings and prong heights for the small size nasal interface 100, the medium size nasal interface 100', and the large size nasal interface 100".

At least the large first prong 111, 111', 111", and optionally also the small second prong 112, 112', 112", is made of soft material and has a thin wall to allow it to deform and accommodate different nose geometries. The septum spacing may be optimised. That is because the septum will contact the prongs closer to the base than the outer nose skin (ala). The further away from the base this contact occurs, the more flexibly the nasal interface will behave. Within the nasal vestibule, the septum wall is more sensitive and less tolerant to pressure from the nasal interface than the ala which are compliant. Therefore, the septum spacing D1 may by chosen so minimise contact between the prongs and the septum.

The gap or septum spacing D1 between adjacent outer surfaces of the first prong 111, 111', 111" and the second prong 112, 112', 112" adjacent a base of the first prong 111, 111', 111" and the second prong 112, 112', 112" may be between about 5 mm and about 15 mm, optionally between about 6 mm and about 14 mm, optionally between about 7 mm and about 13 mm, optionally between about 8 mm and about 12 mm, optionally between about 9 mm and about 11 mm, optionally about 5 mm, about 6 mm, about 7 mm, about 8 mm, about 9 mm, about 10 mm, about 11 mm, about 12 mm, about 13 mm, about 14 mm, about 15 mm, or any value between any two of those values.

Table 3 lists exemplary dimensions. It will be appreciated that other dimensions could be used.

**Table 3**

| **Nasal interface** | **Septum spacing D1 (mm)** | **First prong 111 height D2 (mm)** | **Difference in height D3 between first prong 111 and second prong 112 (mm)** | **Second prong height D2-D3 (mm)** |
|---|---|---|---|---|
| Small size 100 | 10 +/- 1.0 | 12.2 +/- 1.5 | 1.2 | 11 +/- 1.5 |
| Medium size 100' | 9.9 +/- 1.0 | 14.6 +/- 1.5 | 1.4 | 13.2 +/- 1.5 |
| Large size 100" | 9.8 +/- 1.0 | 17.7 +/- 1.5 | 1.86 | 15.84 +/- 1.5 |

In some configurations, the nasal interface 100, 100', 100" comprises a cannula body 118, 118', 118" comprising the first prong 111, 111', 111" and the second prong 112, 112', 112". An external surface of the cannula body between the first prong and the second prong comprises a dip 118e (as shown schematically in broken lines in Figure 3) to accommodate a portion of a patient's nose and reduce pressure on an underside of the accommodated portion.

In the configuration shown, the dip 118e comprises a hollowed outer portion and/or dipped outer profile in an upper surface of the cannula body 118, 118', 118" between bases of the prongs 111 and 112 to alleviate pressure at the septum/columella to enhance patient comfort and reduce pressure injuries to the columella and philtrum.

The hollowing should be as much as possible without significantly compromising the flow delivered to the patient. The dipped portion may be complementary to the periphery 123a, 123a' of the outlet 123, 123' of the gases manifold 120, 120' (as shown in Figures 9 and 10 for example) to maintain an effective seal between the cannula body 118, 118', 118" and the gases manifold 120, 120'. The dipped portion may be received in the outlet 123 of the gases manifold.

The combination of the dip 118e in the external surface of the cannula body 118 and the lower flow required for a given amount of flushing using asymmetric prongs 111, 112 together enhance patient comfort.

Referring to Figures 1C, 2 to 3, and 4A to 4C, in some configurations a nasal interface 100 of the present disclosure comprises a first prong 111 and a second prong 112 that are asymmetrical to each other, and a gases manifold 120 comprising a gases inlet 121. The first prong 111 and the second prong 112 are in fluid communication with the gases inlet 121. The first prong 111 has a larger inner cross-sectional area A1 in a direction transverse to gases flow GFD1 through the first prong 111 than a corresponding inner cross-sectional area A2 of the second prong 112 in a direction transverse to gases flow GFD2 through the second prong 112. The second prong 112 has a substantially ovate or elliptical cross-sectional shape in the direction transverse to gases flow GFD2 through the second prong, the substantially ovate or substantially elliptical cross-sectional shape having a first ratio of a widest dimension to a narrowest dimension, and the first prong 111 has a less ovate or less elliptical cross-sectional shape in the direction transverse to gases flow GFD1 through the first prong 111. The less ovate or less elliptical cross-sectional shape of the first prong may either have a second ratio of a widest dimension to a narrowest dimension that is smaller than the first ratio, or a substantially circular cross-sectional shape.

In an alternative configuration, both prongs 111, 112 may have substantially the same cross-sectional shapes in the direction transverse to gases flow through the respective prong. For example, both prongs 111, 112 may both have a substantially circular cross-sectional shape or may both have a different shape.

In some configurations, the direction transverse to gases flow is substantially perpendicular or normal to gases flow through the respective prong. Alternatively, the direction transverse to gases flow could be at an acute or obtuse angle relative to gases flow through the respective prong 111, 112.

The inner cross-sectional areas A1, A2 and/or inner cross-sectional shapes could be substantially constant along the length of the prongs 111, 112. Alternatively, the inner cross-sectional areas A1, A2 and/or inner cross-sectional shapes could vary along at least part of the length of the prongs 111, 112. The inner cross-sectional areas and inner cross-sectional shapes of the first and second prongs could be at the outlets 111a, 112a of the first and second prongs 111, 112 and/or at the distal portions of the first and second prongs 111, 112 adjacent the outlets 111a, 112a.

The first prong 111 is more flexible than the second prong 112. This may be as a result of the first prong 111 having a decreased wall thickness relative to the total width of the first prong than the second prong.

The larger first prong 111 may be more comfortable when having a less ovate, less elliptical, or more circular cross-sectional shape so it can most easily conform to the shape of the patient's nasal cavity.

The smaller second prong 112 is less flexible. By having a substantially ovate or substantially elliptical cross-sectional shape, the second prong 112 can match the shape of the patient's nasal cavity when at rest.

In some exemplary configurations, the first ratio is greater than 1.0. In some configurations, the first ratio is at least about 1.05, optionally at least about 1.1, optionally at least about 1.2, optionally at least about 1.3, optionally at least about 1.4, optionally at least about 1.5, optionally at least about 1.6, optionally at least about 1.7, optionally at least about 1.8, optionally at least about 1.9, optionally at least about 2.0, optionally more than about 2.

In some exemplary configurations, the second ratio is approximately 1.

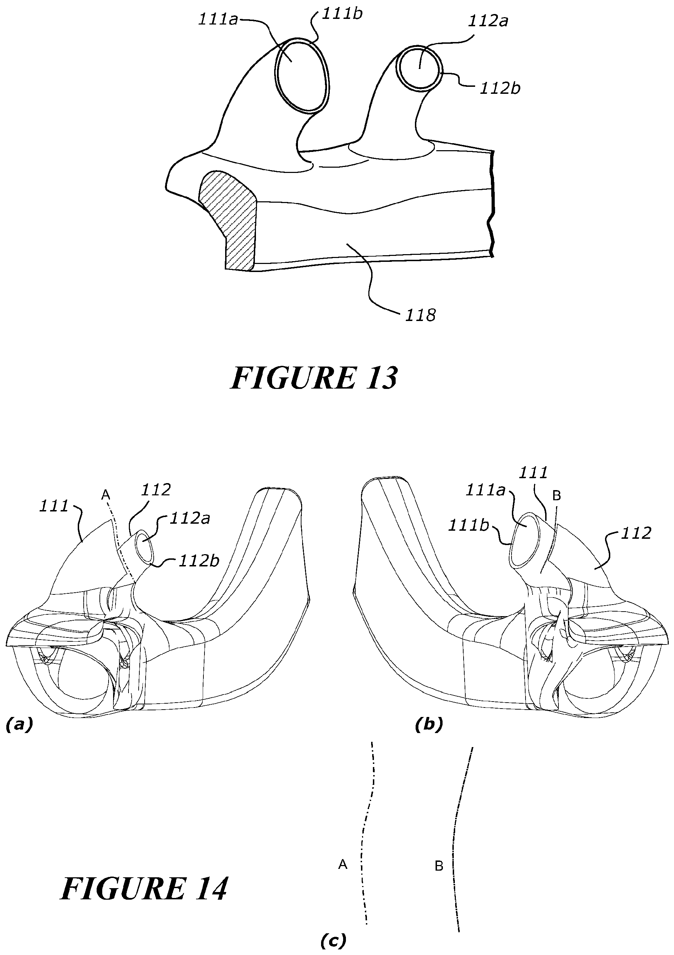

Referring to Figures 13 and 14, the first prong 111 has a first terminal end 111b adjacent the first opening 111a. The second prong 112 has a second terminal end 112b adjacent the second opening 112a.

With reference to Figure 14(a) the first terminal end 111b comprises a substantially scalloped surface. The scalloped surface is represented by the dot-dash line A in Figure 14(a) and 14(c).

In the configuration shown, a lower portion of the scalloped surface is concave when viewed from the exterior of the first prong 111 in a direction toward the opening 111a. An upper portion of the scalloped surface may be convex when viewed from the exterior of the first prong in a direction toward the opening 111a. The combination of the concave lower portion and convex upper portion together provide an overall sinuous shape.

With reference to Figure 14(b) and 14(c) the second terminal end 112b has a less scalloped surface. The face is represented by dot-dash line B in Figure 14(b) and 14(c). Although the face of the second terminal end is concave when viewed from the exterior of the second prong 112 in a direction toward the opening 112a, the extent of the concavity or scalloping is less than for the first prong 111. In some configurations, the face of the second prong may be substantially planar.

The scalloped surface of the first nasal prong 111 may provide a number of advantages. The first nasal prong 111 can deform or misshape more easily than if it had a planar face, as it has less structural rigidity. This makes the larger prong more comfortable in a patient's nasal passage. Because the smaller second nasal prong 112 has more clearance in the patient's nasal cavity, deformation of the second nasal prong 112 is not required. With a scalloped surface, the gases do not exit from the nasal prong as a jet, through a small aperture. The scalloping provides a larger area of exit opening at the exit of the prong, so that the velocity or air speed of the gases is reduced at the point where they exit the prong. That is, the size of the exit aperture (defined by the edge or perimeter of the cut-out section) is greater than the size or cross-sectional area of the inlet aperture of the nasal prong, which is defined by the base of the prong where it is connected to the face mount part 110. The air speed of the gases reduces as the area increases. That is, the prong is shaped so that the velocity of gases exiting said prong is reduced in comparison to the velocity of gases at or close to the gases point of entry to the prong. This allows a proportionally greater volume of gases to be delivered to a patient without causing discomfort (in comparison to a nasal prong which does not include a scalloped surface). With the scalloped surface, air jetting effects are reduced. The jetting of the airflow is reduced based on the continuity equation for energy or mass conservation, which states that increasing the cross-sectional area equates to a reduction in the velocity of the airflow. A jet of gas delivered into a user's nasal passage can irritate or potentially damage the tissue within the nasal passage. A reduction in the velocity of the flow of gases as delivered by the nasal prong reduces irritation in the user's nostril and thus the jetting effects. It also follows from the continuity equation that the larger the aperture a gas is flowing through, the larger the amount of diffusion. The stream of gases is directed in a generally rearwards direction (relative to the head of a patient) relative to the nasal passage of a patient. These effects may be more beneficial for the larger first nasal prong 111 than for the smaller second nasal prong 112 that has more clearance in the nare in use.

In some configurations, the nasal interface 100 may be configured such that the gases velocities exiting the first prong 111 and the second prong 112 are substantially similar. A benefit of having substantially similar exit velocities is patient comfort and low noise levels. The patient comfort may result from reduced or avoided jetting of gases flow against sensitive insides of the nares. In some configurations or applications, the nasal interface 100 disclosed herein may have a lower average exit velocity than a symmetric nasal interface at the same flow rate, but may be perceived as being more comfortable due to the reduction in the work of breathing. The reduction in the work of breathing may be a result of greater dead space clearance by the nasal interface 100 relative to a symmetric nasal interface at the same flow rate.

With reference to Figures 3, 4A, 12(a), and 12(b) for example, in some configurations, a nasal interface 100 of the present disclosure comprises a gases inlet 121, a first prong 111 and a second prong 112 that are asymmetrical to each other, and wherein the first prong 111 has a first prong outlet 111a and the second prong 112 has a second prong outlet 112a, and a gases flow path 122 from the gases inlet 121 to the first prong 111 and the second prong 112. The first prong 111 has a larger inner cross-sectional area A1 in a direction transverse to gases flow GFD1 through the first prong 111 than a corresponding inner cross-sectional area A2 of the second prong 112. For a given flow rate of gases at the gases inlet 121 in use, different flow rates of gases are provided through the first prong 111 and the second prong 112 and a velocity of gases exiting the first prong outlet 111a and the second prong outlet 112a is substantially similar.

The velocities referred to in this section may be the average velocities of gases exiting the respective first prong outlet 111a and second prong outlet 112a, rather than velocity profiles or peak velocities. In some configurations, the velocities may be peak velocities.

In some configurations, the nasal interface is a non-sealing nasal interface.

Although different flow rates of gases are provided through the first prong 111 and the second prong 112, the larger first prong 111 will have a greater flow and the smaller second prong 112 will have a lesser flow, so that the exit velocities from each prong are substantially similar.

In some configurations, the velocity of gases exiting the first prong outlet 111a is within about 20% of the velocity of gases exiting the second prong outlet 112a.

In some configurations, the velocity of gases exiting the first prong outlet 111a is within about 16% of the velocity of gases exiting the second prong outlet 112a.

In some configurations, the velocity of gases exiting the gases outlet 111a is within about 10% of the velocity of gases exiting the second prong outlet 112a at flow rates above about 42 Ipm.

The inventors have discovered a substantially linear trend between the total volumetric flow rate of gases flow into the gases inlet 121 and the velocity of gases exiting the first prong outlet 111a and the second prong outlet 112a. That is, for a given increase of total volumetric flow rate of gases flow into the gases inlet, there is a corresponding increase of average gases flow out of the two outlets 111a, 112a.

In some configurations, the velocity of gases exiting each of the first prong outlet 111a and the second prong outlet 112a is more than 0 m/s and less than about 32 m/s for a total volumetric flow rate of gases flow into the gases inlet 121 of more than 0 lpm and up to about 70 lpm.

In some configurations, the velocity of gases exiting each of the first prong outlet 111a and the second prong outlet 112a is more than 0 m/s and less than 32 m/s for a total volumetric flow rate of gases flow into the gases inlet 121 of more than 0 lpm and up to about 70 lpm.

In some configurations, the velocity of gases exiting each of the first prong outlet 111a and the second prong outlet 112a is more than about 2 m/s and less than about 32 m/s, optionally more than about 2 m/s and less than 32 m/s, optionally more than about 2 m/s and up to about 25 m/s, and optionally more than about 2.5 m/s and up to about 20 m/s for a total volumetric flow rate of gases flow into the gases inlet of more than 9 lpm and up to about 70 lpm.

The exit velocity values and relationships are with the first prong 111 more distal from the gases inlet 121 and the second prong more proximal to the gases inlet 121. If the configuration was reversed, depending on how balanced the gases manifold 120 is, there may be a small change (for example less than about 20%) in the velocities with the first prong 111 being more proximal to the gases inlet 121 and the second prong 112 being more distal from the gases inlet 121. The first prong 111 may have a higher flow rate and higher average exit velocity when it is more distal from the gases inlet 121 than when it is more proximal to the gases inlet 121. The second prong 112 may have a higher flow rate and higher average exit velocity when it is more distal from the gases inlet 121 than when it is more proximal to the gases inlet 121.

The velocities described above are for a medium size nasal interface 100'. The velocities for the small size nasal interface 100 or large size nasal interface 100" may reduce or increase from those values proportionally with the change in inner cross-sectional areas of the prongs.

In some configurations, the nasal interface 100 is configured such that a total volumetric flow rate of gases flow into the gases inlet 121 is at least about 5 liters per minute (lpm).

In some configurations, the nasal interface 100 is configured such that the total volumetric flow rate of gases flow into the gases inlet 121 is between about 5 lpm and about 120 Ipm.

In some configurations, the nasal interface 100 is configured such that the total volumetric flow rate of gases flow into the gases inlet 121 is between about 5 lpm and about 70 lpm.

In some configurations, the nasal interface 100 is configured such that about 7 lpm is delivered out of the nasal interface 100 through the first prong 111 at a volumetric flow rate of about 9.5 lpm at the gases inlet 121 and/or such that about 13.5 lpm is delivered out of the nasal interface 100 through the first prong 111 at a volumetric flow rate of about 19 lpm at the gases inlet 121 and/or such that about 21 lpm is delivered out of the nasal interface 100 through the first prong 111 at a volumetric flow rate of about 29 Ipm at the gases inlet 121 and/or such that about 28 Ipm is delivered out of the nasal interface 100 through the first prong 111 at a volumetric flow rate of about 38.5 lpm at the gases inlet 121 and/or about 35 lpm is delivered out of the nasal interface 100 through the first prong 111 at a volumetric flow rate of about 47.5 lpm at the gases inlet 121 and/or about 44 lpm is delivered out of the nasal interface 100 through the first prong 111 at a volumetric flow rate of about 58 lpm at the gases inlet 121 and/or about 48.5 lpm is delivered out of the nasal interface 100 through the first prong 111 at a volumetric flow rate of about 64 lpm at the gases inlet 121.

The remainder of the volumetric flow rate of gases flow will typically be delivered out of the nasal interface through the second prong 112. Table 4 shows exemplary approximate flow rates.

**Table 4**

| **Total volumetric flow rate at the gases inlet (lpm)** | **Flow rate through first prong 111** | **Flow rate through second prong 112** |
|---|---|---|
| 9.5 | 7 | 2.5 |
| 19 | 13.5 | 5.5 |
| 29 | 21 | 8 |
| 38.5 | 28 | 10.5 |
| 47.5 | 35 | 12.5 |
| 58 | 44 | 14 |
| 64 | 48.5 | 15.5 |

The nasal interface 100 may have any of the features or functionality described herein.

With reference to Figures 3, 4A, 12(a), and 12(b) for example, in some configurations, a nasal interface 100 of the present disclosure comprises a gases inlet 121, a first prong 111 and a second prong 112 that are asymmetrical to each other, and a gases flow path 122 from the gases inlet 121 to the first prong and the second prong. The first prong 111 has a larger inner cross-sectional area A1 in a direction transverse to gases flow GFD1 through the first prong 111 than a corresponding inner cross-sectional area A2 of the second prong 112 in a direction transverse to gases flow GFD2 through the second prong 112. The first prong 111 is downstream in the gases flow path 122 from the second prong 112.

In some configurations, the direction transverse to gases flow is substantially perpendicular or normal to gases flow through the respective prong. Alternatively, the direction transverse to gases flow could be at an acute or obtuse angle relative to gases flow through the respective prong 111, 112.

The gases flow path 122 is defined by a flow channel or lumen 124 in the gases manifold 120. A gases flow direction GFD3 of the gases flow path 122 is substantially perpendicular to the gases flow directions GFD1, GFD2 of the gases flow paths through the first prong 111 and the second prong 112. The first prong is more distal the gases inlet 121 and the second prong is more proximal the gases inlet 121.

In the configuration shown in Figure 3, a first section 124a of the flow channel or lumen in the gases manifold 120 has a first large vertical dimension V1. An opposite end of the flow channel or lumen forms a flow cavity 124b in the cannula body 118 that delivers gases to the first and second prongs 111, 112. The flow cavity 124b is in fluid communication with the flow passages through the first and second prongs 111, 112 when the gases manifold 120 is in position in the cannula body 118. At least part of the flow cavity 124b has a vertical dimension V2 that is smaller than the first vertical dimension V1.

The gases manifold 120 comprises one or more internal angled walls to provide the reduction in dimensions and to direct the gases flow into the first prong 111 and/or the second prongs 112.

The gases manifold 120 is configured to not obstruct any part of the internal cross-section of either prong 111, 112. In an alternative configuration, the manifold may be configured to partly obstruct the internal cross-section of one or both of the prongs 111, 112.

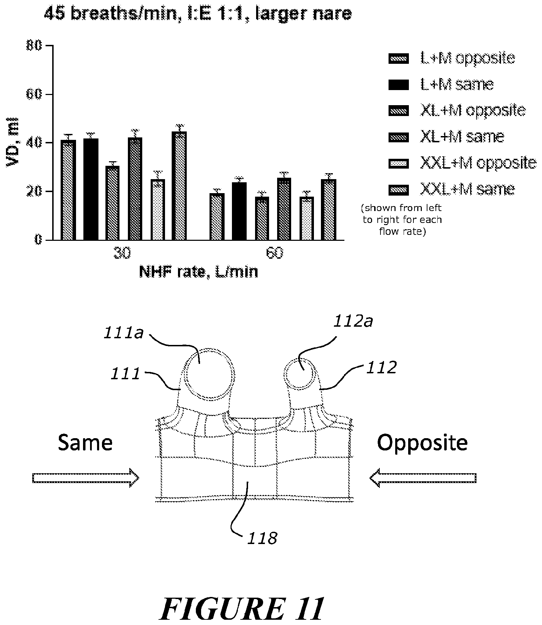

Benchtop testing has shown a reduction in the anatomical dead space when the larger first prong 111 is more distal the gases inlet 121 and the smaller second prong 112 is more proximal the gases inlet 121, as indicated by the results shown in Figure 11. This may be a consequence of having an opposed angled wall in the manifold that assists with directing gases into the larger first prong 111.

In Figure 11, the references to M, L, XL, and XXL relate to the size of the prongs used in the nasal interfaces in the testing. For example, L + M refers to a nasal interface with a large sized prong and a medium sized prong, XL + M refers to a nasal interface with an extra-large sized prong and a medium sized prong. XXL + M refers to a nasal interface with an extra extra-large sized prong and a medium sized prong.

As outlined above, the nasal interface may be configured such that at least about 60% of a total volumetric flow rate of gases flow into the gases inlet 121 is delivered out of the nasal interface through the first prong 111, optionally such that between about 60% and about 90% of the total volumetric flow rate of gases flow into the gases inlet 121 is delivered out of the nasal interface through the first prong 111, optionally such that between about 60% and about 80% of the total volumetric flow rate of gases flow into the gases inlet 121 is delivered out of the nasal interface through the first prong 111, optionally such that between about 65% and about 80% of the total volumetric flow rate of gases flow into the gases inlet 121 is delivered out of the nasal interface through the first prong 111, optionally such that between about 70% and about 80% of the total volumetric flow rate of gases flow into the gases inlet 121 is delivered out of the nasal interface through the first prong 111, optionally such that between about 70% and about 75% of the total volumetric flow rate of gases flow into the gases inlet 121 is delivered out of the nasal interface through the first prong 111, optionally such that about 70% of the total volumetric flow rate of gases flow into the gases inlet 121 is delivered out of the nasal interface through the first prong 111, optionally such that between about 75% and about 80% of the total volumetric flow rate of gases flow into the gases inlet 121 is delivered out of the nasal interface through the first prong 111, optionally such that about 75% of the total volumetric flow rate of gases flow into the gases inlet 121 is delivered out of the nasal interface through the first prong 111, optionally such that about 80% of the total volumetric flow rate of gases flow into the gases inlet 121 is delivered out of the nasal interface through the first prong 111.

In some configurations, a nasal interface 100 disclosed herein comprises a cannula body 118 comprising a first prong 111 and a second prong 112 that are asymmetrical to each other, and a gases manifold 120 comprising a gases inlet 121. The first prong 111 and the second prong 112 are in fluid communication with the gases inlet 121. The nasal interface 100 is configured to cause an asymmetrical flow of gases at a patient's nares.

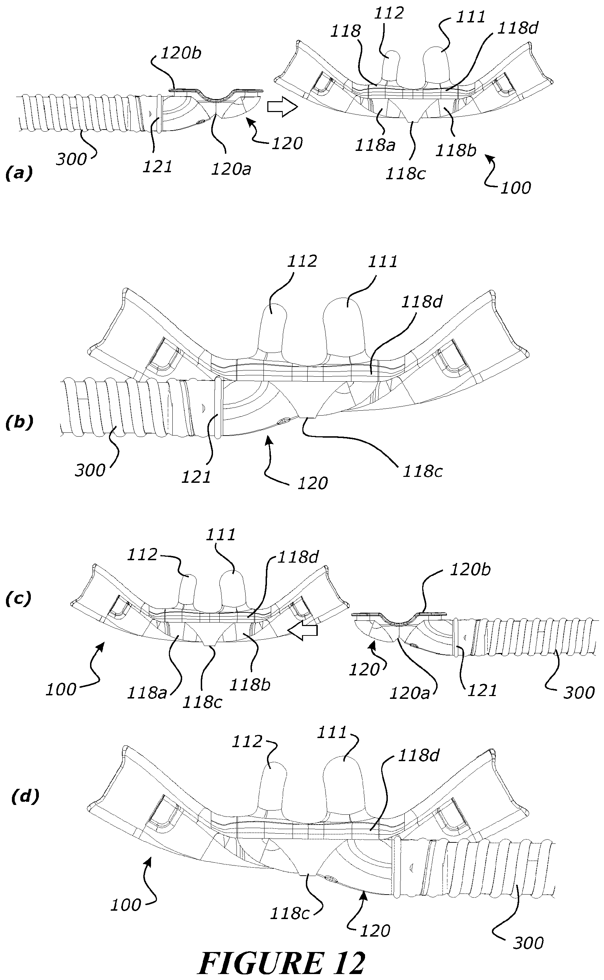

The cannula body 118 comprises the first prong 111 and the second prong 112. The gases manifold 120 is reconfigurable relative to the cannula body 118 between a first configuration e.g. as shown in Figure 12(a) and 12(b) and a second configuration e.g. as shown in Figure 12(c) and 12(d). The first configuration corresponds to the gases manifold 120 being inserted into the cannula body 118 from a first side of the cannula body 118 such that the second prong 112 is more proximal the gases inlet 121 and the first prong 111 is more distal the gases inlet 121. The second configuration corresponds to the gases manifold 120 being inserted into the cannula body 118 from a second side of the cannula body such that the first prong 111 is more proximal the gases inlet 121 and the second prong 112 is more distal the gases inlet 121.

The gases manifold may comprise a flow channel or lumen that has a gases flow direction GFD3 that is substantially perpendicular to gases flow directions GFD1, GFD2 through the first prong 111 and the second prong 112.

The cannula body 118 and/or the gases manifold 120 may comprise retaining feature(s) to removably retain the gases manifold 120 in engagement in the cannula body 118 in the first and second configuration.

In the configuration shown, the retaining features comprise a resilient annular portion 118c of the cannula body that is received in a complementary recess 120a of the gases manifold to removably retain the gases manifold 120 in engagement in the cannula body 118. The resilient annular portion 118c can be flexed to enable the gases manifold 120 to be removed from the cannula body 118. The annular portion may be circular or non-circular in shape.

Additionally, or alternatively, as shown in Figure 3, the gases manifold 120 may comprise a retaining flange 120b around a face thereof which is removably received in a complementary resilient rim 118d of the cannula body 118. The engagement of the retaining flange 120b with the complementary resilient rim 118d of the cannula body 118 assists with forming a seal between the gases manifold 120 and the cannula body 118.

Any other suitable type of retaining feature(s) could be used, such as clips or fasteners for example.

Side-swapping of the gases manifold 120 relative to the cannula body 118 enables a user to adjust which side the gases conduit 300 is on according to comfort and where the respiratory therapy apparatus is located. Additionally, side-swapping enables selection of the amount of asymmetry of the gases flow from the prongs 111, 112, which may be beneficial depending on the desired application or patient requirements.

In some configurations, a nasal interface 100 disclosed herein comprises a first prong 111 and a second prong 112, and a gases manifold 120 comprising a gases inlet 121. The first prong 111 and the second prong 112 are in fluid communication with the gases inlet 121. The nasal interface 100 is configured to cause an asymmetrical flow of gases at a patient's nares. The gases inlet 121 is in fluid communication with a breathable tube.

For example, the conduit 300 may comprise a breathable tube. A breathable tube is one in which water vapor can pass through the wall of the tube, but liquid water and the bulk flow of gases cannot flow through the wall of the tube. For example, the water vapour may be able to pass through the material and/or sealing skin of the wall of the tube, but liquid water and bulk flow of gases cannot flow through the material and/or sealing skin of the wall of the tube.

The conduit 300 could, for example, be made of an open cell foam material with a sealing skin.

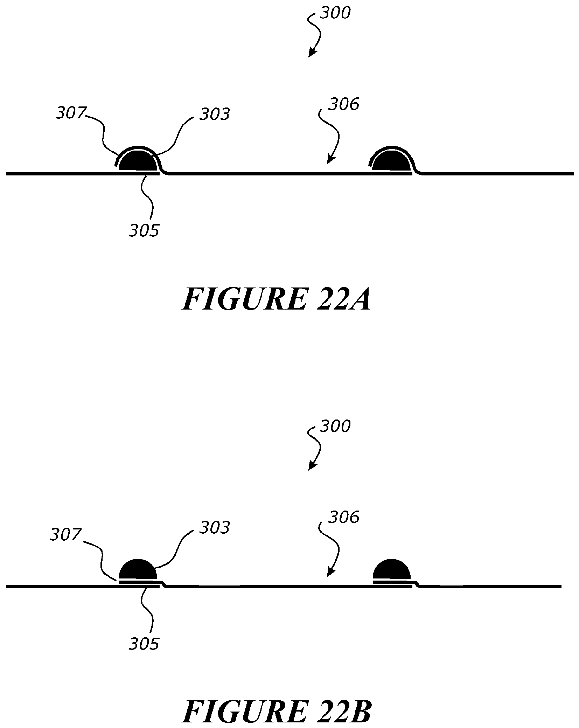

In an alternative configuration, the conduit 300 could comprise a thin film. Figure 22A schematically shows an exemplary method of manufacture of a single walled breathable tube. This method may be particularly suited to thin-walled conduits. The thin film 306 is arranged in a spiral or helix such that the edge portions of adjacent layers overlap and form the wall of a breathing gas conduit 300. Interposed the overlapping edges of adjacent winds of film 306 is a reinforcing element comprising a bead 303 of polymer material bonded with the overlapping portions of film 306 sealing the joint between windings and forming a continuous breathing gas conduit 300. The seam is formed between the edge 305 of a first layer of film 306 and the edge 307 of a second, adjacent layer of film 306 which is laid over top of the polymer bead 303 while the bead is molten. The overlapping layer of film, because it is so thin, follows the contour of the bead 303 very closely and results in a smooth inner conduit wall. In another alternative shown schematically in Figure 22B, the bead 303 is not interposed between overlapping edges of adjacent winds of film 306 but rather is disposed on both layers, on an exterior surface of the film 306. More specifically, the thin film 306 is arranged first in a spiral or helix such that edge portions of adjacent layers overlap. Then, the bead 303 of polymer material is disposed on the overlapping edges of the thin film 306 so as to form the breathing gas conduit 300. In some configurations, the bead 303 may be disposed on an interior surface of the film 306 in that the bead 303 is exposed to the lumen of the gas conduit 300. In such configurations, the elongate film is wrapped around the outside of the bead 303 such that the bead 303 interacts with the lumen of the gas conduit 300 and the film 306 forms the outer surface of the gas conduit 300.

The conduit 300 may have any one or more features outlined in US patent application publication no. 2019/0224439 titled "Breathing circuit components for respiratory apparatus" or US patent application publication no. 2017/0304578 titled "Tubes for medical systems".

In an alternative configuration, a tube between the patient conduit 300 and the gases inlet 121 may comprise the breathable tube. The breathable tube fluidly connects the patient conduit 300 with the gases inlet 121.

The gases manifold 120 may be integrally formed with the breathable tube or may be coupled to the breathable tube.

Having the gases inlets 121 in fluid communication with a breathable tube is beneficial when the patient interfaces are used with humidified gases. The breathable tube allows for high levels of humidity whilst mitigating the risk of rainout in which condensation forms in the flow path.

For the nasal interfaces 100, 100', 100" of the present disclosure, if the prong inner diameter ID1 is larger than the manifold 120 width, then some portion of the prong 111, 112 interior is restricted and there could be increased noise levels. The gases manifold 120 will advantageously be configured so that the manifold width is as large as or larger than the prong inner diameter ID1.

Figures 9(a) and 9(b) show an exemplary gases manifold 120 that can be used with the small nasal interface 100. The width W of the gases flow path 122 adjacent the first and second prongs 111, 112 is as large as or larger than the inner diameter ID1 of the first prong 111. For example, the width W of the gases flow path 122 may be at least about 1.2x the inner diameter ID1 of the first prong 111. Exemplary dimensions are ID1 = 5.6 mm and W = 6.8 mm, although it will be appreciated that the dimensions could vary.

Figures 10(a) and 10(b) show an exemplary gases manifold 120' that can be used with the medium nasal interface 100' or the large nasal interface 100". Like reference numbers indicate like parts to gases manifold 120 with the addition of a prime ('). The width W' of the gases flow path 122' adjacent the first and second prongs 111', 112' in use, is as large as or larger than the inner diameter ID1 of the first prong 111' of the medium nasal interface 100'. For example, the width W' of the gases flow path 122' may be at least about 1.04x the inner diameter ID1 of the first prong 111'. Exemplary dimensions are ID1 = 7.5 mm and W' = 7.8 mm, although it will be appreciated that the dimensions could vary.

The gases manifold 120' may also be used with the large nasal interface 100" while still reducing noise, even though the inner diameter of the first prong 111" of the large nasal interface 100" may be larger than the width W", at 9.4 mm for example.

It will be appreciated that these are exemplary dimensions only, and the dimensions of the prongs of the nasal interfaces 100, 100', 100" and of the gases manifolds 120, 120' may vary.

The nasal interfaces 100, 100', 100" described herein could have any one or more of the features and/or functionality described in PCT publication no. WO 2015/020540 or US patent no. 10,569,043.

In the configurations shown, the larger first nasal prong 111, 111', 111" is on one side of the cannula body 118 and the smaller second nasal prong 112, 112', 112" is on the other side of the cannula body 118. It will be appreciated that these prongs could be swapped so they are on the opposite sides to what is shown. Alternatively, the nasal interface 100, 100', 100" may be designed in a way that the left and right nasal prongs can be swapped.

When using the nasal interfaces 100, 100', 100", pressure and flow may be measured and controlled in the nares simultaneously or separately. Flow may be continuous in one nare, while it is varied in the other nare according to the breathing cycle. Different interfaces, each delivering asymmetrical flow in the nose, may be used to continuously deliver supplemental oxygen, and to deliver continuous or variable nasal high flow. One nasal prong element may be used to deliver oxygen, gases, aerosols or the like to the patient while another nasal delivery prong may be used to deliver a higher flow of air, or a different flow of oxygen, gases, aerosols or the like to the patient. Each nasal delivery element may supply different flow rates to the patient, and may connect to different flow generating elements.

The respiratory therapy systems disclosed herein with the nasal interfaces 100, 100'. 100" may improve the performance of NHF therapy, particularly in the therapy delivered to infants and children. It may reduce resistance compared to existing nasal interfaces and may extend and improve functionality of respiratory devices without modification of the hardware or software.

Asymmetrical flow useful herein can be provided by a nasal interface using any form of pressure support, such as continuous positive airway pressure (CPAP) or non-invasive therapy (NIV). Anatomical dead space can be cleared by transnasal unidirectional flow during a therapy with increased airway pressure, where one nare may be sealed or may be used for inspiration from the apparatus without entrainment of room air and the other nare may be used for expiration.

One prong, and thereby one nare, may be connected to the inspiratory limb of a two-limbed ventilator circuit or to a breathing tube in a one-limbed circuit, such as a CPAP blower. The other prong, and thereby the other nare, may be connected to conventional ventilation holes in the interface for biased flow, or connected to the expiratory limb in a two-limbed circuit ventilator. Connection to the expiratory limb of a ventilator may allow the use of flow variations to control the breathing in periodic breathing or Central Sleep Apnoea due to carbon dioxide clearance in the upper airway or re-breathing from the expiratory limb.

Opening the mouth may decrease the pressure delivered to the patient and may improve clearance of anatomical dead space. A mouthpiece may be inserted to maintain the leak, and may be further connected to a negative pressure line or the expiratory limb to increase or control clearance of dead space. The amount of leak may be configurable to control the amount of pressure.

To achieve comfortable asymmetrical flow, a high level of humidity, such as that delivered by the devices known as AIRVO^{™} or ICON^{™} (AIRVO^{™} is a humidifier with integrated flow generator device and ICON^{™} is a CPAP device, manufactured by Fisher & Paykel Healthcare Limited), may be necessary to prevent drying of the nasal epithelium. The comfort level of temperature and dew point may be determined from a ratio, and may be, but is not limited to, a range of 27°C - 37°C, optionally 31°C - 37°C, optionally 33°C - 37°C, and may depend on the flow rate.

In some configurations, the system is configured to deliver gases through the nasal interface with a relative humidity of up to 100%.

In some configurations, the system is configured to deliver gases through the nasal interface with an absolute humidity of greater than about 33 mg/l. In some configurations, the system is configured to deliver gases through the nasal interface with an absolute humidity of up to about 44 mg/l.

One or both of the nasal prongs may be provided with fittings such as, but not limited to, sleeves and inserts to optimise NHF therapy. Sleeves as described herein refer to any structure added externally to a nasal delivery element of a nasal interface. Inserts as described herein refer to any structure added internally into a nasal delivery element of a nasal interface.

The NHF therapy can be improved or optimised to deliver a desired pressure profile and efficiently clear anatomical dead space. A nasal delivery element of a nasal interface with a smaller diameter may produce a jet with a higher velocity that may more efficiently clear patient dead space than a nasal delivery element with a larger diameter. Efficient clearance of dead space reduces the amount of carbon dioxide rebreathing that occurs. However a larger diameter may reduce the leak that occurs around the nasal delivery elements of the nasal interface and may result in a higher delivered pressure during both inspiration and expiration. A larger diameter may be more preferable in an acute setting, particularly when a patient is suffering from respiratory distress, as a higher expiratory pressure may decrease respiratory rate and improve ventilation.

By adding fittings to the nasal delivery elements of the nasal interface, it is possible to have nasal delivery elements which combine a smaller inner and a larger outer diameter to improve or optimise dead space clearance while maintaining a high pressure at the same flow. A combination of a nasal delivery element with a large outer diameter and a smaller inner diameter may have similar pressure effects to a nasal delivery element with a large diameter and no insert, while a smaller inner diameter may provide less pressure. If the outer diameter is too large for a patient, the inspiratory pressure may become negative as the flow from the interface may be lower than the peak inspiratory flow.

It generally is not desirable to increase the wall thickness of a nasal delivery element as it may be stiff in the nose of the patient, which may damage the inner surface of the nares, causing patient discomfort. However by attaching the different fittings to the interface it may be possible to benefit from the combination of the inner and outer diameters, while still providing the patient with soft nasal delivery elements to be fitted into the nares, maintaining patient comfort.

For example, by adding a sleeve onto a nasal delivery element of a nasal interface, the inner diameter of the nasal delivery element remains the same and may allow jetting effects to efficiently clear the anatomical dead space, while the outer diameter has been increased to reduce the leak around the nasal delivery element and may produce higher pressure swings during breathing. The added sleeve may then be removed once the desired therapy has been delivered, or a higher pressure is no longer required. A sleeve may also function as a one-way valve which may inflate on expiration and increase expiratory pressure. To inhibit or prevent condensate accumulation a semi-permeable material may be used, a leak may be introduced, or a combination of these may be used. A sleeve may also be added to the interface to decrease the outer diameter and also thereby decrease the inner diameter, which may increase jetting effects, deviate or split the flow from the centre of the nasal delivery element to the periphery, or may combine these.

A second example is to add an insert inside the nasal delivery element. This may decrease the inner diameter to reduce pressure and increase dead space clearance, while keeping the outer diameter the same. A smaller inner diameter increases jetting effects, deviates or splits the flow from the centre of the nasal delivery element to the periphery, or may combine the flow jetting effects with deviation or splitting of the flow from the centre of the nasal delivery element to the periphery.

Other configurations may include, using a fitting that may block a nasal delivery element, allowing NHF to be delivered through the unblocked nasal delivery element to the patient, using fittings that may cause asymmetrical flow to occur, or that may make an asymmetrical interface symmetrical. Adding sleeves that have been individually fit to a patient may reduce operational flow which may result in reduced noise, reduced supplemental oxygen use, improved patient comfort, and the like. Reduced operational flow may also allow less heating, water use, and the like, to be required. Only one interface is needed per patient and it can be specifically fit to the patient to vary pressure or dead space clearance.

Figure 20 shows the results of testing of the nasal interfaces of the present disclosure.

Figure 20(a) shows how a nasal interface 100, 100', 100" of the present disclosure can be used to achieve an increased area of occlusion while still maintaining a safe clearance in one naris. A patient can still breathe though the naris with the safe clearance in the event of a device or system failure.

Figure 20(b) shows test data showing increased positive-end expiratory pressure (PEEP) and reduced rebreathing when using a nasal interface of the present disclosure with asymmetric prongs vs a nasal interface with symmetric prongs when nasal high flow of 30 liters per minute (lpm) is applied. The data is shown for rebreathing patterns with respiratory rates of 15 breaths per minute and 35 breaths per minute and an I:E ratio of 0.69, where I:E is the ratio of inspiratory time to expiratory time. The dotted line represents the rebreathing that occurs if no nasal high flow is applied.

Figure 20(c) shows similar test data to Figure 20(b) but for nasal high flow of 60 Ipm. The data is shown for rebreathing patterns with respiratory rates of 15 breaths per minute and 35 breaths per minute and an I:E ratio of 0.69, where I:E is the ratio of inspiratory time to expiratory time. The dotted line represents the rebreathing that occurs if no nasal high flow is applied.

The data indicates that the nasal high flow delivered via a nasal interface of the present disclosure with increased occlusion compared to a nasal interface with symmetric prongs can result in greater positive airway pressure and dead-space clearance and reduced rebreathing.

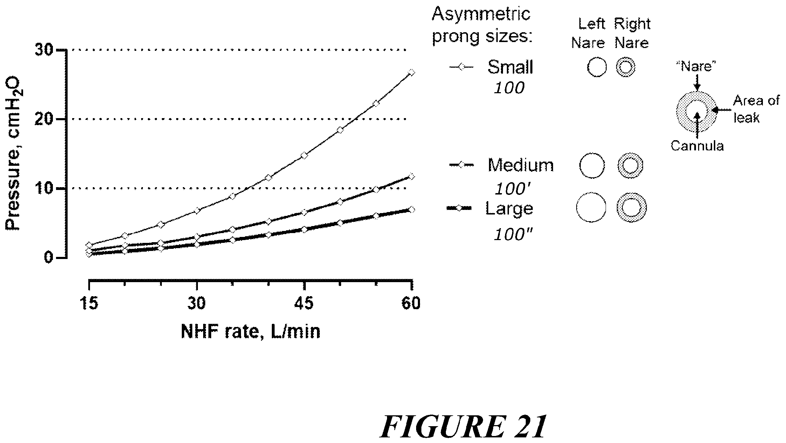

Figure 21 shows the maximum airway pressure that can be achieved for each size of nasal interface of the present disclosure when the larger prong fully occludes one of the patient's nares.

More particularly, Figure 21 shows the airway pressure that can be achieved in a static condition for each size of nasal interface 100, 100', 100" when the larger prong fully occludes one of the patient's nares. This represents the maximum possible occlusion for each nasal interface 100, 100', 100", and in term represents the maximum pressure that can be achieved in a static condition.

The data shows that even at maximum flow rate with possible user error resulting in the incorrect size of nasal interface 100, 100', 100" being used, the maximum pressure at static conditions is still within a safe range.

In the nasal interfaces 100, 100', 100" of the present disclosure, the first prong 111 has a shape and the second prong 112 has a shape. The first prong 111 has a larger inner diameter ID1 and/or larger inner cross-sectional area A1 in a direction transverse to gases flow GFD1 through the first prong 111 than a corresponding inner diameter ID2 and/or inner cross-sectional area A2 of the second prong 112 in a direction transverse to gases flow GFD2 through the second prong 112. At least the first prong 111 may be made of an elastomeric material that enables the first prong to deform and set its shape in use in response to temperature and contact with the patient's naris. That is, the first prong 111 is configured to deform and set its shape in use of the nasal interface 100, 100', 100" in response to temperature and contact with the patient's naris.

In some configurations, the temperature may be between about 20°C and about 41°C, optionally more than 20°C and up to about 41°C, optionally between about 31°C and about 41°C, optionally between about 36°C and about 39°C, optionally about 37°C, or may be any other suitable temperature that is experienced during therapy. The temperature will generally be above ambient temperature.

In some configurations, the first prong 111 may be configured to deform and set its shape in use to substantially match the internal shape of the patient's naris. In alternative configurations, the first prong 111 may be configured to bend or deform in response to temperature and contact with the patient's naris to set the shape, but may not substantially match the internal shape of the patient's naris after the shape is set. For example, one or more discrete portions of an outer surface of the first prong 111 may contact one or more discrete regions of the patient's naris in use, such that the one or more discrete portions of the outer surface deforms and set its shape.

The deformation and setting of the shape may be a permanent deformation. Alternatively, the deformation and setting of the shape may be reversible upon application of a suitable combination of temperature and time.

The elastomeric material may exhibit time and temperature dependent properties at or below a desired therapy temperature to enable in-use shape setting of at least the first prong 111 to conform more appropriately to the patient's naris. For example, the elastomeric material may exhibit compression set properties to enable the setting of the shape. The elastomeric material may also exhibit tensile set and/or stress relaxation properties which will typically be related to the compression set properties. The elastomeric material which exhibits compression set, tensile set, and/or stress relaxation properties at or below therapy temperature may reduce the discomfort that a user may experience during the provision of therapy owing to a nasal prong impacting the inner surface of the naris.

Both the first prong 111 and the second prong 112 may be made of the elastomeric material. In that configuration, both the first prong 111 and the second prong 112 may deform and set their shapes in use. The cannula body 118, the first prong 111, and the second prong 112 may be made of the elastomeric material. Alternatively, the second prong 112 may be made of a different material.

The elastomeric material allows at least the larger first prong 111, and optionally the second prong 112, to deform and set its shape in use in relation to contact between the outside of the prong(s) and the inside of the patient's nares.

As the larger first prong 111 may be sized to have a smaller clearance than symmetric prongs, having the larger first prong 111 deform and set its shape in use to at least partly conform to the patient's naris may increase comfort.

To achieve this performance, at least the first prong 111 of the patient interface, and optionally both prongs 111, 112 of the patient interface, is made of an elastomeric material that enables the prong(s) to deform and set its/their shape at or below the temperature of the gases flow through the prong(s) 111, 112 of the nasal interface. The material may be selected so as to not enable shape setting at ambient temperatures so that the prong(s) do not set their shapes when the nasal interface 100, 100', 100" is not in use.

In some configurations, the elastomeric material enables the first prong to deform and set its shape to substantially match the internal shape of the patient's naris at therapy temperatures of between about 31°C and about 41°C, optionally between about 36°C and about 39°C, optionally about 37°C.

In some configurations, the first prong 111 is not made of silicone and does not comprise silicone as it does not enable shape setting at therapy temperatures.

In some configurations, at least the first prong 111 is made of a thermoplastic elastomer.

In some configurations, the elastomeric material exhibits between about 10% and about 50% compression set at temperatures between about 20°C and about 40°C after 72 hours when tested according to Method A of ISO 815-1:2014.

In some configurations, the elastomeric material exhibits between about 10% and about 45%, optionally between about 10% and about 40%, optionally between about 10% and about 35%, optionally between about 10% and about 30%, optionally between about 10% and about 25%, optionally between about 10% and about 20%, optionally between about 11% and about 19%, optionally between about 12% and about 18%, optionally between about 13% and about 17%, optionally between about 14% and about 16%, optionally about 15% compression set at temperatures between about 20°C and about 40°C after 72 hours when tested according to Method A of ISO 815-1:2014.

In some configurations, the elastomeric material exhibits between about 10% and about 45%, optionally between about 10% and about 40%, optionally between about 10% and about 35%, optionally between about 10% and about 30%, optionally between about 10% and about 25%, optionally between about 10% and about 20%, optionally between about 11% and about 19%, optionally between about 12% and about 18%, optionally between about 13% and about 17%, optionally between about 14% and about 16%, optionally about 15% compression set at temperatures above about 20°C and up to about 35°C, optionally at temperatures above about 20°C and up to about 30°C, optionally at temperatures above about 20°C and up to about 25°C, optionally at a temperature of about 21°C or about 22°C or about 23°C or about 24°C or about 25°C or higher after 72 hours when tested according to Method A of ISO 815-1:2014.

The elastomeric material may be selected so that shape setting occurs at a temperature of about 23°C or higher, which is generally above ambient temperature but below usage temperature.

The elastomeric material could comprise any elastomer that demonstrates shape setting properties at therapy temperatures. In some configurations, the elastomeric material is THERMOLAST^{®} K TF3STE - TPE - from Kraiburg TPE GmbH & Co. KG.

In addition to the elastomeric material, the nasal interfaces 100, 100', 100" may otherwise have any one or more of the features described herein.

Patient interfaces 1 with nasal interfaces 100, 100', 100" according to the configurations described herein may be employed in a respiratory therapy method. The respiratory therapy method comprise delivering gas to the airway of a patient in need thereof, improving the ventilation of a patient in need thereof, reducing the volume of anatomical dead space within the volume of the airway of a patient in need thereof, and/or treating a respiratory condition in a patient in need thereof, as described above.

Patient interfaces 1 comprising nasal interfaces 100, 100', 100" of the type disclosed herein may be used in a respiratory therapy system for delivering gases to a patient.

In some configurations, the respiratory therapy system 1000 comprises a respiratory therapy apparatus 1100 and a patient interface comprising 1 a nasal interface 100, 100', 100".

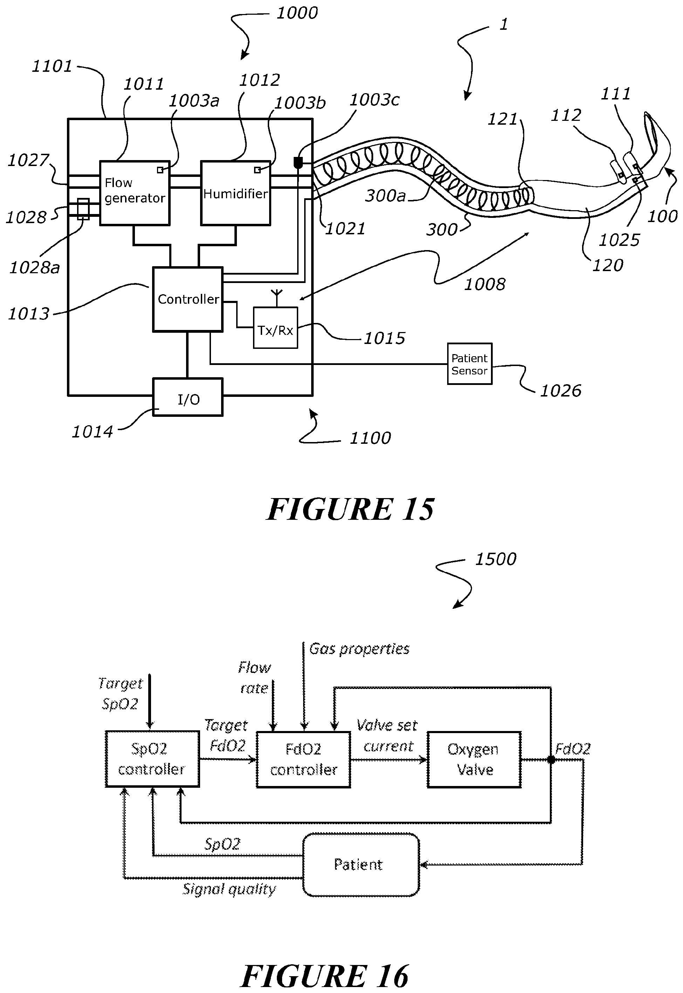

An exemplary respiratory therapy apparatus 1100 is shown in Figure 15.

The respiratory therapy apparatus 1100 comprises a main housing 1101 that contains a flow generator 1011 in the form of a motor/impeller arrangement (for example, a blower), an optional humidifier 1012 to humidify gases, a controller 1013, and a user interface 1014 (comprising, for example, a display and input device(s) such as button(s), a touch screen, or the like).

The controller 1013 can be configured or programmed to control the operation of the apparatus. For example, the controller can control components of the apparatus, including but not limited to: operating the flow generator 1011 to create a flow of gas (gases flow) for delivery to a patient, operating the humidifier 1012 (if present) to humidify and/or heat the generated gases flow, control a flow of oxygen into the flow generator blower, receiving user input from the user interface 1014 for reconfiguration and/or user-defined operation of the apparatus 1000, and outputting information (for example on the display) to the user.

The user can be a patient, healthcare professional, or anyone else interested in using the apparatus. As used herein, a "gases flow" can refer to any flow of gases that may be used in the breathing assistance or respiratory device, such as a flow of ambient air, a flow comprising substantially 100% oxygen, a flow comprising some combination of ambient air and oxygen, and/or the like.

A patient breathing conduit 300 is coupled at one end to a gases flow outlet 1021 in the housing 1100 of the respiratory therapy apparatus 1100. The patient breathing conduit 300 is coupled at another end to the nasal interface 100 with the gases manifold 120 and nasal prongs 111, 112.

The gases flow that is generated by the respiratory therapy apparatus 1100 may be humidified, and delivered to the patient via the patient conduit 300 through the nasal interface 100. The patient conduit 300 can have a heater to heat gases flow passing through to the patient. For example, the patient conduit 300 can have a heater wire 300a to heat gases flow passing through to the patient. The heater wire 300a can be under the control of the controller 1013. The patient conduit 300 and/or nasal interface 100 can be considered part of the respiratory therapy apparatus 1100, or alternatively peripheral to it. The respiratory therapy apparatus 1100, breathing conduit 300, and patient interface 1 comprising a nasal interface 100 together can form a respiratory therapy system 1000.

The controller 1013 can control the flow generator 1011 to generate a gases flow of the desired flow rate. The controller 1013 can also control a supplemental oxygen inlet to allow for delivery of supplemental oxygen, the humidifier 1012 (if present) can humidify the gases flow and/or heat the gases flow to an appropriate level, and/or the like. The gases flow is directed out through the patient conduit 300 and nasal interface 100 to the patient. The controller 1013 can also control a heating element in the humidifier 1012 and/or the heating element 300a in the patient conduit 300 to heat the gas to a desired temperature for a desired level of therapy and/or level of comfort for the patient. The controller 1013 can be programmed with or can determine a suitable target temperature of the gases flow. In some configurations, gas mixture compositions including supplemental oxygen and/or administration of therapeutic medicaments may be provided through the supplemental oxygen inlet. The gas mixtures compositions may comprise oxygen, heliox, nitrogen, nitric oxide, carbon dioxide, argon, helium, methane, sulfur hexafluoride, and combinations thereof, and/or the supplemental gas can comprise an aerosolized medicament.

The oxygen inlet port 1028 can include a valve 1028a through which a pressurized gas may enter the flow generator or blower. The valve can control a flow of oxygen into the flow generator blower. The valve can be any type of valve, including a proportional valve or a binary valve. The source of oxygen can be an oxygen tank or a hospital oxygen supply. Medical grade oxygen is typically between 95% and 100% purity. Oxygen sources of lower purity can also be used. Examples of valve modules and filters are disclosed in PCT publication number WO 2018/074935 and US patent application publication no. 2019/0255276, both titled "Valve Module and Filter.

The respiratory therapy apparatus 1100 can measure and control the oxygen content of the gas being delivered to the patient, and therefore the oxygen content of the gas inspired by the patient. During high flow therapy, the high flow rate of gas delivered meets or exceeds the peak inspiratory demand of the patient. This means that the volume of gas delivered by the device to the patient during inspiration meets, or is in excess of, the volume of gas inspired by the patient during inspiration. High flow therapy therefore helps to prevent entrainment of ambient air when the patient breathes in, as well as flushing the patient's airways of expired gas. So long as the flow rate of delivered gas meets or exceeds peak inspiratory demand of the patient, the likelihood of entrainment of ambient air is reduced, and the gas delivered by the device is typically substantially the same as the gas the patient breathes in. As such, the oxygen concentration measured in the device, fraction of delivered oxygen, (FdO2) would be substantially the same as the oxygen concentration the user is breathing, fraction of inspired oxygen (FiO2), and as such the terms may can be seen as equivalent.

Operation sensors 1003a, 1003b, 1003c, such as flow, temperature, humidity, and/or pressure sensors can be placed in various locations in the respiratory therapy apparatus 1100. Additional sensors (for example, sensors 1020, 1025) may be placed in various locations on the patient conduit 300 and/or nasal interface 100 (for example, there may be a temperature sensor 1029 at or near the end of the inspiratory tube). Output from the sensors can be received by the controller 1013, to assist the controller in operating the respiratory therapy apparatus 1100 in a manner that provides suitable therapy. In some configurations, providing suitable therapy includes meeting a patient's inspiratory demand, optionally the patient's peak inspiratory demand. The apparatus 1100 may have a transmitter and/or receiver 1015 to enable the controller 1013 to receive signals 1008 from the sensors and/or to control the various components of the respiratory therapy apparatus 1100, including but not limited to the flow generator 1011, humidifier 1012, and heater wire 300a, or accessories or peripherals associated with the respiratory therapy apparatus 1100. Additionally, or alternatively, the transmitter and/or receiver 1015 may deliver data to a remote server or enable remote control of the apparatus 1100.

Oxygen may be measured by placing one or more gas composition sensors (such as an ultrasonic transducer system, also referred to as an ultrasonic sensor system) after the oxygen and ambient air have finished mixing. The measurement can be taken within the device, the delivery conduit, the patient interface, or at any other suitable location.

The respiratory therapy apparatus 1100 can include a patient sensor 1026, such as a pulse oximeter or a patient monitoring system, to measure one or more physiological parameters of the patient, such as a patient's blood oxygen saturation (SpO2), heart rate, respiratory rate, perfusion index, and provide a measure of signal quality.

The sensor 1026 can communicate with the controller 1013 through a wired connection or by communication through a wireless transmitter on the sensor 1026.

The sensor 1026 may be a disposable adhesive sensor designed to be connected to a patient's finger. The sensor 1026 may be a non-disposable sensor.

Sensors are available that are designed for different age groups and to be connected to different locations on the patient, which can be used with the respiratory therapy apparatus 1100.

The pulse oximeter would be attached to the user, typically at their finger, although other places such as an earlobe are also an option. The pulse oximeter would be connected to a processor in the device and would constantly provide signals indicative of the patient's blood oxygen saturation. The patient sensor 1026 can be a hot swappable device, which can be attached or interchanged during operation of the respiratory therapy apparatus 1100. For example, the patient sensor 1026 may connect to the respiratory therapy apparatus 1100 using a USB interface or using wireless communication protocols (such as, for example, near field communication, WiFi or Bluetooth^{®}). When the patient sensor 1026 is disconnected during operation, the respiratory therapy apparatus 1100 may continue to operate in its previous state of operation for a defined time period. After the defined time period, the respiratory therapy apparatus 1100 may trigger an alarm, transition from automatic mode to manual mode, and/or exit control mode (e.g., automatic mode or manual mode) entirely. The patient sensor 1026 may be a bedside monitoring system or other patient monitoring system that communicates with the respiratory therapy apparatus 1100 through a physical or wireless interface.

The respiratory therapy apparatus 1100 may comprise a high flow therapy apparatus. High flow therapy as discussed herein is intended to be given its typical ordinary meaning as understood by a person of skill in the art, which generally refers to a respiratory assistance system delivering a targeted flow of humidified respiratory gases via an intentionally unsealed (non-sealing) patient interface with flow rates generally intended to meet or exceed inspiratory flow of a patient. Typical patient interfaces include, but are not limited to, a nasal or tracheal patient interface. Typical flow rates for adults often range from, but are not limited to, about fifteen liters per minute (lpm) to about seventy liters per minute or greater. Typical flow rates for pediatric patients (such as neonates, infants and children) often range from, but are not limited to, about one liter per minute per kilogram of patient weight to about three liters per minute per kilogram of patient weight or greater. High flow therapy can also optionally include gas mixture compositions including supplemental oxygen and/or administration of therapeutic medicaments. High flow therapy is often referred to as nasal high flow (NHF), humidified high flow nasal cannula (HHFNC), high flow nasal oxygen (HFNO), high flow therapy (HFT), or tracheal high flow (THF), among other common names. The flow rates used to achieve "high flow" may be any of the flow rates listed below. For example, in some configurations, for an adult patient 'high flow therapy' may refer to the delivery of gases to a patient at a flow rate of greater than or equal to about 10 liters per minute (10 lpm), such as between about 10 lpm and about 100 Ipm, or between about 15 lpm and about 95 Ipm, or between about 20 lpm and about 90 Ipm, or between 25 lpm and 75 Ipm, or between about 25 lpm and about 85 Ipm, or between about 30 lpm and about 80 Ipm, or between about 35 lpm and about 75 Ipm, or between about 40 lpm and about 70 Ipm, or between about 45 Ipm and about 65 Ipm, or between about 50 lpm and about 60 lpm. In some configurations, for a neonatal, infant, or child patient 'high flow therapy' may refer to the delivery of gases to a patient at a flow rate of greater than 1 Ipm, such as between about 1 lpm and about 25 Ipm, or between about 2 lpm and about 25 Ipm, or between about 2 lpm and about 5 Ipm, or between about 5 lpm and about 25 Ipm, or between about 5 lpm and about 10 Ipm, or between about 10 lpm and about 25 Ipm, or between about 10 lpm and about 20 Ipm, or between about 10 lpm and 15 Ipm, or between about 20 Ipm and 25 lpm. A high flow therapy apparatus with an adult patient, a neonatal, infant, or child patient, may deliver gases to the patient at a flow rate of between about 1 lpm and about 100 Ipm, or at a flow rate in any of the sub-ranges outlined above. The flow therapy apparatus 1000 can deliver any concentration of oxygen (e.g., FdO2), up to 100%, at any flow rate between about 1 lpm and about 100 lpm. In some configurations, any of the flow rates can be in combination with oxygen concentrations (FdO2s) of about 20%-30%, 21%-30%, 21%-40%, 30%-40%, 40%-50%, 50%-60%, 60%-70%, 70%-80%, 80%-90%, and 90%-100%. In some combinations, the flow rate can be between about 25 lpm and 75 lpm in combination with an oxygen concentration (FdO2) of about 20%-30%, 21%-30%, 21%-40%, 30%-40%, 40%-50%, 50%-60%, 60%-70%, 70%-80%, 80%-90%, and 90%-100%. In some configurations, the respiratory therapy apparatus 1100 may include safety thresholds when operating in manual mode that prevent a user from delivering to much oxygen to the patient.

In some configurations, the respiratory therapy apparatus 1100 comprises a controller 1013; a blood oxygen saturation sensor 1026; an ambient air inlet 1027; an oxygen inlet 1028; a valve 1028a in fluid communication with the oxygen inlet 1028 to control a flow of oxygen through the oxygen inlet 1028; and a gases outlet 1021; wherein the controller 1013 is configured to control the valve 1028a based on at least one measurement of oxygen saturation from the blood oxygen saturation sensor 1026.

The patient interface 1 used in the respiratory therapy system 1000 with the respiratory therapy apparatus 1100 comprises a nasal interface 100 comprising: a first prong 111 and a second prong 112 that are asymmetrical to each other; and a gases manifold 120 comprising a gases inlet 121, wherein the first prong 111 and the second prong 112 are in fluid communication with the gases inlet 121. The nasal interface 100 is configured to cause an asymmetrical flow of gases at a patient's nares.

The first prong 111 and the second prong 112 are asymmetrical to each other or are not symmetrical to each other or differ in shape and configuration to each other or are asymmetrical when compared to each other.

In some configurations, the nasal interface 100 comprises a cannula body 118 comprising the first prong 111 and the second prong 112.

In some configurations, the gases manifold 120 is integral with the cannula body 118 or is separate from and couplable with the cannula body 118.

In some configurations, the first and second prongs 111, 112 are configured to engage with the nasal passages in an unsealed (non-sealing) manner.

In some configurations, the first and second prongs 111, 112 allow exhaled gases to escape around the first and second prongs.

In some configurations, the first and second prongs 111, 112 are configured to provide gases to the patient without interfering with the patient's spontaneous respiration.

The nasal interface 100 may have any one or more of the features and/or functionality described herein for nasal interfaces 100, 100', 100".

In some configurations, the respiratory therapy apparatus 1000 comprises a flow generator 1011 and a humidifier 1012.

In some configurations, the respiratory therapy system comprises a patient conduit 300 with a heater 300a.

In some configurations, the patient interface comprises a breathable tube that is in fluid communication with the gases inlet 121, and the patient interface further comprises a headgear to retain the nasal interface against a patient's face.

Patients suffering from various health conditions and diseases can benefit from oxygen therapy. For example, patients suffering from chronic obstructive pulmonary disease (COPD), pneumonia, asthma, bronchopulmonary dysplasia, heart failure, cystic fibrosis, sleep apnea, lung disease, trauma to the respiratory system, acute respiratory distress, receiving pre- and post- operative oxygen delivery, and other conditions or diseases can benefit from oxygen therapy. A common way of treating such problems is by supplying the patients with supplemental oxygen to prevent their blood oxygen saturation (SpO2) from dropping too low (e.g., below about 90%). However, supplying the patient with too much oxygen can over oxygenate their blood, and is also considered dangerous. Generally, the patient's SpO2 is kept in a range from about 80% to about 99%, and preferably about 92% to about 96%, although these ranges may differ due to patient conditions. Due to various factors such as respiratory rate, lung tidal volume, heart rate, activity levels, height, weight, age, gender, and other factors, there is no one prescribed level of supplemental oxygen that can consistently achieve an SpO2 response in the targeted range for each patient. Individual patients will regularly need their fraction of oxygen delivered to the patient (FdO2) monitored and adjusted to ensure they are receiving the correct FdO2 to achieve the targeted SpO2. Achieving a correct and consistent SpO2 is an important factor in treating patients with various health conditions or diseases. Additionally, patients suffering from these health problems may find benefit from a system that automatically controls oxygen saturation. The present disclosure is applicable to a wide range of patients that require fast and accurate oxygen saturation control.

With reference to Figure 15, the controller 1013 can be programmed with or configured to execute a closed loop control system for controlling the operation of the respiratory therapy apparatus 1100. The closed loop control system can be configured to ensure the patient's SpO2 reaches a target level and consistently remains at or near this level.

The controller 1013 can receive input(s) from a user that can be used by the controller 1013 to execute the closed loop control system. The target SpO2 value can be a single value or a range of values. The value(s) could be pre-set, chosen by a clinician, or determined based on the type of patient, where type of patient could refer to current affliction, and/or information about the patient such as age, weight, height, gender, and other patient characteristics. Similarly, the target SpO2 could be two values, each selected in any way described above. The two values would represent a range of acceptable values for the patient's SpO2. The controller can target a value within said range. The targeted value could be the middle value of the range, or any other value within the range, which could be pre-set or selected by a user. Alternatively, the range could be automatically set based on the targeted value of SpO2. The controller can be configured to have one or more set responses when the patient's SpO2 value moves outside of the range. The responses may include alarming, changing to manual control of FdO2, changing the FdO2 to a specific value, and/or other responses. The controller can have one or more ranges, where one or more different responses occur as it moves outside of each range.

Generally, SpO2 would be controlled between about 80% and about 100%, or about 80% and about 90%, or about 88% and about 92%, or about 90% and about 99%, or about 92% and about 96%. The SpO2 could be controlled between any two suitable values from any two of the aforementioned ranges. The target SpO2 could be between about 80% and about 100%, or between about 80% and about 90%, or between about 88% and about 92%, or between about 90% and about 99%, or between about 92% and about 96%, or about 94%, or 94% or about 90%, or 90%, or about 85%, or 85%. The SpO2 target could be any value between any two suitable values from any two of the aforementioned ranges. The SpO2 target can correspond to the middle of the SpO2 for a defined range.

The FdO2 can be configured to be controlled within a range. The oxygen concentration measured in the apparatus (FdO2) would be substantially the same as the oxygen concentration the patient is breathing (FiO2) so long as the flow rate meets or exceeds the peak inspiratory demand of the patient, and as such the terms may can be seen as equivalent. Each of the limits of the range could be pre-set, selected by a user, or determined based on the type of patient, where the type of patient could refer to current affliction, and/or information about the patient such as age, weight, height, gender, and/or other patient characteristic. Alternatively, a single value for FdO2 could be selected, and the range could be determined at least partially based on this value. For example, the range could be a set amount above and below the selected FdO2. The selected FdO2 could be used as the starting point for the controller. The system could have one or more responses if the controller tries to move the FdO2 outside of the range. These responses could include alarming, preventing the FdO2 moving outside of the range, switching to manual control of FdO2, and/or switching to a specific FdO2. The device could have one or more ranges where one or more different responses occur as it reaches the limit of each range.

With reference to Figure 16, a schematic diagram of the closed loop control system 1500 is illustrated. The closed loop control system may utilize two control loops. The first control loop may be implemented by the SpO2 controller. The SpO2 controller can determine a target FdO2 based in part on the target SpO2 and/or the measured SpO2. As discussed above, the target SpO2 value can be a single value or a range of acceptable values. The value(s) could be pre-set, chosen by a clinician, or determined automatically based on client characteristics. Generally, target SpO2 values are received or determined before or at the beginning of a therapy session, though target SpO2 values may be received at any time during the therapy session. During a therapy session, the SpO2 controller can also receive as inputs: measured FdO2 reading(s) from a gases composition sensor, and measured SpO2 reading(s) and a signal quality reading(s) from the patient sensor. In some configurations, the SpO2 controller can receive target FdO2 as an input, in such a case, the output of the SpO2 controller may be provided directly back to the SpO2 controller as the input. Based at least in part on the inputs, the SpO2 controller can output a target FdO2 to the second control loop.

During the therapy session, the SpO2 and FdO2 controllers can continue to automatically control the operation of the respiratory therapy apparatus 1100 until the therapy session ends or an event triggers a change from the automatic mode to manual mode.

The increase in flushing caused by the asymmetry of the prongs 111, 112 in the nasal interface 100, 100', 100" can improve the effectiveness of the supplemental oxygen. Closed loop SpO2 control with an asymmetric nasal interface 100, 100', 100" can allow for the patient's SpO2 to be maintained at or near a target value with a reduced amount of oxygen being used when compared with symmetric nasal high flow. This can result in oxygen conservation.

The respiratory therapy system may have any one or more of the features and functionality described in PCT publication no. WO 2021/049954 and U.S. provisional application no. 62/898,464.

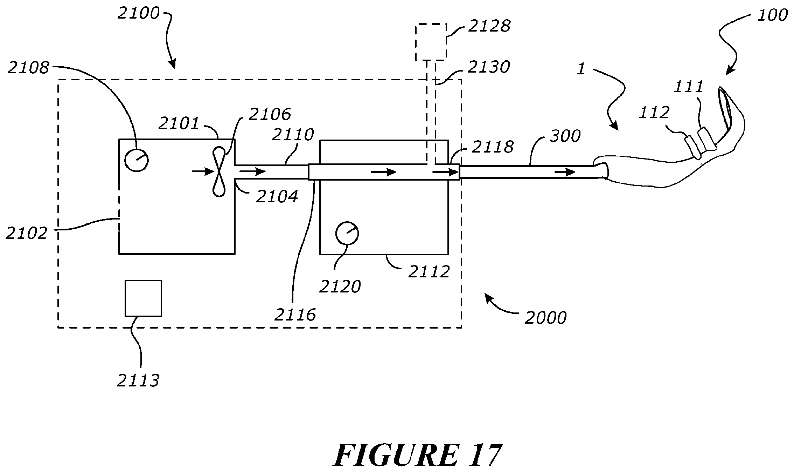

Figure 17 shows an alternative exemplary respiratory therapy system 2000 that can make use of the patient interface 1 comprising a nasal interface 100, 100', 100".

In the illustrated configuration, the respiratory therapy system 2000 comprises a respiratory therapy apparatus 2100. The respiratory therapy apparatus may comprise a flow generator 2101.

The illustrated flow generator 2101 comprises a gases inlet 2102 and a gases outlet 2104. The flow generator 2101 may comprise a blower 2106. The blower 2106 can draw in gas from the gases inlet 2102. In some configurations, the flow generator 2101 can comprise a source or container of compressed gas (e.g., air, oxygen, etc.). The container can comprise a valve that can be adjusted to control the flow of gas leaving the container. In some configurations, the flow generator 2101 can use such a source of compressed gas and/or another gas source in lieu of the blower 2106. In some configurations, the blower 2106 can be used in conjunction with another gas source. In some configurations, the blower 2106 can comprise a motorized blower or can comprise a bellows arrangement or some other structure capable of generating a gas flow. In some configurations, the flow generator 2101 draws in atmospheric gases through the gases inlet 2102. In some configurations, the flow generator 2101 is adapted both to draw in atmospheric gases through the gases inlet 2102 and to accept other gases (e.g., oxygen, nitric oxide, carbon dioxide, etc.) through the same gases inlet 2102 or a different gases inlet. Other configurations also are possible.

The illustrated flow generator 2101 comprises a user control interface 2108. The user control interface 2108 can comprise one or more buttons, knobs, dials, switches, levers, touch screens, speakers, displays, and/or other input or output modules that a user might use to input commands into the flow generator 2101, to view data, and/or to control operations of the flow generator 2101, and/or to control operations of other aspects of the respiratory therapy system 2000.

The flow generator 2101 can direct gases through the gases outlet 2104 to a first conduit 2110. In the illustrated configuration, the first conduit 2110 channels the gases to a gas humidifier 2112. The gas humidifier is optional.

The gas humidifier 2112 is used to entrain moisture in the gases in order to provide a humidified gas stream. The illustrated gas humidifier 2112 comprises a humidifier inlet 2116 and a humidifier outlet 2118. The gas humidifier 2112 can comprise, be configured to contain or contain water or another humidifying or moisturizing agent (hereinafter referred to as water).

In some configurations, the gas humidifier 2112 comprises a heating element (not shown). The heating element can be used to heat the water in the gas humidifier 2112 to encourage water vaporization and/or entrainment in the gas flow and/or increase the temperature of gases passing through the gas humidifier 2112. The heating element can, for example, comprise a resistive metallic heating plate. However, other heating elements are contemplated. For example, the heating element could comprise a plastic electrically conductive heating plate or a chemical heating system having a controllable heat output.

In the illustrated configuration, the gas humidifier 2112 comprises a user control interface 2120. The user control interface 2120 comprises one or more buttons, knobs, dials, switches, levers, touch screens, speakers, displays and/or other input or output modules that a user might use to input commands into the gas humidifier 2112, to view data, and/or to control operations of the gas humidifier 2112, and/or control operations of other aspects of the respiratory therapy system 2000.

In some configurations, the flow generator 2101 and the gas humidifier 2112 may share a housing 2126. In some configurations, the gas humidifier 2112 may share only part of the housing 2126 with the flow generator 2101. Other configurations also are possible. For example, the flow generator 2101 and the gas humidifier 2112 may comprise separate housings.

In the illustrated configuration, gases travel from the humidifier outlet 2118 to a second conduit 300. The second conduit 300 can comprise a conduit heater as described in relation to Figure 15. The conduit heater can be used to add heat to gases passing through the second conduit 300. The heat can reduce or eliminate the likelihood of condensation of water entrained in the gas stream along a wall of the second conduit 300. The conduit heater can comprise one or more resistive wires located in, on, around or near a wall of the second conduit 300. In one or more configuration, such one or more resistive wires can be located outside of any gas passage. In one or more configurations, such one or more resistive wires are not in direct contact with the gases passing through the second conduit 300. In one or more configurations, a wall or surface of the second conduit 300 intercedes between the one or more resistive wires and the gases passing through the second conduit 300.

Gases passing through the second conduit 300 can be delivered to a nasal interface 100. The nasal interface 100 can pneumatically link the respiratory therapy system 100 to an airway of a patient. In some configurations, the respiratory therapy system 2000 utilizes a two-limb system comprising separate inspiratory and expiratory gas passageways that interface with one or more airways of the patient.

In some configurations, a short length of tubing connects the nasal interface 100 to the second conduit 300. In some configurations, the short length of tubing can have a smooth bore. For example, a short flexible length of tubing can connect the nasal interface to the second conduit 300. The short length of tubing connecting the nasal interface to the second conduit 300 may be breathable such that it allows the transmission of vapour through the wall of the tube. In some configurations, the short length of tubing can incorporate one or more heating wires as described elsewhere herein. The smooth bore, whether heated or not, can improve the efficiency in delivering nebulized substances, as described elsewhere herein.

The respiratory therapy apparatus 2100 comprises a nebulizer 2128. In some configurations, if a nebulizer 2128 is used, the flow generator 2101, the gas humidifier 2112, and the nebulizer 2128 can share the housing 2126. In some configurations, the nebulizer 2128 is separate of the housing 2126.

The nebulizer 2128 can be linked to a portion of the gas passageway extending between the flow generator 2101 (which may include the gas inlet 2102) and the nasal interface 100, although other arrangements for the nebulizer 2128 or another nebulizer may be utilized. In some configurations, the nebulizer 2128 is not positioned in-line in any location between the humidifier outlet 2118 and the nasal interface 100. Rather, the nebulizer 2128 is positioned upstream of the humidifier outlet 2118 or upstream of the inlet to the second conduit 2122. In some configurations, the nebulizer 2128 can be positioned upstream of an inlet into the humidifier. In some configurations, the nebulizer 2128 can be positioned between the source of gases flow and the chamber.

The nebulizer 2128 can comprise a substance (e.g., a medicinal substance, trace gases, etc.) that can be introduced into the gas flow. The substance can be caught up in the gas flow and can be delivered along with respiratory gases to an airway of the patient. The nebulizer 2128 can be linked to the portion of the gas passageway by a conveyor 2130, which can comprise a conduit or an adaptor. Alternatively, the nebulizer 2128 can interface directly with the gas passageway, which can render the conveyor 2130 unnecessary.

The respiratory therapy apparatus 2100 may comprise a controller 2113. The controller 2113 can be configured or programmed to control the operation of the apparatus. For example, the controller 2113 can control components of the apparatus, including but not limited to: operating the flow generator 2101 to create a flow of gas (gases flow) for delivery to a patient, operating the humidifier 2112 (if present) to humidify and/or heat the generated gases flow, control a flow of oxygen into the flow generator blower, receiving user input from the user interface 2108 and/or 2120 for reconfiguration and/or user-defined operation of the apparatus 2100, and outputting information (for example on a display) to the user.

The controller 2113 can control the flow generator 2101 to generate a gases flow of the desired flow rate. The controller 2113 can also control a supplemental oxygen inlet to allow for delivery of supplemental oxygen, the humidifier 2112 (if present) can humidify the gases flow and/or heat the gases flow to an appropriate level, and/or the like. The controller 2113 may also control the operation of the nebulizer 2128. The gases flow is directed out through the patient conduit 300 and nasal interface 100 to the patient. The controller 2113 can also control a heating element in the humidifier 2112 and/or a heating element in the patient conduit 300 to heat the gas to a desired temperature for a desired level of therapy and/or level of comfort for the patient. The controller 2113 can be programmed with or can determine a suitable target temperature of the gases flow. In some configurations, gas mixture compositions including supplemental oxygen and/or administration of therapeutic medicaments may be provided through the supplemental oxygen inlet. The gas mixtures compositions may comprise oxygen, heliox, nitrogen, nitric oxide, carbon dioxide, argon, helium, methane, sulfur hexafluoride, and combinations thereof, and/or the supplemental gas can comprise an aerosolized medicament from the nebulizer 2128.

In some configurations, the respiratory therapy apparatus 2100 comprises a gases inlet 2102, a gases outlet 2118, and a nebulizer 2128 to deliver one or more substances into a gases flow. The nasal interface 100 used in the respiratory therapy system 2000 with the respiratory therapy apparatus 2100 comprises: a gases inlet 121 in fluid communication with the gases outlet 2118 to receive gases and the one or more substances from the respiratory therapy apparatus; a first prong 111 and a second prong 112 that are asymmetrical to each other; and a gases manifold 120 comprising a gases inlet 121. The first prong 111 and second prong 112 are in fluid communication with the gases inlet 121. The nasal interface 100 is configured to cause an asymmetrical flow of gases at a patient's nares.

The respiratory therapy system 2000 may comprise a conduit 300, 320 (examples of which are described below) to receive the gases and the one or more substances from the respiratory therapy apparatus 2100 and deliver the gases and the one or more substances to the gases inlet 121 of the nasal interface 100.

In the illustrated configuration, the respiratory therapy system 2000 can operate as follows. Gases can be drawn into the flow generator 2101 through the gas inlet 2102 due to the rotation of an impeller of the motor of the blower 2106. The gases are propelled out of the gas outlet 2104 and through the first conduit 2110. The gases enter the gas humidifier 2112 through the humidifier inlet 2116. Once in the gas humidifier 2112, the gases entrain moisture when passing over or near water in the gas humidifier 2112. The water is heated by the heating element, which aids in the humidification and/or heating of the gases passing through the gas humidifier 2112. The gases leave the gas humidifier 2112 through the humidifier outlet 2118 and enter the second conduit 300. Prior to entering the second conduit 300, the gases receive one or more substances from the nebulizer 128. The gases are passed from the second conduit 300 to the nasal interface 100, where the gases are taken into the patient's airways to aid in the treatment of respiratory disorders.

With reference to Figures 2, 3, and 15 for example, in some configurations, a respiratory therapy system 1000 of the present disclosure comprises:
a respiratory therapy apparatus 1100 comprising:
   at least one gases inlet 1027, 1028;
   a humidifier 1012 to humidify gases; and
   a gases outlet 1021;
and a patient interface 1 comprising a nasal interface 100, wherein the nasal interface comprises:
   a first prong 111 and a second prong 112 that are asymmetrical to each other, and wherein the first prong 111 has a first prong outlet 111a and the second prong 112 has a second prong outlet 112a;
   and a gases manifold 120 comprising a gases inlet 121, wherein the first prong 111 and the second prong 112 are in fluid communication with the gases inlet 121;
   wherein the nasal interface 100 is configured to cause an asymmetrical flow of gases at a patient's nares;
wherein the respiratory therapy system 1000 is configured to deliver gases through the first prong outlet 111a and the second prong outlet 112a at a temperature range of between about 27°C - 37°C, at a relative humidity of greater than about 33 mg/l, and/or at a velocity of more than 0 m/s and less than about 32 m/s for a total volumetric flow rate of gases flow into the gases inlet of more than 0 lpm and up to about 70 lpm.

In some configurations, the respiratory therapy system 1000 is configured to deliver gases through the first prong outlet 111a and the second prong outlet 112a at a temperature range of between about 31°C - 37°C.

In some configurations, the respiratory therapy system 1000 is configured to deliver gases through the first prong outlet 111a and the second prong outlet 112a with a relative humidity of up to about 44 mg/l.

In some configurations, the respiratory therapy system 1000 is configured to provide a total volumetric flow rate of gases flow into the gases inlet 121 of at least about 5 liters per minute (lpm), optionally of between about 5 lpm and about 120 Ipm, and optionally of between about 5 lpm and about 70 lpm.

In some configurations, the respiratory therapy system 1000 is configured to deliver at least about 60% of a total volumetric flow rate of gases flow into the gases inlet 121 out of the nasal interface through the first prong 111, optionally between about 60% and about 90% of the total volumetric flow rate of gases flow into the gases inlet 121 out of the nasal interface through the first prong 111, optionally between about 60% and about 80% of the total volumetric flow rate of gases flow into the gases inlet 121 out of the nasal interface through the first prong 111, optionally between about 65% and about 80% of the total volumetric flow rate of gases flow into the gases inlet 121 out of the nasal interface through the first prong 111, optionally between about 70% and about 80% of the total volumetric flow rate of gases flow into the gases inlet 121 out of the nasal interface through the first prong 111, optionally between about 70% and about 75% of the total volumetric flow rate of gases flow into the gases inlet 121 out of the nasal interface through the first prong 111, optionally about 70% of the total volumetric flow rate of gases flow into the gases inlet 121 out of the nasal interface through the first prong 111, optionally between about 75% and about 80% of the total volumetric flow rate of gases flow into the gases inlet 121 out of the nasal interface through the first prong 111, optionally about 75% of the total volumetric flow rate of gases flow into the gases inlet 121 out of the nasal interface through the first prong 111, optionally about 80% of the total volumetric flow rate of gases flow into the gases inlet 121 out of the nasal interface through the first prong 111.

In some configurations, the respiratory therapy system 1000 is configured to provide different flow rates of gases through the first prong 111 and the second prong 112 and to deliver a substantially similar velocity of gases through the first prong outlet 111a and the second prong outlet 112a.

In some configurations, the velocity of gases exiting the first prong outlet 111a is within about 20% of the velocity of gases exiting the second prong outlet 112a, optionally within about 16% of the velocity of gases exiting the second prong outlet 112a, and optionally within about 10% of the velocity of gases exiting the second prong outlet 112a at flow rates above about 42 lpm.

In some configurations, the velocity of gases exiting each of the first prong outlet 111a and the second prong outlet 112a is more than 0 m/s and less than 32 m/s for a total volumetric flow rate of gases flow into the gases inlet 121 of more than 0 lpm and up to about 70 lpm.

In some configurations, the velocity of gases exiting each of the first prong outlet 111a and the second prong outlet 112a is more than about 2 m/s and less than about 32 m/s, optionally more than about 2 m/s and less than 32 m/s, optionally more than about 2 m/s and up to about 25 m/s, and optionally more than about 2.5 m/s and up to about 20 m/s for a total volumetric flow rate of gases flow into the gases inlet 121 of more than 9 lpm and up to about 70 lpm.

In some configurations, the nasal interface 100 comprises a cannula body 118 comprising the first prong 111 and the second prong 112.

In some configurations, the gases manifold 120 is integral with the cannula body 118 or is separate from and couplable with the cannula body 118.

In some configurations, the first and second prongs 111, 112 are configured to engage with the nasal passages in an unsealed (non-sealing) manner.

In some configurations, the first and second prongs 111, 112 allow exhaled gases to escape around the first and second prongs 111, 112.

In some configurations, the first and second prongs 111, 112 are configured to provide gases to the patient without interfering with the patient's spontaneous respiration.

In some configurations, the first and second prongs are configured to provide gases to the patient independent of the patient's respiration.

In some configurations, the respiratory therapy system comprises a conduit 300 to receive the gases from the respiratory therapy apparatus and deliver the gases to the gases inlet 121 of the nasal interface.

The respiratory therapy system 1000, patient interface 1, and nasal interface 100 may have any of the features and functionality described herein.

A method of providing respiratory support to a patient is disclosed, the method comprising:
providing a respiratory therapy system 1000 comprising:
   a respiratory therapy apparatus 1100 comprising:
      at least one gases inlet 1027, 1028;
      a flow generator 1011;
      a gases outlet 1021;
   and a patient interface 1 comprising a nasal interface 100, wherein the nasal interface 100 comprises:
      a first prong 111 and a second prong 112 that are asymmetrical to each other, and wherein the first prong 111 has a first prong outlet 111a and the second prong 112 has a second prong outlet 112a;
      and a gases manifold 120 comprising a gases inlet 121, wherein the first prong 111 and the second prong 112 are in fluid communication with the gases inlet 121;
operating the respiratory therapy apparatus 1100 to provide a flow of gases to the nasal interface 100; and
delivering an asymmetrical flow of gases from the respiratory therapy apparatus 1100 through the first prong outlet 111a and the second prong outlet 112a at a patient's nares.

In some configurations, the method comprises delivering the asymmetrical flow of gases at a temperature range of between about 27°C - 37°C, at a relative humidity of greater than about 33 mg/l, and/or at a velocity of more than 0 m/s and less than about 32 m/s for a total volumetric flow rate of gases flow into the gases inlet 121 of more than 0 lpm and up to about 70 lpm.

In some configurations, the method comprises delivering the asymmetrical flow of gases at a temperature range of between about 31°C - 37°C.

In some configurations, the method comprises providing a total volumetric flow rate of gases flow into the gases inlet 121 of at least about 5 liters per minute (lpm), optionally providing a total volumetric flow rate of gases flow into the gases inlet of between about 5 lpm and about 120 Ipm, and optionally providing a total volumetric flow rate of gases flow into the gases inlet of between about 5 lpm and about 70 lpm.

In some configurations, the method comprises delivering at least about 60% of a total volumetric flow rate of gases flow into the gases inlet 121 out of the nasal interface through the first prong 111, optionally delivering between about 60% and about 90% of the total volumetric flow rate of gases flow into the gases inlet 121 out of the nasal interface through the first prong 111, optionally delivering between about 60% and about 80% of the total volumetric flow rate of gases flow into the gases inlet 121 out of the nasal interface through the first prong 111, optionally delivering between about 65% and about 80% of the total volumetric flow rate of gases flow into the gases inlet 121 out of the nasal interface through the first prong 111, optionally delivering between about 70% and about 80% of the total volumetric flow rate of gases flow into the gases inlet 121 out of the nasal interface through the first prong 111, optionally delivering between about 70% and about 75% of the total volumetric flow rate of gases flow into the gases inlet 121 out of the nasal interface through the first prong 111, optionally delivering about 70% of the total volumetric flow rate of gases flow into the gases inlet 121 out of the nasal interface through the first prong 111, optionally delivering between about 75% and about 80% of the total volumetric flow rate of gases flow into the gases inlet 121 out of the nasal interface through the first prong 111, optionally delivering about 75% of the total volumetric flow rate of gases flow into the gases inlet 121 out of the nasal interface through the first prong 111, optionally delivering about 80% of the total volumetric flow rate of gases flow into the gases inlet 121 out of the nasal interface through the first prong 111.

In some configurations, the method comprises delivering gases through the first prong outlet 111a and the second prong outlet 112a with a relative humidity of up to about 44 mg/l.

In some configurations, the method comprises providing different flow rates of gases through the first prong 111 and the second prong 112 and delivering a substantially similar velocity of gases through the first prong outlet 111a and the second prong outlet 112a.

In some configurations, the velocity of gases exiting the first prong outlet 111a is within about 20% of the velocity of gases exiting the second prong outlet 112a, optionally within about 16% of the velocity of gases exiting the second prong outlet 112a, and optionally within about 10% of the velocity of gases exiting the second prong outlet 112a at flow rates above about 42 lpm.

In some configurations, the velocity of gases exiting each of the first prong outlet 111a and the second prong outlet 112a is more than 0 m/s and less than 32 m/s for a total volumetric flow rate of gases flow into the gases inlet 121 of more than 0 lpm and up to about 70 lpm.

In some configurations, the velocity of gases exiting each of the first prong outlet 111a and the second prong outlet 112a is more than about 2 m/s and less than about 32 m/s, optionally more than about 2 m/s and less than 32 m/s, optionally more than about 2 m/s and up to about 25 m/s, and optionally more than about 2.5 m/s and up to about 20 m/s for a total volumetric flow rate of gases flow into the gases inlet 121 of more than 9 lpm and up to about 70 lpm.

In some configurations, the nasal interface 100 comprises a cannula body 118 comprising the first prong 111 and the second prong 112.

In some configurations, the gases manifold 120 is integral with the cannula body 118 or is separate from and couplable with the cannula body 118.

In some configurations, the method comprises engaging the first and second prongs 111, 112 with the nasal passages in an unsealed (non-sealing) manner.

In some configurations, the method comprises allowing exhaled gases to escape around the first and second prongs 111, 112.

In some configurations, the method comprises providing gases to the patient without interfering with the patient's spontaneous respiration.

In some configurations, the method comprises providing gases to the patient independent of the patient's respiration.

In some configurations, the nasal interface 100 is as outlined above or herein.

In some configurations, the respiratory therapy apparatus 1100 comprises a humidifier 1012, and the method comprises humidifying the flow of gases using the humidifier 1012.

In some configurations, the respiratory therapy system 1000 comprises a patient conduit 300 with a heater 300a, and the method comprises operating the heater 300a.

In some configurations, the patient interface comprises a breathable tube that is in fluid communication with the gases inlet, and the method comprises allowing water vapour to pass through a wall of the tube, but preventing liquid water and a bulk flow of gases from flowing through the wall of the tube.

The respiratory therapy system 1000, patient interface 1, and nasal interface 100 used in the method may have any of the features and functionality described herein.

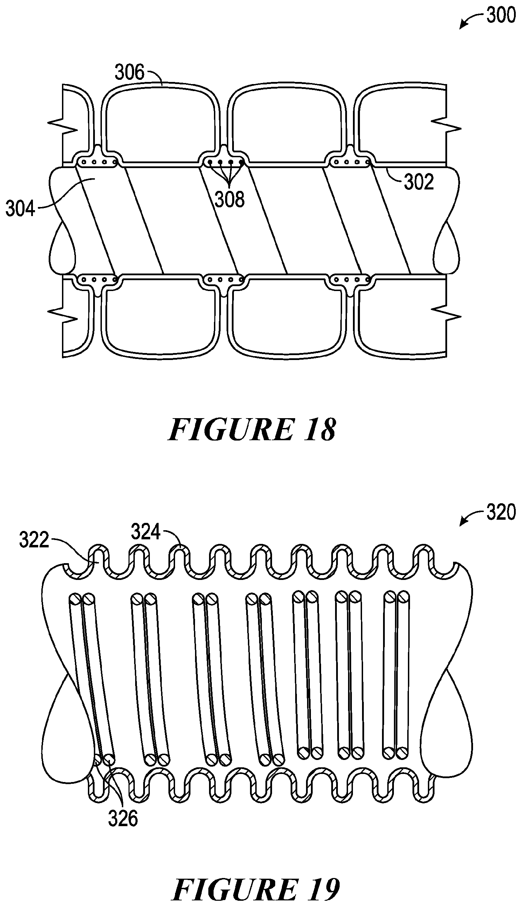

Figure 18 shows an exemplary type of tubing or conduit 300 that can be used to deliver the gases to the nasal interface 100. The tubing or conduit 300 is illustrated featuring a smooth bore 302 or a non-corrugated bore. This type of tubing is best described and illustrated in in US patent application publication no. 2014/0202462 (also published as PCT publication no. WO2012/164407A1) and PCT publication no. WO2014/088430 and US patent no. 11,058,844, for example. As described therein, the tubing is formed of a bead 304 and a small tube or bubble 306. In general, the peak to valley surface roughness of such tubing is on the order of 0.15-0.25 mm. In one configuration, the conduit or tubing has an internal bore diameter of 13-14 mm. The two components 304, 306 combine to define a conduit or tube with a lumen that has minimal surface deviations. In some configurations, the bead 304 contains wires 308. One or more of the wires can be used for heating the wall of the conduit without being positioned within the flow being conveyed by the conduit or tubing 300. In the illustrated configuration, the bead 304 contains four wires 308. In some configurations, the bead 304 may contain two wires 308. Other number of wires also can be used.

Figure 19 shows an alternative exemplary type of tubing or conduit 320 that can be used to deliver the gases to the nasal interface 100. With reference to Figure 20, the illustrated conduit or tubing 320 is corrugated tubing. In one configuration, the conduit or tubing 320 has an internal bore diameter of 20-21 mm. The corrugated tubing 320 includes deep furrows 322 along a wall 324 of the tubing 320. In many cases, the furrows 322 result in one or more helical interruption that extends along a length of the lumen defined by the wall 324. As such, the inner surface of the conduit or tubing is significantly rougher than the smooth bore tubing 300 illustrated in Figure 18. In general, the corrugated conduit or tubing has peak to valley surface roughness on the order of 1.5-2.5 mm. In the illustrated configuration of Figure 19, one or more heating wires 326 also can be coiled and positioned in direct contact with the gas flow through the lumen. When the wires are positioned within the gas flow path, the heater wire adds 2-3 mm of added "surface roughness" although this is merely an estimate of the effect of the heater wire being positioned within the gas flow path.

Use of the smooth bore heating tube 300, such as that illustrated in Figure 18, for use in drug transportation from the nebulizer 2128 described above, has resulted in significant increases in drug transportation efficiency compared to use of a more conventional heated breathing tube 320, such as that illustrated in Figure 19. The efficiency improvement is believed to be due to a large reduction of the amount of nebulised drug being caught within the furrows 322 and the exposed heating wires 326 of the more conventional heated breathing tube 320. For example, it has been estimated that 300% more of the nebulised drug is captured by the surfaces than that which is retained within the smooth bore heated breathing tube 300, such as that shown in Figure 18, for example but without limitation. It is believed there is a reduction in the deposition processes, such as impaction, due to less vorticity in the flow and less obstacles that present an effective roughness.

In some configurations, when the flow rate exceeds an optimal flow rate, the transportation efficiency has been found to decrease. In other words, at some high flow rates above 30 Ipm, the flow rate is somewhat inversely proportional to nebulization efficiency (i.e., high flow rates result in more medication become trapped within the circuit instead of being delivered to the patient).

With the nasal cannula 100 with asymmetrical nasal prongs 111, 112, a reduction in flow rate for an equivalent dead space clearance may be possible which may improve the provision of respiratory therapy with nebulized medicament. The nebulized medicament may be less likely to 'crash out' in which a portion of the medicament is deposited on the internal surface of the flow path instead of being delivered to the patient, or suffer from other losses owing to impacting on surfaces due to smoother flow transitions. With the partial unidirectional flow provided by the nasal interface 100, when a patient is breathing out against the flow, less medicament is wasted than may otherwise be the case. Other aspects of the nasal cannula 100 with asymmetrical nasal prongs 111, 112, including the cross-sectional areas of the prongs and the relationships of those cross-sectional areas, may improve the provision of respiratory therapy with nebulized medicament.

The patient interface 1 and nasal interface 100 used in the respiratory therapy system 2000 may have any one or more of the features and/or functionality described herein for nasal interfaces 100, 100', 100".

The respiratory therapy system 2000 may have any one or more of the features and/or functionality of the system described in PCT publication no. WO 2016/085354 or US patent application publication no. 2017/0312472.

Additionally, or alternatively, the respiratory therapy system 2000 may have any one or more of the features and/or functionality of the system described in relation to the respiratory therapy system 1000.

The nasal interfaces 100, 100', 100" disclosed herein could be used in a medical care facility, home environment, emergency vehicle, or any other suitable environment. Therefore, references herein to "patient" should be interpreted to be any suitable subject that the nasal interfaces are used for or by.

Although the present disclosure has been described in terms of certain embodiments, other embodiments apparent to those of ordinary skill in the art also are within the scope of this disclosure. Thus, various changes and modifications may be made without departing from the scope of the disclosure. For instance, various components may be repositioned as desired. Features from any of the described embodiments may be combined with each other and/or an apparatus may comprise one, more, or all of the features of the above described embodiments. Moreover, not all of the features, aspects and advantages are necessarily required to practice the present disclosure. Accordingly, the scope of the present disclosure is intended to be defined only by the claims that follow.

## Claims

1. A nasal interface (100) comprising
a gases inlet (121),
a first prong (111) and a second prong (112) that are asymmetrical to each other,
and a gases flow path (122) from the gases inlet (121) to the first prong (111) and the second prong (112),
wherein the first prong (111) has a larger inner cross-sectional area (A1) in a direction transverse to gases flow (GFD1) through the first prong (111) than a corresponding inner cross-sectional area (A2) of the second prong (112),
**characterised in that**
the first prong (111) is downstream in the gases flow path (122) from the second prong (112).

2. The nasal interface (100) according to claim 1, wherein the direction transverse to gases flow (GFD1, GFD2) is substantially perpendicular or normal to gases flow through the respective prong (111, 112).

3. The nasal interface (100) according to claim 1 or 2, wherein the inner cross-sectional areas (A1, A2) are at outlets (111a, 112a) of the first and second prongs (111, 112).

4. The nasal interface (100) according to any one of claims 1 to 3, wherein the nasal interface (100) comprises a cannula body (118) comprising the first prong (111) and the second prong (112); and optionally comprising a gases manifold (120), wherein the gases manifold (120) is integral with the cannula body (118) or is separate from and couplable with the cannula body (118).

5. The nasal interface (100) according to claim 4, wherein the nasal interface (100) comprises a face mount part (110), and wherein the first prong (111) and the second prong (112) comprise a pair of asymmetrical tubular nasal prongs (111, 112), integrally moulded with or removably attached to the face mount part (110); and optionally wherein the face mount part (110) comprises at least one substantially horizontal side entry passage (118a, 118b) to the interior of a base portion or cannula body (118) of the face mount part (110) for releasably receiving an outlet (123) of the gases manifold (120) therethrough; and optionally wherein the face mount part (110) comprises a pair of opposed side entry passages (118a, 118b) to the interior of the base portion or cannula body (118), each adapted to releasably receive the outlet (123) of the gases manifold (120) therethrough.

6. The nasal interface (100) according to claim 5, wherein the gases manifold (120) is insertable into the face mount part (110), or wherein the gases manifold (120) is insertable into the face mount part (110) from either of two opposing horizontal directions.

7. The nasal interface (100) according to claim 5 or 6, wherein the gases manifold (120) is formed from a relatively harder material and the face mount part (110) provides a soft interfacing component to a patient, wherein the gases manifold (120) fluidly couples the conduit to the nasal prongs (111, 112) of the face mount part (110).

8. The nasal interface (100) according to any one of claims 1 to 7, wherein the first prong (111) and second prong (112) are configured to engage with nasal passages of a patient in an unsealed manner and/or wherein the first and second prongs (111, 112) allow exhaled gases to escape around the first and second prongs (111, 112); and/or wherein the first and second prongs (111, 112) are configured to provide gases to a patient without interfering with the patient's spontaneous respiration.

9. The nasal interface (100) according to any one of claims 1 to 8, wherein the first and second prongs (111, 112) are curved to extend into a patient's nares in use and to provide a smooth flow path for gases to flow through, and/or inner surfaces of the first and second prongs (111, 112) are contoured to reduce noise, and/or bases of the first and second prongs (111, 112) include curved surfaces to provide for smoother gases flow, and/or the first and second prongs (111, 112) are substantially hollow and substantially tubular in shape, and/or the first and second prongs (111, 112) are consistent in diameter along their lengths or alternatively are shaped to fit the contours of nares of a patent, and/or a or the face mount part (110) is shaped to generally follow contours of a patient's face around the upper lip area, and/or a or the face mount part (110) is moulded or pre-formed to be able to conform to and/or is pliable to adapt, accommodate and/or correspond with contours of a patient's face in the region of the face where the nasal interface (100) is to be located.

10. The nasal interface (100) according to any one of claims 1 to 9, wherein a ratio of the inner cross-sectional area (A1) of the first prong (111) to the inner cross-sectional area (A2) of the second prong (112) is between about 60:40 and about 80:20; or between about 65:35 and about 80:20; or between about 70:30 and about 80:20; or between about 70:30 and about 75:25; or about 70:30, about 71:29, about 72:28, about 73:27, about 74:26, or about 75:25; or between about 75:25 and 80:20; or about 75:25, about 76:24, about 77:23, about 78:22, about 79:21, or about 80:20.

11. The nasal interface (100) according to any one of claims 1 to 10, wherein the gases flow path (122) is defined by a flow channel (124) that has a gases flow direction (GFD3) that is substantially perpendicular to gases flow paths through the first prong (111) and the second prong (112), and wherein the first prong (111) is more distal the gases inlet (121) and the second prong (112) is more proximal the gases inlet (121), and optionally wherein the nasal interface (100) comprises a cannula body (118) comprising the first prong (111) and the second prong (112), wherein the nasal interface (100) comprises a gases manifold (120), wherein the gases manifold (120) is integral with the cannula body (118) or is separate from and couplable with the cannula body (118), wherein a first section (124a) of the flow channel (124) in the gases manifold (120) has a first large vertical dimension (V1), an opposite end of the flow channel (124) forms a flow cavity (124b) in the cannula body (118) that delivers gases to the first prong (111) and the second prong (112), the flow cavity (124b) is in fluid communication with the flow passages through the first prong (111) and the second prong (112) when the gases manifold (120) is in position in the cannula body (118), and wherein at least part of the flow cavity (124b) has a vertical dimension (V2) that is smaller than the first vertical dimension (V1), and optionally wherein the gases manifold (120) comprises one or more internal angled walls to provide a reduction in dimensions from the first vertical dimension (V1) to the vertical dimension (V2) that is smaller than the first vertical dimension (V1) and to direct the gases flow into the first prong (111) and/or the second prong (112).

12. The nasal interface (100) according to any one of claims 1 to 11, wherein a or the gases manifold (120) of the nasal interface (100) is configured to not obstruct any part of the internal cross-section of either of the first prong (111) and the second prong (112), or wherein the gases manifold (120) is configured to partly obstruct the internal cross-section of one or both of the prongs (111, 112).

13. The nasal interface (100) according to any one of claims 1 to 12, wherein the nasal interface (100) is configured such that at least about 60% of a total volumetric flow rate of gases flow into the gases inlet (121) is delivered out of the nasal interface (100) through the first prong (111), or such that between about 60% and about 90% of the total volumetric flow rate of gases flow into the gases inlet (121) is delivered out of the nasal interface (100) through the first prong (111), or such that between about 60% and about 80% of the total volumetric flow rate of gases flow into the gases inlet (121) is delivered out of the nasal interface (100) through the first prong (111), or such that between about 65% and about 80% of the total volumetric flow rate of gases flow into the gases inlet (121) is delivered out of the nasal interface (100) through the first prong (111), or such that between about 70% and about 80% of the total volumetric flow rate of gases flow into the gases inlet (121) is delivered out of the nasal interface (100) through the first prong (111), or such that between about 70% and about 75% of the total volumetric flow rate of gases flow into the gases inlet (121) is delivered out of the nasal interface (100) through the first prong (111), or such that about 70% of the total volumetric flow rate of gases flow into the gases inlet (121) is delivered out of the nasal interface (100) through the first prong (111), or such that between about 75% and about 80% of the total volumetric flow rate of gases flow into the gases inlet (121) is delivered out of the nasal interface (100) through the first prong (111), or such that about 75% of the total volumetric flow rate of gases flow into the gases inlet (121) is delivered out of the nasal interface (100) through the first prong (111), or such that about 80% of the total volumetric flow rate of gases flow into the gases inlet (121) is delivered out of the nasal interface (100) through the first prong (111).

14. The nasal interface (100) according to any one of claims 1 to 13, wherein the nasal interface (100) is configured such that between about 60% and about 80% of a total volumetric flow rate of gases flow into the gases inlet (121) is delivered out of the nasal interface (100) through the first prong (111) when the total volumetric flow rate of gases flow into the gases inlet (121) is at least about 5 litres per minute (lpm), or is more than about 5 Ipm, or is between about 5 lpm and about 120 Ipm, or is between about 5 lpm and about 70 lpm; and/or wherein the nasal interface (100) is configured such that between about 70% and about 80% of a total volumetric flow rate of gases flow into the gases inlet (121) is delivered out of the nasal interface (100) through the first prong (111) when the total flow rate of gases flow into the gases inlet (121) is between about 5 litres per minute (lpm) and about 70 Ipm, or the nasal interface (100) is configured such that between about 70% and about 75% of the total volumetric flow rate of gases flow into the gases inlet (121) is delivered out of the nasal interface (100) through the first prong (111) when the total flow rate of gases flow into the gases inlet (121) is between about 5 lpm and about 70 Ipm, or the nasal interface (100) is configured such that between about 75% and about 80% of the total volumetric flow rate of gases flow into the gases inlet (121) is delivered out of the nasal interface (100) through the first prong (111) when the total flow rate of gases flow into the gases inlet (121) is between about 5 lpm and about 70 Ipm, or the nasal interface (100) is configured such that about 75% of the total volumetric flow rate of gases flow into the gases inlet (121) is delivered out of the nasal interface (100) through the first prong (111) when the total flow rate of gases flow into the gases inlet (121) is between about 5 lpm and about 70 lpm.

15. The nasal interface (100) according to any one of claims 1 to 14, wherein the gases inlet (121) is in fluid communication with a breathable tube, and optionally wherein water vapour can pass through a wall of the breathable tube, but liquid water and a bulk flow of gases cannot flow through the wall of the breathable tube; and/or wherein the nasal interface (100) comprises a or the cannula body (118) comprising the first prong (111) and the second prong (112), and comprises a or the gases manifold (120), wherein the gases manifold (120) is reconfigurable relative to the cannula body (118) between a first configuration and a second configuration, wherein the first configuration corresponds to the gases manifold (120) being inserted into the cannula body (118) from a first side of the cannula body (118) such that the second prong (112) is more proximal the gases inlet (121) and the first prong (111) is more distal the gases inlet (121), and the second configuration corresponds to the gases manifold (120) being inserted into the cannula body (118) from a second side of the cannula body (118) such that the first prong (111) is more proximal the gases inlet (121) and the second prong (112) is more distal the gases inlet (121), and/or; wherein the first prong (111) and the second prong (112) are asymmetrical to each other and/or are not symmetrical to each other and/or differ in shape and configuration to each other and/or are asymmetrical when compared to each other.

## Patentansprüche

1. Nasenschnittstelle (100), umfassend
einen Gaseinlass (121),
einen ersten Stutzen (111) und einen zweiten Stutzen (112), die asymmetrisch zueinander sind,
und einen Gasströmungsweg (122) von dem Gaseinlass (121) zu dem ersten Stutzen (111) und dem zweiten Stutzen (112),
wobei der erste Stutzen (111) in einer Richtung quer zur Gasströmung (GFD1) durch den ersten Stutzen (111) einen größeren inneren Querschnittsbereich (A1) aufweist als ein entsprechender innerer Querschnittsbereich (A2) des zweiten Stutzens (112),
**dadurch gekennzeichnet, dass**
der erste Stutzen (111) in dem Gasströmungsweg (122) stromabwärts von dem zweiten Stutzen (112) ist.

2. Nasenschnittstelle (100) nach Anspruch 1, wobei die Richtung quer zur Gasströmung (GFD1, GFD2) im Wesentlichen senkrecht oder normal zur Gasströmung durch den jeweiligen Stutzen (111, 112) ist.

3. Nasenschnittstelle (100) nach Anspruch 1 oder 2, wobei sich die inneren Querschnittsbereiche (A1, A2) an Auslässen (111a, 112a) des ersten und zweiten Stutzens (111, 112) befinden.

4. Nasenschnittstelle (100) nach einem der Ansprüche 1 bis 3, wobei die Nasenschnittstelle (100) einen Kanülenkörper (118) umfasst, der den ersten Stutzen (111) und den zweiten Stutzen (112) umfasst; und optional umfassend einen Gasverteiler (120), wobei der Gasverteiler (120) einstückig mit dem Kanülenkörper (118) ist oder getrennt von und koppelbar mit dem Kanülenkörper (118) ist.

5. Nasenschnittstelle (100) nach Anspruch 4, wobei die Nasenschnittstelle (100) ein Gesichtshalterungsteil (110) umfasst, und wobei der erste Stutzen (111) und der zweite Stutzen (112) ein Paar asymmetrischer röhrenförmiger Nasenstutzen (111, 112) umfassen, einstückig geformt mit oder abnehmbar angebracht an dem Gesichtsbefestigungsteil (110); und wobei optional das Gesichtshalterungsteil (110) mindestens einen im Wesentlichen horizontalen Seiteneintrittsdurchgang (118a, 118b) in das Innere eines Basisabschnitts oder Kanülenkörpers (118) des Gesichtshalterungsteils (110) zur lösbaren Aufnahme eines Auslasses (123) des Gasverteilers (120) durch denselben umfasst; und wobei optional das Gesichtshalterungsteil (110) ein Paar gegenüberliegender Seiteneintrittsdurchgänge (118a, 118b) in das Innere des Basisabschnitts oder Kanülenkörpers (118) umfasst, die jeweils angepasst sind, um den Auslass (123) des Gasverteilers (120) durch dieselben lösbar aufzunehmen.

6. Nasenschnittstelle (100) nach Anspruch 5, wobei der Gasverteiler (120) in das Gesichtshalterungsteil (110) einsetzbar ist, oder wobei der Gasverteiler (120) aus einer von zwei entgegengesetzten horizontalen Richtungen in das Gesichtshalterungsteil (110) einsetzbar ist.

7. Nasenschnittstelle (100) nach Anspruch 5 oder 6, wobei der Gasverteiler (120) aus einem relativ härteren Material ausgebildet ist und das Gesichtshalterungsteil (110) eine weiche Schnittstellenkomponente für einen Patienten bereitstellt, wobei der Gasverteiler (120) die Leitung fluidisch mit den Nasenstutzen (111, 112) des Gesichtshalterungsteils (110) koppelt.

8. Nasenschnittstelle (100) nach einem der Ansprüche 1 bis 7, wobei der erste Stutzen (111) und der zweite Stutzen (112) konfiguriert sind, um auf nicht abdichtende Weise in die Nasengänge eines Patienten einzugreifen, und/oder wobei der erste und der zweite Stutzen (111, 112) es ermöglichen, dass ausgeatmete Gase um den ersten und den zweiten Stutzen (111, 112) herum entweichen; und/oder wobei der erste und der zweite Stutzen (111, 112) konfiguriert sind, um einem Patienten Gase bereitzustellen, ohne die Spontanatmung des Patienten zu beeinträchtigen.

9. Nasenschnittstelle (100) nach einem der Ansprüche 1 bis 8, wobei der erste und der zweite Stutzen (111, 112) gekrümmt sind, um sich in Verwendung in die Nasenlöcher eines Patienten zu erstrecken und einen glatten Strömungsweg bereitzustellen, damit Gase hindurchströmen, und/oder innere Oberflächen des ersten und des zweiten Stutzens (111, 112) konturiert sind, um Geräusche zu reduzieren, und/oder Basen des ersten und des zweiten Stutzens (111, 112) gekrümmte Oberflächen einschließen, um eine glattere Gasströmung bereitzustellen, und/oder der erste und der zweite Stutzen (111, 112) im Wesentlichen hohl und im Wesentlichen röhrenförmig sind, und/oder der erste und der zweite Stutzen (111, 112) entlang ihrer Länge in Bezug auf ihren Durchmesser konsistent sind oder alternativ so geformt sind, dass sie sich an die Konturen der Nasenlöcher eines Patienten anpassen, und/oder ein oder das Gesichtshalterungsteil (110) so geformt ist, dass es im Allgemeinen den Konturen des Gesichts eines Patienten um den oberen Lippenbereich folgt, und/oder ein oder das Gesichtshalterungsteil (110) modelliert oder vorgeformt ist, um sich an Konturen des Gesichts eines Patienten in der Region des Gesichts, in dem die Nasenschnittstelle (100) angeordnet werden soll, angleichen zu können, und/oder ist biegsam, um sich an diese anzupassen, diese zu beherbergen und/oder diesen zu entsprechen.

10. Nasenschnittstelle (100) nach einem der Ansprüche 1 bis 9, wobei ein Verhältnis des inneren Querschnittsbereichs (A1) des ersten Stutzens (111) zu dem inneren Querschnittsbereich (A2) des zweiten Stutzens (112) zwischen etwa 60:40 und etwa 80:20 beträgt; oder zwischen etwa 65:35 und etwa 80:20; oder zwischen etwa 70:30 und etwa 80:20; oder zwischen etwa 70:30 und etwa 75:25; oder etwa 70:30, etwa 71:29, etwa 72:28, etwa 73:27, etwa 74:26 oder etwa 75:25; oder zwischen etwa 75:25 und 80:20; oder etwa 75:25, etwa 76:24, etwa 77:23, etwa 78:22, etwa 79:21 oder etwa 80:20.

11. Nasenschnittstelle (100) nach einem der Ansprüche 1 bis 10, wobei der Gasströmungsweg (122) durch einen Strömungskanal (124) definiert ist, der eine Gasströmungsrichtung (GFD3) aufweist, die im Wesentlichen senkrecht zu Gasströmungswegen durch den ersten Stutzen (111) und den zweiten Stutzen (112) ist, und wobei der erste Stutzen (111) distaler zu dem Gaseinlass (121) ist und der zweite Stutzen (112) proximaler zu dem Gaseinlass (121) ist, und wobei optional die Nasenschnittstelle (100) einen Kanülenkörper (118) umfasst, der den ersten Stutzen (111) und den zweiten Stutzen (112) umfasst, wobei die Nasenschnittstelle (100) einen Gasverteiler (120) umfasst, wobei der Gasverteiler (120) einstückig mit dem Kanülenkörper (118) ist oder getrennt von und koppelbar mit dem Kanülenkörper (118) ist, wobei ein erster Abschnitt (124a) des Strömungskanals (124) in dem Gasverteiler(120) eine erste große vertikale Abmessung (V1) aufweist, ein gegenüberliegendes Ende des Strömungskanals (124) einen Strömungshohlraum (124b) in dem Kanülenkörper (118) ausbildet, der dem ersten Stutzen (111) und dem zweiten Stutzen (112) Gase zuführt, der Strömungshohlraum (124b) durch den ersten Stutzen (111) und den zweiten Stutzen (112) in Fluidkommunikation mit den Strömungsdurchgängen steht, wenn sich der Gasverteiler (120) in Position in dem Kanülenkörper (118) befindet, und wobei mindestens ein Teil des Strömungshohlraums (124b) eine vertikale Abmessung (V2) aufweist, die kleiner als die erste vertikale Abmessung (V1) ist, und wobei optional der Gasverteiler (120) eine oder mehrere innere abgewinkelte Wände umfasst, um eine Reduzierung der Abmessungen von der ersten vertikalen Abmessung (V1) auf die vertikale Abmessung (V2), die kleiner als die erste vertikale Abmessung (V1) ist, bereitzustellen und um die Gase dazu zu lenken, in den ersten Stutzen (111) und/oder den zweiten Stutzen (112) zu strömen.

12. Nasenschnittstelle (100) nach einem der Ansprüche 1 bis 11, wobei ein oder der Gasverteiler (120) der Nasenschnittstelle (100) konfiguriert ist, um keinen Teil des inneren Querschnitts weder des ersten Stutzens (111) noch des zweiten Stutzens (112) zu blockieren, oder wobei der Gasverteiler (120) konfiguriert ist, um den inneren Querschnitt eines oder beider der Stutzen (111, 112) teilweise zu blockieren.

13. Nasenschnittstelle (100) nach einem der Ansprüche 1 bis 12, wobei die Nasenschnittstelle (100) so konfiguriert ist, dass mindestens etwa 60 % einer Gesamtvolumenströmungsrate von Gasen, die in den Gaseinlass (121) strömen, aus der Nasenschnittstelle (100) durch den ersten Stutzen (111) zugeführt werden, oder so, dass zwischen etwa 60 % und etwa 90 % der Gesamtvolumenströmungsrate von Gasen, die in den Gaseinlass (121) strömen, aus der Nasenschnittstelle (100) durch den ersten Stutzen (111) zugeführt werden, oder so, dass zwischen etwa 60 % und etwa 80 % der Gesamtvolumenströmungsrate von Gasen, die in den Gaseinlass (121) strömen, aus der Nasenschnittstelle (100) durch den ersten Stutzen (111) zugeführt werden, oder so, dass zwischen etwa 65 % und etwa 80 % der Gesamtvolumenströmungsrate von Gasen, die in den Gaseinlass (121) strömen, aus der Nasenschnittstelle (100) durch den ersten Stutzen (111) zugeführt werden, oder so, dass zwischen etwa 70 % und etwa 80 % der Gesamtvolumenströmungsrate von Gasen, die in den Gaseinlass (121) strömen, aus der Nasenschnittstelle (100) durch den ersten Stutzen (111) zugeführt werden, oder so, dass zwischen etwa 70 % und etwa 75 % der Gesamtvolumenströmungsrate von Gasen, die in den Gaseinlass (121) strömen, aus der Nasenschnittstelle (100) durch den ersten Stutzen (111) zugeführt werden, oder so, dass etwa 70 % der Gesamtvolumenströmungsrate von Gasen, die in den Gaseinlass (121) strömen, aus der Nasenschnittstelle (100) durch den ersten Stutzen (111) zugeführt werden, oder so, dass zwischen etwa 75 % und etwa 80 % der Gesamtvolumenströmungsrate von Gasen, die in den Gaseinlass (121) strömen, aus der Nasenschnittstelle (100) durch den ersten Stutzen (111) zugeführt werden, oder so, dass etwa 75 % der Gesamtvolumenströmungsrate von Gasen, die in den Gaseinlass (121) strömen, aus der Nasenschnittstelle (100) durch den ersten Stutzen (111) zugeführt werden, oder so, dass etwa 80 % der Gesamtvolumenströmungsrate von Gasen, die in den Gaseinlass (121) strömen, aus der Nasenschnittstelle (100) durch den ersten Stutzen (111) zugeführt werden.

14. Nasenschnittstelle (100) nach einem der Ansprüche 1 bis 13, wobei die Nasenschnittstelle (100) so konfiguriert ist, dass zwischen etwa 60 % und etwa 80 % einer Gesamtvolumenströmungsrate von Gasen, die in den Gaseinlass (121) strömen, aus der Nasenschnittstelle (100) durch den ersten Stutzen (111) zugeführt werden, wenn die Gesamtvolumenströmungsrate von Gasen, die in den Gaseinlass (121) strömen, mindestens etwa 5 Liter pro Minute (lpm) beträgt, oder mehr als etwa 5 lpm beträgt, oder zwischen etwa 5 lpm und etwa 120 lpm beträgt, oder zwischen etwa 5 lpm und etwa 70 lpm beträgt; und/oder wobei die Nasenschnittstelle (100) so konfiguriert ist, dass zwischen etwa 70 % und etwa 80 % einer Gesamtvolumenströmungsrate von Gasen, die in den Gaseinlass (121) strömen, aus der Nasenschnittstelle (100) durch den ersten Stutzen (111) zugeführt werden, wenn die Gesamtströmungsrate von Gasen, die in den Gaseinlass (121) strömen, zwischen etwa 5 Litern pro Minute (lpm) und etwa 70 lpm beträgt, oder die Nasenschnittstelle (100) so konfiguriert ist, dass zwischen etwa 70 % und etwa 75 % der Gesamtvolumenströmungsrate von Gasen, die in den Gaseinlass (121) strömen, aus der Nasenschnittstelle (100) durch den ersten Stutzen (111) zugeführt werden, wenn die Gesamtströmungsrate von Gasen, die in den Gaseinlass (121) strömen, zwischen etwa 5 lpm und etwa 70 lpm beträgt, oder die Nasenschnittstelle (100) so konfiguriert ist, dass zwischen etwa 75 % und etwa 80 % der Gesamtvolumenströmungsrate von Gasen, die in den Gaseinlass (121) strömen, aus der Nasenschnittstelle (100) durch den ersten Stutzen (111) zugeführt werden, wenn die Gesamtströmungsrate von Gasen, die in den Gaseinlass (121) strömen, zwischen etwa 5 lpm und etwa 70 lpm beträgt, oder die Nasenschnittstelle (100) so konfiguriert ist, dass etwa 75 % der Gesamtvolumenströmungsrate von Gasen, die in den Gaseinlass (121) strömen, aus der Nasenschnittstelle (100) durch den ersten Stutzen (111) zugeführt werden, wenn die Gesamtströmungsrate von Gasen, die in den Gaseinlass (121) strömen, zwischen etwa 5 lpm und etwa 70 lpm beträgt.

15. Nasenschnittstelle (100) nach einem der Ansprüche 1 bis 14, wobei der Gaseinlass (121) in Fluidkommunikation mit einem atmungsfähigen Schlauch steht, und wobei optional Wasserdampf durch eine Wand des atmungsfähigen Schlauchs hindurchtreten kann, aber flüssiges Wasser und eine Massenströmung von Gasen nicht durch die Wand des atmungsfähigen Schlauchs strömen können; und/oder wobei die Nasenschnittstelle (100) einen oder den Kanülenkörper (118) umfasst, der den ersten Stutzen (111) und den zweiten Stutzen (112) umfasst, und einen oder den Gasverteiler (120) umfasst, wobei der Gasverteiler (120) relativ zu dem Kanülenkörper (118) zwischen einer ersten Konfiguration und einer zweiten Konfiguration rekonfigurierbar ist, wobei die erste Konfiguration dem entspricht, dass der Gasverteiler (120) von einer ersten Seite des Kanülenkörpers (118) in den Kanülenkörper (118) eingesetzt ist, sodass der zweite Stutzen (112) proximaler zu dem Gaseinlass (121) ist und der erste Stutzen (111) distaler zu dem Gaseinlass (121) ist, und die zweite Konfiguration dem entspricht, dass der Gasverteiler (120) von einer zweiten Seite des Kanülenkörpers (118) in den Kanülenkörper (118) eingesetzt ist, sodass der erste Stutzen (111) proximaler zu dem Gaseinlass ist (121) und der zweite Stutzen (112) distaler zu dem Gaseinlass (121) ist, und/oder; wobei der erste Stutzen (111) und der zweite Stutzen (112) asymmetrisch zueinander sind und/oder nicht symmetrisch zueinander sind und/oder sich in Form und Konfiguration voneinander unterscheiden und/oder im Vergleich zueinander asymmetrisch sind.

## Revendications

1. Interface nasale (100) comprenant
une entrée de gaz (121),
une première branche (111) et une seconde branche (112) qui sont asymétriques l'une par rapport à l'autre,
et un chemin d'écoulement de gaz (122) à partir de l'entrée des gaz (121) vers la première branche (111) et la seconde branche (112),
dans laquelle la première branche (111) présente une zone de section transversale interne (A1) plus grande dans une direction transversale à l'écoulement de gaz (GFD1) à travers la première branche (111) qu'une zone de section transversale interne correspondante (A2) de la seconde branche (112), **caractérisée en ce que**
la première branche (111) est en aval dans le chemin d'écoulement de gaz (122) par rapport à la seconde branche (112).

2. Interface nasale (100) selon la revendication 1, dans laquelle la direction transversale vers l'écoulement de gaz (GFD1, GFD2) est sensiblement perpendiculaire ou normale à l'écoulement de gaz à travers la branche respective (111, 112).

3. Interface nasale (100) selon la revendication 1 ou 2, dans laquelle les zones de section transversale internes (A1, A2) se trouvent au niveau des sorties (111a, 112a) des première et seconde branches (111, 112).

4. Interface nasale (100) selon l'une quelconque des revendications 1 à 3, dans laquelle l'interface nasale (100) comprend un corps de canule (118) comprenant la première branche (111) et la seconde branche (112) ; et comprenant éventuellement un collecteur de gaz (120), dans lequel le collecteur de gaz (120) est solidaire du corps de canule (118) ou est séparé du corps de canule (118) et peut être accouplé à celui-ci.

5. Interface nasale (100) selon la revendication 4, dans laquelle l'interface nasale (100) comprend une pièce de montage frontal (110), et dans laquelle la première branche (111) et la seconde branche (112) comprennent une paire de branches nasales tubulaires asymétriques (111, 112), moulées d'un seul tenant avec la pièce de montage frontal (110) ou fixées de manière amovible à celle-ci ; et éventuellement dans laquelle la pièce de montage frontal (110) comprend au moins une voie d'entrée latérale sensiblement horizontale (118a, 118b) vers l'intérieur d'une partie de base ou d'un corps de canule (118) de la pièce de montage frontal (110) permettant de recevoir de manière amovible une sortie (123) du collecteur de gaz (120) à travers celui-ci ; et éventuellement dans laquelle la pièce de montage frontal (110) comprend une paire de voies d'entrée latérales opposées (118a, 118b) vers l'intérieur de la partie de base ou du corps de canule (118), chacune étant adaptée pour recevoir de manière amovible la sortie (123) du collecteur de gaz (120) à travers celle-ci.

6. Interface nasale (100) selon la revendication 5, dans laquelle le collecteur de gaz (120) peut être inséré dans la pièce de montage frontal (110), ou dans laquelle le collecteur de gaz (120) peut être inséré dans la pièce de montage frontal (110) depuis l'une ou l'autre de deux directions horizontales opposées.

7. Interface nasale (100) selon la revendication 5 ou 6, dans laquelle le collecteur de gaz (120) est formé d'un matériau relativement plus dur et la pièce de montage frontal (110) fournit un composant d'interface souple à un patient, dans laquelle le collecteur de gaz (120) accouple de manière fluidique le conduit aux branches nasales (111, 112) de la pièce de montage frontal (110).

8. Interface nasale (100) selon l'une quelconque des revendications 1 à 7, dans laquelle la première branche (111) et la seconde branche (112) sont conçues pour entrer en prise dans les voies nasales d'un patient de manière non étanche et/ou dans laquelle les première et seconde branches (111, 112) permettent aux gaz exhalés de s'échapper autour des première et seconde branches (111, 112) ; et/ou dans laquelle les première et seconde branches (111, 112) sont conçues pour fournir des gaz à un patient sans interférer avec la respiration spontanée du patient.

9. Interface nasale (100) selon l'une quelconque des revendications 1 à 8, dans laquelle les première et seconde branches (111, 112) sont incurvées pour s'étendre dans les narines d'un patient lors de l'utilisation et pour fournir un chemin d'écoulement régulier pour que des gaz s'écoulent à travers celui-ci, et/ou des surfaces internes des première et seconde branches (111, 112) sont profilées pour réduire le bruit, et/ou des bases des première et seconde branches (111, 112) comportent des surfaces incurvées pour permettre un écoulement de gaz plus régulier, et/ou les première et seconde branches (111, 112) sont de forme sensiblement creuse et sensiblement tubulaire, et/ou les première et seconde branches (111, 112) présentent un diamètre constant sur leurs longueurs ou, en variante, sont profilées pour épouser les contours des narines d'un patient, et/ou une ou la pièce de montage frontal (110) est profilée pour suivre globalement les contours du visage d'un patient autour de la lèvre supérieure, et/ou une ou la pièce de montage frontal (110) est moulée ou préformée pour pouvoir se conformer à et/ou est souple pour s'adapter, s'ajuster et/ou correspondre aux contours du visage d'un patient dans la région du visage où l'interface nasale (100) doit être située.

10. Interface nasale (100) selon l'une quelconque des revendications 1 à 9, dans laquelle un rapport de la zone de section transversale interne (A1) de la première branche (111) sur la zone de section transversale interne (A2) de la seconde branche (112) est compris entre environ 60:40 et environ 80:20 ; ou entre environ 65:35 et environ 80:20 ; ou entre environ 70:30 et environ 80:20 ; ou entre environ 70:30 et environ 75:25 ; ou environ 70:30, environ 71:29, environ 72:28, environ 73:27, environ 74:26, ou environ 75:25 ; ou entre environ 75:25 et 80:20 ; ou environ 75:25, environ 76:24, environ 77:23, environ 78:22, environ 79:21, ou environ 80:20.

11. Interface nasale (100) selon l'une quelconque des revendications 1 à 10, dans laquelle le chemin d'écoulement de gaz (122) est défini par un canal d'écoulement (124) qui présente une direction d'écoulement de gaz (GFD3) qui est sensiblement perpendiculaire aux chemins d'écoulement de gaz à travers la première branche (111) et la seconde branche (112), et dans laquelle la première branche (111) est plus distale par rapport à l'entrée de gaz (121) et la seconde branche (112) est plus proximale par rapport à l'entrée de gaz (121), et éventuellement dans laquelle l'interface nasale (100) comprend un corps de canule (118) comprenant la première branche (111) et la seconde branche (112), dans laquelle l'interface nasale (100) comprend un collecteur de gaz (120), dans laquelle le collecteur de gaz (120) est solidaire du corps de canule (118) ou est séparé du corps de canule (118) et peut être accouplé à celui-ci, dans laquelle une première section (124a) du canal d'écoulement (124) dans le collecteur de gaz (120) présente une première grande dimension verticale (V1), une extrémité opposée du canal d'écoulement (124) forme une cavité d'écoulement (124b) dans le corps de canule (118) qui distribue des gaz à la première branche (111) et à la seconde branche (112), la cavité d'écoulement (124b) est en communication fluidique avec les voies d'écoulement à travers la première branche (111) et la seconde branche (112) lorsque le collecteur de gaz (120) est en position dans le corps de canule (118), et dans laquelle au moins une partie de la cavité d'écoulement (124b) présente une dimension verticale (V2) qui est plus petite que la première dimension verticale (V1), et éventuellement dans laquelle le collecteur de gaz (120) comprend une ou plusieurs parois inclinées internes pour fournir une réduction de dimensions de la première dimension verticale (V1) à la dimension verticale (V2) qui est plus petite que la première dimension verticale (V1) et pour diriger l'écoulement de gaz dans la première branche (111) et/ou la seconde branche (112).

12. Interface nasale (100) selon l'une quelconque des revendications 1 à 11, dans laquelle un ou le collecteur de gaz (120) de l'interface nasale (100) est conçu pour n'obstruer aucune partie de la section transversale interne de l'un ou l'autre de la première branche (111) et de la seconde branche (112), ou dans laquelle le collecteur de gaz (120) est conçu pour obstruer partiellement la section transversale interne de l'une des branches (111, 112) ou des deux.

13. Interface nasale (100) selon l'une quelconque des revendications 1 à 12, dans laquelle l'interface nasale (100) est conçue de telle sorte qu'au moins environ 60 % d'un débit volumétrique total d'écoulement de gaz dans l'entrée de gaz (121) est délivré hors de l'interface nasale (100) à travers la première branche (111), ou de telle sorte qu'entre environ 60 % et environ 90 % du débit volumétrique total d'écoulement de gaz dans l'entrée de gaz (121) est délivré hors de l'interface nasale (100) à travers la première branche (111), ou de telle sorte qu'entre environ 60 % et environ 80 % du débit volumétrique total d'écoulement de gaz dans l'entrée de gaz (121) est délivré hors de l'interface nasale (100) à travers la première branche (111), ou de telle sorte qu'entre environ 65 % et environ 80 % du débit volumétrique total d'écoulement de gaz dans l'entrée de gaz (121) est délivré hors de l'interface nasale (100) à travers la première branche (111), ou de telle sorte qu'entre environ 70 % et environ 80 % du débit volumétrique total d'écoulement de gaz dans l'entrée de gaz (121) est délivré hors de l'interface nasale (100) à travers la première branche (111), ou de telle sorte qu'entre environ 70 % et environ 75 % du débit volumétrique total d'écoulement de gaz dans l'entrée de gaz (121) est délivré hors de l'interface nasale (100) à travers la première branche (111), ou de telle sorte qu'environ 70 % du débit volumétrique total d'écoulement de gaz dans l'entrée de gaz (121) est délivré hors de l'interface nasale (100) à travers la première branche (111), ou de telle sorte qu'entre environ 75 % et environ 80 % du débit volumétrique total d'écoulement de gaz dans l'entrée de gaz (121) est délivré hors de l'interface nasale (100) à travers la première branche (111), ou de telle sorte qu'environ 75 % du volumétrique total débit d'écoulement de gaz dans l'entrée de gaz (121) est délivré hors de l'interface nasale (100) à travers la première branche (111), ou de telle sorte qu'environ 80 % du débit volumétrique total d'écoulement de gaz dans l'entrée de gaz (121) est délivré hors de l'interface nasale (100) par la première branche (111).

14. Interface nasale (100) selon l'une quelconque des revendications 1 à 13, dans laquelle l'interface nasale (100) est conçue de telle sorte qu'entre environ 60 % et environ 80 % d'un débit volumétrique total d'écoulement de gaz dans l'entrée de gaz (121) est délivré hors de l'interface nasale (100) à travers la première branche (111) lorsque le débit volumétrique total d'écoulement de gaz dans l'entrée de gaz (121) est d'au moins environ 5 litres par minute (lpm), ou est supérieur à environ 5 lpm, ou est compris entre environ 5 lpm et environ 120 lpm, ou est compris entre environ 5 lpm et environ 70 lpm ; et/ou dans laquelle l'interface nasale (100) est conçue de telle sorte qu'entre environ 70 % et environ 80 % d'un débit volumétrique total d'écoulement de gaz dans l'entrée de gaz (121) est distribué hors de l'interface nasale (100) à travers la première branche (111) lorsque le débit total d'écoulement de gaz dans l'entrée de gaz (121) est compris entre environ 5 litres par minute (lpm) et environ 70 lpm, ou l'interface nasale (100) est conçue de telle sorte qu'entre environ 70 % et environ 75 % du débit volumétrique total d'écoulement de gaz dans l'entrée de gaz (121) est distribué hors de l'interface nasale (100) à travers la première branche (111) lorsque le débit total d'écoulement de gaz dans l'entrée de gaz (121) est compris entre environ 5 lpm et environ 70 lpm, ou l'interface nasale (100) est conçue de telle sorte qu'entre environ 75 % et environ 80 % du débit volumétrique total d'écoulement de gaz dans l'entrée de gaz (121) est délivré hors de l'interface nasale (100) à travers la première branche (111) lorsque le débit total d'écoulement de gaz dans l'entrée de gaz (121) est compris entre environ 5 l/min et environ 70 l/min, ou l'interface nasale (100) est conçue de telle sorte qu'environ 75 % du débit volumétrique total d'écoulement de gaz dans l'entrée de gaz (121) est délivré hors de l'interface nasale (100) à travers la première branche (111) lorsque le débit total d'écoulement de gaz dans l'entrée de gaz (121) est compris entre environ 5 l/min et environ 70 l/min.

15. Interface nasale (100) selon l'une quelconque des revendications 1 à 14, dans laquelle l'entrée de gaz (121) est en communication fluidique avec un tube respirant, et éventuellement dans laquelle la vapeur d'eau peut traverser une paroi du tube respirant, mais l'eau liquide et un écoulement global de gaz ne peuvent pas s'écouler à travers la paroi du tube respirant ; et/ou dans laquelle l'interface nasale (100) comprend un ou le corps de canule (118) comprenant la première branche (111) et la seconde branche (112), et comprend un ou le collecteur de gaz (120), dans laquelle le collecteur de gaz (120) est reconfigurable par rapport au corps de canule (118) entre une première configuration et une seconde configuration, dans laquelle la première configuration correspond au collecteur de gaz (120) étant inséré dans le corps de canule (118) depuis un premier côté du corps de canule (118) de telle sorte que la seconde branche (112) est plus proximale par rapport à l'entrée de gaz (121) et la première branche (111) est plus distale par rapport à l'entrée de gaz (121), et la seconde configuration correspond au collecteur de gaz (120) étant inséré dans le corps de canule (118) depuis un second côté du corps de canule (118) de telle sorte que la première branche (111) est plus proximale par rapport à l'entrée de gaz (121) et la seconde branche (112) est plus distale par rapport à l'entrée de gaz (121), et/ou ; dans laquelle la première branche (111) et la seconde branche (112) sont asymétriques l'une par rapport à l'autre et/ou ne sont pas symétriques l'une par rapport à l'autre et/ou diffèrent en forme et en configuration l'une par rapport à l'autre et/ou sont asymétriques lorsqu'elles sont comparées l'une à l'autre.
